(19) Europäisches Patentamt
European Patent Office
Office européen des brevets

(11) EP 2 844 279 B1

(12) **EUROPEAN PATENT SPECIFICATION**

(45) Date of publication and mention of the grant of the patent:
**09.12.2020 Bulletin 2020/50**

(21) Application number: **13785028.5**

(22) Date of filing: **02.05.2013**

(51) Int Cl.:
***A61K 38/47*** *(2006.01)* ***A61K 31/445*** *(2006.01)*
***A61P 3/00*** *(2006.01)*

(86) International application number:
**PCT/US2013/039215**

(87) International publication number:
**WO 2013/166249 (07.11.2013 Gazette 2013/45)**

(54) **DOSING REGIMENS FOR THE TREATMENT OF POMPE DISEASE**

DOSIERUNGSPLÄNE ZUR BEHANDLUNG VON MORBUS POMPE

SCHÉMA POSOLOGIQUE POUR LE TRAITEMENT DE LA MALADIE DE POMPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**03.05.2012 US 201261642311 P**
**25.06.2012 US 201261664011 P**
**05.09.2012 US 201261697179 P**
**04.01.2013 US 201361749132 P**
**04.01.2013 US 201361749234 P**

(43) Date of publication of application:
**11.03.2015 Bulletin 2015/11**

(73) Proprietor: **Amicus Therapeutics, Inc.**
**Cranbury, NJ 08512 (US)**

(72) Inventors:
- **GREENE, Douglas, Stuart**
  **Newtown, PA 18940 (US)**
- **VALENZANO, Kenneth, Joseph**
  **East Brunswick, NJ 08816 (US)**

(74) Representative: **Miller Sturt Kenyon**
**9 John Street**
**London WC1N 2ES (GB)**

(56) References cited:
**US-A1- 2006 264 467**
**US-A1- 2010 119 502**
**US-A1- 2010 266 571**

- **VALENZANO ET AL.: 'Identification and Characterization of Pharmacological Chaperones to Correct Enzyme Deficiencies in Lysosomal Storage Disorders' ASSAY AND DRUG DEVELOPMENT TECHNOLOGIES vol. 9, no. 3, June 2011, pages 213 - 235, XP055175843**
- **BECK ET AL.: 'Alglucosidase alfa: Long term use in the treatment of patients with Pompe disease' THERAPEUTICS AND CLINICAL RISK MANAGEMENT vol. 5, 18 September 2009, pages 767 - 772, XP055175847**
- **U.S. DEPARTMENT OF HEALTH AND HUMAN SERVICES, FOOD AND DRUG ADMINISTRATION, AND CENTER FOR DRUG EVALUATION AND RESEARCH: "Guidance for Industry: Estimating the Maximum Safe Starting Dose in Initial Clinical Trials for Therapeutics in Adult Healthy Volunteers ("Industry Guidance")", GUIDANCE FOR INDUSTRY, 2005,**
- **PRIYA KISHNANI,1 MARK TARNOPOLSKY, ' MARK ROBERTS,1 KUMARSWAMY SIVAKUMAR,4 MAJED DASOUKI,5: "Duvoglustat HCl Increases Systemic and Tissue Exposure of Active Acid a-Glucosidase in PompePatients Co-administered with Alglucosidase a", MOLECULAR THERAPY, 2017,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 844 279 B1

Printed by Jouve, 75001 PARIS (FR)

## Description

## FIELD OF THE APPLICATION

The present application provides a dosing regimen and administration schedule for the use of 1-deoxynojirimycin and enzyme replacement therapy for the treatment of Pompe disease.

## BACKGROUND

Mutations in the lysosomal enzyme acid $\alpha$-glucosidase (GAA) alter lysosomal glycogen catabolism and lead to Pompe disease, also referred to as glycogen storage disease type II or acid maltase deficiency. GAA normally hydrolyzes glycogen at $\alpha$-1,4 and $\alpha$-1,6 linkages to yield glucose. Mutations in the GAA gene result in a deficiency or absence of GAA activity, which leads to an accumulation of glycogen. The glycogen accumulation is thought to lead to progressive muscle myopathy throughout the body, affecting various body tissues, particularly the heart, skeletal muscles, liver, and nervous system. In some cases of Pompe disease, reduced levels of the 110-kDa GAA precursor protein are observed, while in other cases normal levels of 110-kDa precursor protein are synthesized but not processed into the mature, properly glycosylated 76- and 70-kDa GAA forms.

Pompe disease has been historically divided into three major phenotypic expressions (infantile-, juvenile- and adult-onset). However, it is now currently accepted that the disease is a spectrum of phenotypes, ranging from the more severe early-onset form to the less severe late-onset form. The disorder is clinically heterogeneous in age of onset, extent of organ involvement, and rate of progression. The early-onset form of the disease is the most severe, and progresses most rapidly, with accumulation of glycogen most prevalent in cardiac muscle, skeletal muscle, and hepatic tissue. This form of the disease typically is characterized by musculoskeletal, pulmonary, gastrointestinal, and cardiac symptoms. Death due to cardiorespiratory failure usually occurs between 1 and 2 years of age. The late-onset form of the disease usually begins between childhood and adulthood and has a slower rate of progression, usually without cardiac involvement. The phenotype is characterized by musculoskeletal and pulmonary symptoms that leads to progressive weakness and respiratory insufficiency. The symptoms tend to be less severe, and glycogen accumulation less extensive, in the late-onset than in the early-onset form of the disease, resulting in longer survival. Death usually results due to cardiorespiratory failure.

Current treatment of Pompe disease involves symptomatic treatment of the cardiac and respiratory symptoms. There is no approved treatment for the underlying genetic defect. Use of replacement GAA, alglucosidase alfa (Myozyme® (Genzyme Corporation) and Lumizyme® (Genzyme Corporation)) is approved by the F.D.A. in the United States. However, clinical evaluations using enzyme replacement therapy (ERT) to replace defective GAA in infantile Pompe patients was only moderately successful in improving cardiac and skeletal function (Klinge et al., Neuropediatrics. 2005; 36(1): 6-11). Recombinant GAA was shown to be more effective in resolving the cardiomyopathy than the skeletal muscle myopathy (Raben et al., Mol Ther. 2005; 11(1): 48-56), largely because recombinant enzyme cannot penetrate connective tissue. A method for treating Pompe disease using recombinant GAA is specifically described in U.S. Pat. No. 6,537,785 to Canfield. One of the main complications with ERT is the attainment and maintenance of therapeutically effective amounts of enzyme due to rapid degradation of the infused enzyme.

1-deoxynojirimycin and its salt, 1-deoxynojirimycin hydrochloride, acts as a pharmacological chaperone for mutant GAA by selectively binding to the enzyme, thereby increasing its stability and helping the enzyme fold into its correct three-dimensional shape. This stabilization of GAA allows the cell's quality control mechanisms to recognize the enzyme as properly folded so that trafficking of the enzyme to the lysosome is increased, allowing it to carry out its intended biological function, the metabolism of glycogen. As a result of restoring the proper trafficking of GAA from the ER to the lysosome, 1-deoxynojirimycin hydrochloride also reduces the accumulation of misfolded protein in the ER, which can alleviate stress on cells and some inflammatory-like responses that can be contributing factors in Pompe disease. Multiple *in vitro* and *in vivo* preclinical studies, as well as clinical studies, of 1-deoxynojirimycin hydrochloride have been conducted. 1-deoxynojirimycin hydrochloride has been shown to increase the amount of intracellular GAA protein and to enhance transport of mutant enzyme to the lysosome.

US 2006/264467 discloses a method for increasing the activity of a mutant or wild-type alpha-glucosidase enzyme by contacting the enzyme with a specific pharmacological chaperone which is a derivative of 1-deoxynojirimycin. The invention also provides a method for the treatment of Pompe disease.

US 2010/119502 provides a method for treating Pompe disease by administering a GAA enzyme in combination with an ASSC for the GAA enzyme.

## SUMMARY

According to an aspect of the invention, there is provided 1-deoxynojirimycin (1-DNJ), n-butyl deoxynojirimycin

(NB-DNJ), or a pharmaceutically acceptable salt thereof and an effective amount of human recombinant acid α-glucosidase enzyme replacement therapy for use in a method for the treatment of Pompe disease in a human subject; wherein: the 1-DNJ, the NB-DNJ, or the pharmaceutically acceptable salt thereof is administered in an amount of 250 mg or 600 mg and is administered from 4 hours prior to, to immediately before the administration of the human recombinant acid α-glucosidase enzyme replacement therapy.

According to another aspect of the invention, there is provided a kit containing instructions for the treatment of Pompe disease in a human subject, the kit comprising 250 mg or 600 mg of 1-deoxynojirimycin (1-DNJ), n-butyl deoxynojirimycin (NB-DNJ), or a pharmaceutically acceptable salt thereof, and an effective amount of human recombinant acid α-glucosidase enzyme replacement therapy, wherein the 1-DNJ, NB-DNJ, or pharmaceutically acceptable salt thereof is administered to the from 4 hours prior to, to immediately before the administration of the human recombinant acid α-glucosidase enzyme replacement therapy.

Preferred aspects of the invention are set out in the dependent claims.

## BRIEF DESCRIPTION OF THE DRAWINGS

**Figure 1A-B** shows composite graphs of rhGAA plasma activity in the four Cohort 1 subjects by treatment, as described by Example 2. Figure 1A shows rhGAA plasma activity following treatment with GAA ERT alone. Figure 1B shows rhGAA plasma activity following treatment with co-administration of GAA ERT and 50mg 1-deoxynojirimycin hydrochloride.

**Figure 2A-B** shows (A) the mean rhGAA plasma activity in the four Cohort 1 subjects by treatment, and (B) mean total rhGAA protein as measured by western blot in the four Cohort 1 subjects by treatment, as described by Example 2.

**Figure 3A-B** shows composite graphs of rhGAA plasma activity in the six Cohort 2 subjects by treatment, as described by Example 2. Figure 3A shows rhGAA plasma activity following treatment with GAA ERT alone. Figure 3B shows rhGAA plasma activity following treatment with co-administration of GAA ERT and 100mg 1-deoxynojirimycin hydrochloride.

**Figure 4** shows the mean rhGAA plasma activity in the six Cohort 2 subjects by treatment.

**Figure 5** shows the mean (SD) plasma 1-DNJ-HCL concentration-time profiles of Cohort 1 and 2 subjects by treatment.

**Figure 6** shows the total plasma rhGAA protein concentration profiles of Cohort 1 and 2 subjects by treatment, as measured by Western blot.

**Figure 7** shows urine Hex 4 levels by study day of Cohort 1 and 2 subjects, which was collected as part of the safety data.

**Figure 8** shows serum CPK levels by study day of Cohort 1 and 2 subjects, which was collected as part of the safety data.

**Figure 9A-B** shows Composite Plasma rhGAA Activity by Treatment Period for cohort 3 subjects.

**Figure 10A-D** shows AT2220-010 Mean (SD) Plasma rhGAA Activity for cohorts 1-4.

**Figure 11** shows AUC Stick Plot for rhGAA Activity in Plasma for cohort 3 subjects.

**Figure 12A-B** shows rhGAA Activity in Muscle from biopsies taken on Days 3 or 7 of Periods 1 and 2, and an optional baseline biopsy at follow up, for cohort 3 subjects.

**Figure 13** shows a Composite Muscle rhGAA Activity Summary by Treatment Period for cohort 1-3 subjects.

**Figure 14A-B** shows rhGAA Activity in PBMCs (peripheral blood mononuclear cells): Day 3 composites by treatment for cohort 3 subjects.

**Figure 15A-B** shows rhGAA Activity in PBMCs: Day 7 composites by treatment for cohort 3 subjects.

**Figure 16A-B** shows a Composite of plasma AT2220 Concentrations and a semi-log plot for cohort 3 subjects.

**Figure 17A-B** shows a Composite of Plasma rhGAA Activity by Treatment Period for cohort 4 subjects.

**Figure 18** shows AUC Stick Plot for rhGAA Activity in Plasma for cohort 4 subjects.

**Figure 19** shows a Composite Muscle rhGAA Activity Summary by Treatment Period for cohorts 1-4.

**Figure 20** shows Thigh Muscle MRI before (top) and after (bottom) ERT. At one year, 9.8% decrease in muscle mass in the anterior thigh, 11% decrease in muscle mass in posterior thigh, and +8.9% increase in intramuscular fat accumulation (6.6% subcutaneous fat).

**Figure 21** shows a summary of rhGAA Enzyme Activity in Plasma Area Under Curve (AUC) for cohorts 1-4.

**Figure 22** shows a summary of GAA Enzyme Activity in Muscle at Day 3 for cohorts 2-4.

**Figure 23** shows that the skeletal muscle distribution and half-life of N-butyl-DNJ (AT2221) is similar to 1-DNJ (AT2220); Cmax AT2220: 120 uM; AT2221: 140 uM. Eight-week old wild type C57BL/6 mice where administered an oral dose of 100mg/kg of 1-DNJ or N-butyl-DNJ. Plasma and tissue samples were taken at 0.5, 2, 4, 24, 48, 72,

96, 120, 144, & 168 hours post administration and the presence of drug was analyzed. The concentration of drug in plasma is expressed as ng/ml. The concentration of drug in tissue samples is expressed as ng/g.

**Figure 24** shows that N-butyl-DNJ (AT2221) and 1-DNJ (AT2220) have a similar effect on Phramacokinetics of rhGAA. Eight week old GAA KO mice were administered rhGAA (10 mg/kg IV). Oral AT2220 or AT2221 (100 mg/kg) was administered 30 min prior to GAA (Myozyme); Plasma samples were taken at predose, 0.08, 0.25, 0.50, 0.75,1, 2, 4, 8, and 24 hours after administration of GAA and the enzyme activity was determined. AT2220 and AT2221 increased the circulating half-life of rhGAA by at least ~2-fold.

**Figure 25** Western blot of recombinant GAA in plasma at 2, 8, and 24 hours after i.v. administration of GAA.

**Figure 26** Coadministration of DNJ or NB-DNJ with rhGAA has a similar effect on glycogen reduction. Twelve week old GAA KO mice were administered recombinant human GAA (Myozyme) 20 mg/kg i.v. every other week for 8 weeks. An oral dose of AT2220 or AT2221 (30 mg/kg) was administered 30 minutes prior to rhGAA Myozyme. Tissues were collected 21 days after the last dose of rhGAA and the level of glycogen (GAA substrate) was measured. n=5-mice/group; *p<0.05 vs. untreated t-test; #p<0.05 vs. Myozyme alone t-test; Dotted line indicates wild type glycogen levels. Cmax ~40 μM following administration of 30 mg/kg AT2220 or AT2221; equivalent to approximately 600 mg in humans.

## DETAILED DESCRIPTION

The present application discloses a dosing regimen and administration schedule for the use of 1-deoxynojirimycin and enzyme replacement therapy for the treatment of Pompe disease.

### Definitions

"Pompe disease," also referred to as acid maltase deficiency, glycogen storage disease type II (GSDII), and glycogenosis type II, is a genetic lysosomal storage disorder characterized by mutations in the GAA gene which metabolizes glycogen. As used herein, this term includes infantile-, juvenile- and adult-onset types of the disease.

"Acid α-glucosidase" (GAA) is a lysosomal enzyme which hydrolyzes α-1,4- and α-1,6-linked-D-glucose polymers present in glycogen, maltose, and isomaltose. Alternative names are as follows: glucoamylase; 1,4-α-D-glucan glucohydrolase; amyloglucosidase; gamma-amylase; and exo-1,4-α-glucosidase, and gamma-amylase.

The term "rhGAA" refers to human recombinant acid α-glucosidase. Non-limiting examples of rhGAA include alglucosidase alfa and those described in U.S. Pat. No. 7,560,424 and U.S. Pat. No. 7,396,811 to Lebowitz et al., U.S. Published Application Nos. 2009/0203575, 2009/0029467, 2008/0299640, 2008/0241118, 2006/0121018, and 2005/0244400 to Lebowitz et al., U.S. Pat. Nos. 7,423,135, 6,534,300, and 6,537,785; International Published Application No. 2005/077093 and U.S. Published Application Nos. 2007/0280925, and 2004/0029779.

The term "AUC" represents a mathematical calculation to evaluate the body's total exposure over time to a given drug. In a graph plotting how concentration in the blood after dosing, the drug concentration variable lies on the y-axis and time lies on the x-axis. The area between a drug concentration curve and the x-axis for a designated time interval is the AUC. AUCs are used as a guide for dosing schedules and to compare different drugs' availability in the body.

The term "$C_{max}$" represents the maximum plasma concentration achieved after dosing.

The terms "therapeutically effective dose" and "effective amount" refer to the amount of the specific pharmaceutical compound or composition that is sufficient to result in a beneficial therapeutic response. A beneficial therapeutic response can be any response that a user (*e.g.,* a clinician) will recognize as an effective response to the therapy, including the foregoing symptoms and surrogate clinical markers. Thus, a therapeutic response will generally be an amelioration of one or more symptoms of a disease or disorder, *e.g.*, Pompe disease, such as those known in the art for the disease or disorder, *e.g.*, for Pompe disease.

Non-limiting examples of symptoms or surrogate clinical markers of Pompe disease include: decreased GAA tissue activity; cardiomyopathy; cardiomegaly; progressive muscle weakness, especially in the trunk or lower limbs; profound hypotonia; macroglossia (and in some cases, protrusion of the tongue); difficulty swallowing, sucking, and/or feeding; respiratory insufficiency; hepatomegaly (moderate); laxity of facial muscles; areflexia; exercise intolerance; exertional dyspnea; orthopnea; sleep apnea; morning headaches; somnolence; lordosis and/or scoliosis; decreased deep tendon reflexes; lower back pain; and failure to meet developmental motor milestones.

The phrase "pharmaceutically acceptable" refers to molecular entities and compositions that are physiologically tolerable and do not typically produce untoward reactions when administered to a human. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions.

Suitable pharmaceutical carriers are described in "Remington's Pharmaceutical Sciences" by E.W. Martin, 18th Edition, or other editions.

"1-deoxynojirimycin" (DNJ) refers to (2R,3R,4R,5S)-2-hydroxymethyl-piperidine-3,4,5-triol. As used herein, reference to "1-deoxynojirimycin" or "DNJ" throughout includes both the free base and any pharmaceutically acceptable salt forms of the same. The hydrochloride salt of DNJ is known as "1-deoxynojirimycin hydrochloride" or "DNJ HCl."

"1-deoxynojirimycin derivative" or "1-DNJ derivative" or "DNJ derivative" refers to a compound with the following structure:

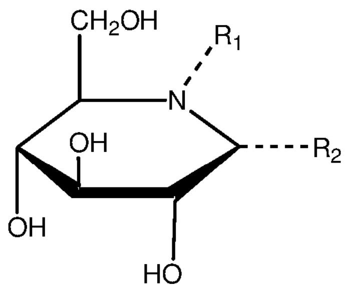

where $R_1$ is H or a straight or branched alkyl, cycloalkyl, alkenyl, alkoxyalkyl or aminoalkyl containing 1 - 12 carbon atoms, an aryl, alkylaryl, heteroaryl, or heteroaryl alkyl containing 5 - 12 ring atoms, where $R_1$ is optionally substituted with one or more -OH, -COOH, -Cl, -F, $-CF_3$, $-OCF_3$, -O-C(=O)N-$(alkyl)_2$; $R_2$ is H; a straight or branched alkyl, cycloalkyl, alkenyl, alkylaryl, or alkoxyalkyl, containing 1 - 9 carbon atoms or aryl containing 5 - 12 carbon atoms, wherein $R_2$ is optionally substituted with -OH, -COOH, $-CF_3$, $-OCF_3$ or a heterocyclic ring; and at least one of $R_1$ and $R_2$ is not H; or a pharmaceutically acceptable salt thereof.

"1-deoxynojirimycin derivative" or "1-DNJ derivative" or "DNJ derivative" may refer to (2R,3R,4R,5S)-1-butyl-2-(hydroxymethyl)piperidine-3,4,5-triol or miglustat or N-butyl-DNJ.

The term "adjuvant" or "adjuvant therapy" refers to any additional substance, treatment, or procedure used for increasing the efficacy, safety, or otherwise facilitating or enhancing the performance of a primary substance, treatment, or procedure.

The term "combination therapy" refers to any therapy wherein the results are enhanced as compared to the effect of each therapy when it is performed individually. The individual therapies in a combination therapy may be administered concurrently or consecutively.

Enhancement may include any improvement of the effect of the various therapies that may result in an advantageous result as compared to the results achieved by the therapies when performed alone. Enhanced effect and determination of enhanced effect may be measured by various parameters such as, but not limited to: temporal parameters (*e.g.*, length of treatment, recovery time, long-term effect of the treatment or reversibility of treatment); biological parameters (*e.g.,* cell number, cell volume, cell composition, tissue volume, tissue size, tissue composition); spatial parameters (*e.g.*, tissue strength, tissue size or tissue accessibility) and physiological parameters (*e.g.*, body contouring, pain, discomfort, recovery time or visible marks). Enhanced effect may include a synergistic enhancement, wherein the enhanced effect is more than the additive effects of each therapy when performed by itself. Enhanced effect may include an additive enhancement, wherein the enhanced effect is substantially equal to the additive effect of each therapy when performed by itself. Enhanced effect may include less than a synergistic effect, wherein the enhanced effect is lower than the additive effect of each therapy when performed by itself, but still better than the effect of each therapy when performed by itself.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Numerical quantities given herein are approximate unless stated otherwise.

## Formulation and Administration

1-deoxynojirimycin can be administered as the free base or as a pharmacologically acceptable salt form, including 1-deoxynojirimycin hydrochloride. It can be administered in a form suitable for any route of administration, including *e.g.,* orally in the form tablets, capsules, or liquid, or in sterile aqueous solution for injection. It can be administered orally in the form of tablets, capsules, ovules, elixirs, solutions or suspensions, gels, syrups, mouth washes, or a dry powder for constitution with water or other suitable vehicle before use, optionally with flavoring and coloring agents for immediate-, delayed-, modified-, sustained-, pulsed-or controlled-release applications. Solid compositions such as tablets, capsules, lozenges, pastilles, pills, boluses, powder, pastes, granules, bullets, or premix preparations can also be used. Solid and liquid compositions for oral use can be prepared according to methods well known in the art. Such compositions can also contain one or more pharmaceutically acceptable carriers and excipients which can be in solid or liquid form. When the compound is formulated for oral administration, the tablets or capsules can be prepared by conventional means with pharmaceutically acceptable excipients such as binding agents (*e.g.*, pregelatinized starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.*, sodium lauryl sulphate). The tablets can be coated by methods well known in the art.

The pharmaceutically acceptable excipients also include, but are not limited to, microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrolidone, hydroxypropyl ethylcellulose (HPMC), hydroxypropyl cellulose (HPC), sucrose, gelatin, and acacia. Additionally, lubricating agents such as magnesium stearate, stearic acid, glyceryl behenate and talc can be included.

In a specific embodiment, 1-deoxynojirimycin hydrochloride is formulated with magnesium stearate and pregelatinized starch in a white, hard gelatin capsule.

**Enzyme Replacement Therapy**

The current approved treatment for Pompe disease is enzyme replacement therapy. Two alglucosidase alfa products are currently available for the treatment of Pompe disease: Myozyme® (Genzyme Corporation) and Lumizyme® (Genzyme Corporation). These two forms of ERT are intended to compensate for a patient's inadequate GAA activity with a recombinant form of the enzyme, administered intravenously. While ERT is effective in many settings, the rhGAA has a short circulating half-life, low tissue uptake, and can elicit immune responses that adversely affect tolerability and efficacy

The recommended dosage of alglucosidase alfa is 20 mg/kg body weight infused every 2 weeks as an intravenous infusion. This infusion is administered over a 4-hour period.

1-deoxynojirimycin hydrochloride stabilizes rhGAA *in vitro* and *in vivo.* Binding of 1-deoxynojirimycin hydrochloride to rhGAA results in significant concentration-dependent increases in the physical stability of the enzyme in a neutral pH buffer, as measured by thermal denaturation and by enzymatic activity. In addition, the stability of rhGAA in human blood is significantly increased when incubated with 1-deoxynojirimycin hydrochloride, as measured by activity (half-life is approximately 6 hours in the absence of 1-deoxynojirimycin hydrochloride, whereas in the presence of 1-deoxynojirimycin hydrochloride there was no measureable loss in enzymatic activity over a 24 hour period).

In rats, bolus intravenous administration of 10 mg/kg rhGAA 30 minutes after a single oral administration of 3 or 30 mg/kg 1-deoxynojirimycin hydrochloride resulted in 1.5- and 2.1-fold increases in the circulating half-life of rhGAA, respectively, as measured by activity and Western blotting. Similar effects were seen on the circulating half-life of rhGAA when 1-deoxynojirimycin hydrochloride (3 or 30 mg/kg PO) was administered to rats, followed 30 minutes later by a 1-hour intravenous infusion of rhGAA (10 mg/kg). Importantly, these doses of 1-deoxynojirimycin hydrochloride result in plasma exposure levels in the rat that are comparable to those that can be achieved following oral administration of 50 or 600 mg 1-deoxynojirimycin hydrochloride, respectively, to humans.

In GAA knock-out mice, oral administration of 10, 100, or 1000 mg/kg 1-deoxynojirimycin hydrochloride 30 minutes before and 8, 16, and 24 hours after bolus intravenous administration of rhGAA (10 mg/kg, once per week for up to 1, 2 or 3 weeks) resulted in significant and dose-dependent increases in tissue GAA levels as measured by activity and Western blotting 2, 4, and 7 days post-administration. Co-administration of 10 mg/kg 1-deoxynojirimycin hydrochloride (which yields exposure in mice that is comparable to that seen in humans following administration of about 200 mg) resulted in maximal GAA increases that were up to 2.5-, 2.3-, 2.2-, 4.0-, and 1.7-fold greater in heart, diaphragm, quadriceps, gastrocnemius, and triceps, respectively, compared to those seen following administration of rhGAA alone. Similar results were seen in wild-type C57BL/6 mice following a single administration of 3, 30, or 100 mg/kg 1-deoxynojirimycin hydrochloride 30 minutes prior to a single bolus intravenous administration of rhGAA (10 mg/kg). Importantly, the 3, 10, and 30 mg/kg doses of 1-deoxynojirimycin hydrochloride result in plasma exposure levels in the mouse that are comparable to those that can be achieved following oral administration of 50, 150 or 600 mg 1-deoxynojirimycin hydrochloride, respectively, to humans.

The effect of a single oral administration of 1-deoxynojirimycin hydrochloride on rhGAA tissue uptake was studied in GAA knock-out mice. Bolus intravenous administration of rhGAA (20 mg/kg) 30 minutes after a single oral administration of 1-deoxynojirimycin hydrochloride (30 mg/kg every other week for 8 weeks; four administrations total) resulted in a significant increase in GAA activity measured 7 days following the last injection. GAA activity increases were approximately 2.1-, 2.0-, 1.5-, 1.7-, 1.6-, and 2.0-fold greater following 1-deoxynojirimycin hydrochloride co-administration compared to administration of rhGAA alone, in heart, diaphragm, gastrocnemius, quadriceps, triceps and tongue,

respectively. These data indicate that in GAA knock-out mice, administration of 30 mg/kg 1-deoxynojirimycin hydrochloride prior to administration of rhGAA every other week for 8 weeks yields significantly greater rhGAA tissue uptake as compared to those seen following rhGAA administration alone.

Repeat rhGAA administration studies were conducted in GAA knock-out mice to assess effects on tissue glycogen levels. Bolus intravenous administration of rhGAA (20 mg/kg) 30 minutes after a single oral administration of 1-deoxynojirimycin hydrochloride (10 or 30 mg/kg once every other week for 8 weeks resulted in a dose-dependent reduction in tissue glycogen levels measured 21 days following the last injection. Glycogen reduction was approximately 2.3-, 1.6-, 2.6-, 2.7-, 2.2-, 1.2-, 1.4-, and 1.3-fold greater in heart, diaphragm, quadriceps, gastrocnemius, triceps, soleus, biceps, and tongue, respectively, following 1-deoxynojirimycin hydrochloride co-administration compared to rhGAA alone, respectively. Similar effects were seen on tissue glycogen levels following four biweekly oral administrations of 1-deoxynojirimycin hydrochloride (30 mg/kg) followed 30 minutes later by bolus intravenous administration of rhGAA (40 mg/kg).

## EXAMPLES

### EXAMPLE 1: Dosing Regimen for the Treatment of Pompe Disease using 1-Deoxynojirimycin Hydrochloride and Alglucosidase Alfa

One objective of the study is to evaluate the safety, effectiveness, and pharmacodynamics of dose regimens comprising co-administering 1-deoxynojirimycin hydrochloride (also referred to as AT2220) and alglucosidase alfa in patients with Pompe disease.

Another objective of the study is to assess the effects of various doses of 1-deoxynojirimycin hydrochloride on the GAA activity. This will be evaluated by measuring the GAA activity in muscle after dosing with alglucosidase alfa alone and alglucosidase alfa in combination with 1-deoxynojirimycin hydrochloride, at 3 and/or 7 days after dosing, by measuring GAA activity and protein levels.

WBC (PBMC) GAA activity and protein levels will be measured, as well as anti-rhGAA antibody titer before initiation of an infusion of alglucosidase alfa. All plasma, WBC and muscle measurements of GAA enzyme activity are performed with and without Con A capture and determination of protein levels is by Western blot.

***Study Design.*** This is a Phase 2 clinical, single-dose, open-label study to assess the safety and effectiveness of co-administering 1-deoxynojirimycin hydrochloride and alglucosidase alfa. The study will be conducted in male and female subjects between 18 and 65 years of age who have been receiving a stable dose of alglucosidase alfa for at least one month before study entry. Approximately 16 subjects will be enrolled.

This study will be to evaluate the safety and effect of ascending doses (50, 100, 250 and 600 mg) of 1-deoxynojirimycin hydrochloride administered 1 hour before initiation of an infusion of alglucosidase alfa on the pharmacokinetics of GAA. Four subjects will be enrolled at each of the four 1-deoxynojirimycin hydrochloride dose levels. Each cohort of four subjects will receive a single intravenous infusion of alglucosidase alfa alone, followed two to four weeks later by a single oral dose of 1-deoxynojirimycin hydrochloride administered 1 hour before initiation of an intravenous infusion of alglucosidase alfa.

Dose escalation to the next dose level of 1-deoxynojirimycin hydrochloride may proceed following review of safety and tolerability data from the previous dose level group(s). Safety data reviewed will include adverse events (including infusion reactions), clinical laboratory tests (including creatine kinase, LDH (LDH-5), alkaline phosphatase, AST, ALT), Hex4, 12-Lead ECGs, physical examinations, muscle strength tests and vital signs.

Each subject will receive alglucosidase alfa alone as an intravenous infusion in Period 1 and a single dose of 1-deoxynojirimycin hydrochloride one hour before initiation of an intravenous infusion of alglucosidase alfa in Period 2. The dose of alglucosidase alfa administered in Periods 1 and 2 will be identical.

Each cohort will consist of 4 subjects. Subjects will be enrolled sequentially to one of the four dosing cohorts to 1-deoxynojirimycin hydrochloride at the following dose levels in Period 2:

Cohort 1: A single 50 mg oral dose of 1-deoxynojirimycin hydrochloride;
Cohort 2: A single 100 mg oral dose of 1-deoxynojirimycin hydrochloride;
Cohort 3: A single 250 mg oral dose of 1-deoxynojirimycin hydrochloride;
Cohort 4: A single 600 mg oral dose of 1-deoxynojirimycin hydrochloride.

For Period 1, prior to their next scheduled alglucosidase alfa infusion, subjects will have the following assessments performed: adverse event assessment, concomitant medications, physical exam, weight, vital signs, 12-lead ECG, clinical laboratory tests (including creatine analysis, LDH (LDH-5), alkaline phosphatase, AST, ALT), Hex4 and muscle strength tests.

On the morning of Day 1, the subject's current alglucosidase alfa dose will be administered as an infusion using

an infusion pump. The infusion rate (and any changes in rate during the infusion), infusion duration and dose of alglucosidase alfa administered should be identical in Periods 1 and 2. Blood samples for pharmacokinetic analysis will be collected immediately before initiation of the alglucosidase alfa infusion and over a 24-hour period after initiation of the alglucosidase alfa infusion. Plasma and WBC GAA enzyme activity and plasma anti-rhGAA antibody titer will be determined from the collected blood samples at the times summarized in Table 2. A 12-lead ECG will be performed at the end of the alglucosidase alfa infusion, immediately after collection of the post-infusion blood sample.

After collection of the last pharmacokinetic sample, the following assessments will be performed: adverse event assessment (including infusion reactions), concomitant medications, vital signs, 12-lead ECG, clinical laboratory tests (including creatine kinase, LDH (LDH-5), alkaline phosphatase, AST, ALT), Hex4 and muscle strength tests.

On Day 7, subjects will have the following assessments performed: adverse event assessment, concomitant medications, physical exam, vital signs, clinical laboratory tests (including creatine kinase, LDH (LDH-5), alkaline phosphatase, AST, ALT), Hex4 and muscle strength tests. A muscle biopsy will be collected from which GAA enzyme activity will be determined. A blood sample for plasma and WBC GAA enzyme level determinations will also be collected.

A muscle biopsy will will also be collected from which GAA enzyme activity and 1-deoxynojirimycin hydrochloride levels will be determined, on Day 3.

For Period 2, prior to their next scheduled alglucosidase alfa infusion, approximately 2 weeks after the administration of the alglucosidase alfa infusion in Period 1, subjects will have the following assessments performed: adverse event assessment, concomitant medications, physical exam, weight, vital signs, 12-lead ECG, clinical laboratory tests (including creatine analysis, LDH (LDH-5), alkaline phosphatase, AST, ALT), Hex4 and muscle strength tests.

On the morning of Day 1, an oral dose of 1-deoxynojirimycin hydrochloride will be administered 1 hour prior to the scheduled alglucosidase alfa infusion. Subjects will fast for at least 2 hours before and 2 hours after 1-deoxynojirimycin hydrochloride administration. The infusion rate (and any changes in rate during the infusion), infusion duration and dose of alglucosidase alfa administered should be identical in Periods 1 and 2.

Blood samples for pharmacokinetic analysis will be collected before dosing 1-deoxynojirimycin hydrochloride and at 1 hour after administration of 1-deoxynojirimycin hydrochloride (i.e., immediately before initiation of the alglucosidase alfa infusion) and over the 24-hour period after initiation of the alglucosidase alfa infusion. Plasma and WBC GAA enzyme activity, plasma 1-deoxynojirimycin hydrochloride concentrations and plasma anti-rhGAA antibody titer will be determined from the collected blood samples at the times summarized in Table 2. A 12-lead ECG will be performed at the end of the alglucosidase alfa infusion, immediately after collection of the post-infusion blood sample.

After collection of the last pharmacokinetic sample, the following assessments will be performed: adverse event assessment (including infusion reactions), concomitant medications, vital signs, 12-lead ECG, clinical laboratory tests (including creatine kinase, LDH (LDH-5), alkaline phosphatase, AST, ALT), Hex4 and muscle strength tests.

On Day 7, subjects will have the following assessments performed: adverse event assessment, concomitant medications, physical exam, vital signs, clinical laboratory tests (including creatine kinase, LDH (LDH-5), alkaline phosphatase, AST, ALT), Hex4 and muscle strength tests. A muscle biopsy will be collected from which GAA enzyme activity and 1-deoxynojirimycin hydrochloride levels will be determined. A blood sample for plasma and WBC GAA enzyme level determinations will also be collected.

A muscle biopsy will will also be collected from which GAA enzyme activity and 1-deoxynojirimycin hydrochloride levels will be determined, on Day 3.

In the follow-up 26 to 30 days after dosing with 1-deoxynojirimycin hydrochloride and alglucosidase alfa in Period 2, subjects will have the following assessments performed: adverse event assessment, concomitant medications, physical exam, vital signs, 12-lead ECG, clinical laboratory tests (including creatine kinase, LDH (LDH-5), alkaline phosphatase, AST, ALT), Hex4, muscle strength tests and anti-rhGAA antibody titers.

***Assessment and Sample Collection Schedules.*** Table 1 shows the schedule of assessments for Periods 1 and 2. Sample collection times and analytes for co-administration of 1-deoxynojirimycin hydrochloride with alglucosidase alfa are shown in Table 2.

**Table 1: Schedule of Assessments**

| | Screening | Periods 1 and 2 | | | | Follow-up |
|---|---|---|---|---|---|---|
| Activity | Within 28 days of Enrollment | Day -1 | Day 1 | Day 2 | Day 7 | 26-30 days after dosing in Period 2 |
| Informed Consent | X | | | | | |
| Medical History and Demographic Data | X | | | | | |
| Physical Exam | X | X | | | X | X |

(continued)

| Activity | Screening | Periods 1 and 2 | | | | Follow-up |
|---|---|---|---|---|---|---|
| | Within 28 days of Enrollment | Day -1 | Day 1 | Day 2 | Day 7 | 26-30 days after dosing in Period 2 |
| ECG (12-lead) | X | X | X | X | | X |
| Vital Signs[1] | X | X | X | X | X | X |
| Hematology | X | X | | X | X | X |
| Urinalysis | X | X | | X | X | X |
| Serum Chemistry | X | X | | X | X | X |
| Hex4 | X | X | | X | X | X |
| Record of Concomitant Medication | X | X | X | X | X | X |
| eGFR | X | | | | | |
| Drug Dose | | | X[2] | | | |
| PK Blood Sampling[3] | | | X | X | X | |
| Muscle Strength Test (dynamometer) | X | X | | X | X | X |
| Muscle Biopsy | | | | | X | |
| Antibody Titer | | | X | | | X |
| Adverse Events | | X | X | X | X | X |

[1] Vital signs include temperature, blood pressure, heart rate and respiration.
[2] Drug(s) administered: alglucosidase alfa (Periods 1 and 2), 1-deoxynojirimycin hydrochloride (Period 2).
[3] Sample collection times and analytes are summarized in Table 2.

**Table 2: Blood and Muscle Collection Times and Sample Analysis**

**Period 1**

| Sample Collected | | Predose | Post-Initiation of Alglucosidase alfa Infusion (hr) 1 | 2 | 3 | 4 | 5 | 6 | 7 | 8 | 9 | 10 | 12 | 24 | Day 7 |
|---|---|---|---|---|---|---|---|---|---|---|---|---|---|---|---|
| **Blood** | | X | X | X | X | X | X | X | X | X | X | X | X | X | X |
| **Muscle** | | | | | | | | | | | | | | | X |
| **Alglucosidase alfa infusion**[1] | | | <--------------------> | | | | | | | | | | | | |
| **Plasma** | Plasma GAA | X | X | X | X | X | X | X | X | X | X | X | X | X | X |
| | WBC GAA | X | | | | X | | | | | | | | X | X |
| | Antibody | X | | | | | | | | | | | | | |
| **Muscle** | GAA | | | | | | | | | | | | | | X |

**Period 2**

| Sample Collected | | Predose DNJ HCl | 0[2] | Post-Initiation of Alglucosidase alfa Infusion (hr) 1 | 2 | 3 | 4 | 5 | 6 | 7 | 8 | 9 | 10 | 12 | 24 | Day 7 |
|---|---|---|---|---|---|---|---|---|---|---|---|---|---|---|---|---|
| **Blood** | | X | X | X | X | X | X | X | X | X | X | X | X | X | X | X |
| **Muscle** | | | | | | | | | | | | | | | | X |
| **Alglucosidase alfa infusion**[1,3] | | | | <-------------------------> | | | | | | | | | | | | |
| | **DNJ HCl Dose** | | X | | | | | | | | | | | | | |
| **Plasma** | Plasma GAA | X | X | X | X | X | X | X | X | X | X | X | X | X | X | X |
| | WBC GAA | X | X | | | | X | | | | | | | | X | X |
| | Antibody | X | | | | | | | | | | | | | | |
| | DNJ HCl | X | X | X | X | X | X | X | X | X | X | X | X | X | X | X |
| **Muscle** | GAA | | | | | | | | | | | | | | | X |
| | DNJ HCl | | | | | | | | | | | | | | | — X |

[1] Duration of infusion for individual subjects may differ from 4 hours, however, infusion duration and infusion rate should be identical in both periods. An additional blood sample immediate before termination of the infusion (to be assayed for plasma GAA only) will be collected if the duration of infusion varies from 4 hours and the end of infusion does not coincide with a pre-established blood sample.

[2] Sample collected 1 hour after 1-deoxynojirimycin hydrochloride dose, immediately before initiation of alglucosidase alfa infusion.

[3] Alglucosidase alfa infusion started one hour after administration of 1-deoxynojirimycin hydrochloride.

***1-Deoxynojirimycin Hydrochloride and GAA Plasma Pharmacokinetics.*** Concentrations of 1-deoxynojirimycin hydrochloride in blood samples will be measured in plasma using a validated LC-MS/MS assay. GAA activity in plasma will be determined by a validated assay measuring enzyme activity using 4-MUG, with and without Con A. GAA protein levels will be measured by Western blotting using anti-human GAA antibody.

***GAA Enzyme Activity and 1-Deoxynojirimycin Hydrochloride Levels in Muscle.*** GAA enzyme activity will be examined in muscle biopsy samples. One piece of muscle tissue will be removed as described in Table 1. GAA activity in muscle will be determined by a validated assay measuring enzyme activity using 4-MUG, with and without Con A. GAA protein levels will be measured by Western blotting using anti-human GAA antibody. 1-deoxynojirimycin hydrochloride concentrations in muscle samples collected in Period 2 will be determined using a validated LC-MS/MS assay.

***WBC (PBMC)GAA Activity.*** GAA activity in WBCs will be determined in blood samples by a validated assay measuring enzyme activity using 4-MUG, with and without Con A. GAA protein levels will be measured by Western blotting using anti-human GAA antibody.

***Ant-rhGAA Antibody Titer.*** Blood samples will be collected and anti-rhGAA antibody titer will be measured in the samples described in Table 2.

***Safety Parameters.*** Safety parameters will be assessed by review of changes in physical exam findings, vital signs, ECG changes over time, clinical labs, Hex4 and adverse events.

***Vital Signs, Weight and Height.*** Body temperature and respirations will be measured at screening and check-in. To monitor safety, body temperature, respiration, seated blood pressure and heart rate will be measured before dosing and approximately 1, 2, 3, 4, and 6 hours following administration of alglucosidase alfa (Period 1) or 1-deoxynojirimycin hydrochloride (Periods 2), and on the days described in Table 1. Where the time of vital sign monitoring coincides with a blood draw, the blood draw takes precedence and the vital signs will be adjusted accordingly.

***ECG Monitoring.*** ECG monitoring will be performed with a standard 12-lead ECG.

***Clinical Laboratory Tests.*** Blood samples for clinical laboratory tests (hematology, serum chemistry) and urinalyses will be collected according to the schedule in Table 1.

- Hematology tests include total hemoglobin, hematocrit, erythrocyte, platelet and leukocyte counts with differential.
- Coagulation (screening only) includes INR and aPTT.
- Serum chemistry includes measurement of AST, ALT, alkaline phosphatase, total bilirubin, creatinine, creatine kinase, urea, glucose, calcium, sodium, potassium, magnesium, total protein, albumin, bicarbonate, LDH (LDH-5), blood urea nitrogen, chloride, and phosphate.
- Urinalysis includes color, appearance, specific gravity, pH, protein, glucose, ketones, blood, leukocyte esterase, nitrite, bilirubin, urobilinogen and microscopy of sediment

***Urinary Tetrasaccharides (Hex4).*** Urine samples for Hex4 determinations will be collected at the times indicated in Table 1.

***Muscle Strength Tests (Handheld Dynamometer).*** Muscular strength tests, assessed by hand held dynamometer, will be conducted at Screening, Days -1 and 7 of each period and at Follow-up. Proximal and distal muscle groups will be evaluated.

***Pharmacokinetic Parameters.*** Non-compartmental pharmacokinetic parameters of $AUC_{0\text{-}t}$, $AUC_{infinity}$, $C_{max}$, $t_{max}$, $k_{el}$ and half-life will be calculated from plasma 1-deoxynojirimycin hydrochloride concentrations and plasma rhGAA enzyme levels. Pharmacokinetic parameters will be summarized by treatment using descriptive statistics. The $AUC_{0\text{-}t}$, $AUC_{infinity}$ ratios for GAA enzyme activity alone or after administration with 1-deoxynojirimycin hydrochloride will be calculated. Pharmacokinetic and pharmacodynamic data for those subjects receiving Myozyme® and Lumizyme® will be analyzed separately.

***Statistical Analysis.*** The descriptive statistics (N, mean, standard deviation, and coefficient of variation, standard error, median, minimum and maximum) will be provided as appropriate. The effect of 1-deoxynojirimycin hydrochloride on GAA enzyme activity will be evaluated by calculation of the individual (by subject) AUC and $C_{max}$ ratios as follows:

$$\text{AUC Ratio} = \frac{AUC_{infinity\ (combination)}}{AUC_{infinity(alone)}}$$

$$\text{Cmax Ratio} = \frac{C_{max\,(combination)}}{C_{max(alone)}}$$

The AUC and $C_{max}$ ratios will be expressed as a mean of the individual ratios and 90% confidence interval for the mean. Pharmacokinetic and pharmacodynamic data for those subjects receiving Myozyme® and Lumizyme® will be analyzed separately. GAA activity in muscle with and without co-administration of 1-deoxynojirimycin hydrochloride will be compared. Results will be presented in tabular and graphic forms, as appropriate. All subjects who will be dosed with study medication and have sufficient data to generate reliable pharmacokinetic parameters will be included in the safety and pharmacokinetic analysis.

It is further to be understood that all values are approximate, and are provided for description.

**REFERENCE EXAMPLE 2: Dosing Regimen for the Treatment of Pompe Disease using 1-Deoxynojirimycin Hydrochloride and Alglucosidase Alfa -- Cohorts 1 and 2**

One objective of the study was to evaluate the safety, effectiveness, and pharmacodynamics of dose regimens comprising co-administering 1-deoxynojirimycin hydrochloride and alglucosidase alfa in patients with Pompe disease.

Another objective of the study was to assess the effects of various doses of 1-deoxynojirimycin hydrochloride on the GAA activity. This was evaluated by measuring the GAA enzyme activity and protein levels in skeletal muscle at Day 3 and/or Day 7 following a single intravenous infusion with alglucosidase alfa alone and after pre-administration of single ascending oral doses of 1-deoxynojirimycin hydrochloride.

***Methods.*** The study was conducted essentially according to the methods described in Example 1. Cohort 1 comprised 4 subjects. Cohort 2 comprised 6 subjects. Each subject received alglucosidase alfa alone as an intravenous infusion in Period 1 and a single 50 mg dose (Cohort 1) or 100 mg (Cohort 2) of 1-deoxynojirimycin hydrochloride one hour before initiation of an intravenous infusion of alglucosidase alfa in Period 2. The genotype, including nucleotide and amino acid changes, for each subject (where available) is shown below:

GAA Genotypes

| Patient ID | Nucleotide Change | Amino Acid Change |
|---|---|---|
| A | c.-32-13T>G | Splicing Mutation |
| **B** | Not Available | - |
| C | Not Available | - |
| D | c.1222A>G/c.-32-13T>G | M408V/Splicing Mutation |
| E | c.-32-13T>G | Splicing Mutation |
| F | Not Available | - |
| G | c.-32-13T>G/c.1445C>G | Splicing Mutation / P482R |
| H | Not Available | - |
| I | c.692+5G>T/c.1211A>G | Splicing Mutation / D404G |
| J | Not Available | - |

Results: Cohort 1

<u>Plasma rhGAA activity increases with co-administration of 1-deoxynojirimycin hydrochloride and acid α-glucosidase relative to acid α-glucosidase alone</u>

For co-administration of acid α-glucosidase with 50mg 1-deoxynojirimycin hydrochloride (Period 2) relative to ERT alone (Period 1), a 1.5-fold mean increase in plasma rhGAA activity AUC (Area Under the Curve) was observed, as shown in Table 3. Table 3 also shows the fold change in rhGAA activity between Periods 1 and 2 in Day 7 muscle biopsies of Cohort 1 subjects.

Table 3. Mean Plasma rhGAA Activity PK Summary for Cohort 1 (4 subjects):

| Treatment | $C_{max}$[a] (nmol/hr/mL) | $T_{max}$[b] (hr) | $AUC_{0-T}$[a] (hr*nmol/hr/mL) | $AUC_{0-\infty}$[a] (hr*nmol/hr/mL) | T½[c] (hr) | $F_{rel}$[d] |
|---|---|---|---|---|---|---|
| 20 mg/kg GAA Alone | 16441 (31.6) | 5.0 (4 - 5) | 105834 (30.7) | 107707 (31.1) | 3.9 (0.6) | - |
| 20 mg/kg GAA + 50 mg AT2220 | 19922 (24.4) | 5.0 (4 - 6) | 152207 (21.3) | 157162 (22.4) | 4.2 (0.8) | 1.5 |

[a] Geometric mean (CV%)
[b] Median (range)
[c] Arithmetic mean (SD)
[d] $F_{rel}$ = Geometric mean of the $AUC_{0-\infty}$ ratios

| Treatment | Period | Biopsy Day | Muscle GAA Activity (pmol/mg/hr) | Fold change |
|---|---|---|---|---|
| rhGAA Alone | 1 | 7 | 1243 (302) | |
| rhGAA + 50 mg AT2220 | 2 | 7 | 1211 (216) | 1.02 (0.30) |

For co-administration of acid α-glucosidase with 50mg 1-deoxynojirimycin hydrochloride (Period 2) relative to ERT alone (Period 1), individual subject increases in plasma rhGAA activity AUC were 1.2-, 1.5-, 1.5-, and 1.6-fold, as shown in Tables 4-7. Tables 4-7 also show the fold change in rhGAA activity between Periods 1 and 2 in Day 7 muscle biopsies.

Table 4. rhGAA Activity in Plasma and Muscle for Subject 2031-0101 (Patient A) (Cohort 1)

| **Treatment [total dose (mg), duration of infusion (hr:min), infusion rate (mL/hr)]** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **T½ (hr)** | **$F_{rel}$** |
|---|---|---|---|---|---|---|
| 20.2 mg/kg GAA Alone (1450, 4:59, 112) | 17415 | 5.0 | 109403 | 111239 | 3.9 | |
| 20.2 mg/kg GAA + 50 mg AT2220 (1450, 5:03, 112) | 20725 | 5.0 | 165304 | 170617 | 4.3 | 1.5 |

Body Weight:71.8 kg

| **Treatment** | **Period** | **Biopsy Day** | **Muscle GAA Activity (pmol/mg/hr)** | **Fold change** |
|---|---|---|---|---|
| rhGAA Alone | 1 | 7 | 1352.8 | |
| rhGAA + 50 mg AT2220 | 2 | 7 | 1398.5 | 1.03 |

Table 5. rhGAA Activity in Plasma and Muscle for Subject 2031-0102 (Patient B) (Cohort 1)

| **Treatment [total dose (mg), duration of infusion (hr:min), infusion rate (mL/hr)]** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **T½ (hr)** | **$F_{rel}$** |
|---|---|---|---|---|---|---|
| 19.5 mg/kg GAA Alone (1750, 4:58, 112) | 19139 | 5.0 | 125684 | 126956 | 3.3 | |
| 19.5 mg/kg GAA + 50 mg AT2220 (1750, 5:10, 112) | 19321 | 6.0 | 179229 | 190630 | 5.3 | 1.5 |

Body weight: 89.8 kg

| **Treatment** | **Period** | **Biopsy Day** | **Muscle GAA Activity (pmol/mg/hr)** | **Fold change** |
|---|---|---|---|---|
| rhGAA Alone | 1 | 7 | 1010.5 | |
| rhGAA + 50 mg AT2220 | 2 | 7 | 956.3 | 0.95 |

Table 6. rhGAA Activity in Plasma and Muscle for Subject 2031-0103 (Patient C) (Cohort 1)

| Treatment [total dose (mg), duration of infusion (hr:min), infusion rate (mL/hr)] | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | T½ (hr) | $F_{rel}$ |
|---|---|---|---|---|---|---|
| 20.6 mg/kg GAA Alone (1150, 5:00, 112) | 10495 | 5.0 | 68617 | 69526 | 3.7 | |
| 20.6 mg/kg GAA + 50 mg AT2220 (1150, 5:04, 112) | 14808 | 5.0 | 111781 | 114447 | 4 | 1.6 |

Body Weight: 55.8 kg

| Treatment | Period | Biopsy Day | Muscle GAA Activity (pmol/mg/hr) | Fold change |
|---|---|---|---|---|
| rhGAA Alone | 1 | 7 | 988.4 | |
| rhGAA + 50 mg AT2220 | 2 | 7 | 1383.1 | 1.40 |

Table 7. rhGAA Activity in Plasma and Muscle for Subject 6003-0101 (Patient D) (Cohort 1)

| Treatment [total dose (mg), duration of infusion (hr:min), infusion rate (mL/hr)] | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | T½ (hr) | $F_{rel}$ |
|---|---|---|---|---|---|---|
| 19.7 mg/kg GAA Alone (2150, 3:50, 175) | 20889 | 4.0 | 132970 | 137064 | 4.6 | |
| 19.7 mg/kg GAA + 50 mg AT2220 (2150, 3:50, 175) | 26568 | 4.0 | 162063 | 163896 | 3.4 | 1.2 |

Body weight 109.0 kg

| Treatment | Period | Biopsy Day | Muscle GAA Activity (pmol/mg/hr) | Fold change |
|---|---|---|---|---|
| rhGAA Alone | 1 | 7 | 1620.4 | |
| rhGAA + 50 mg AT2220 | 2 | 7 | 1109.0 | 0.68 |

Table 8 below shows the cumulative AUCs for rhGAA activity in plasma of cohort 1.

Table 8.

| Time (hr) | 2031-0101 | | 2031-0102 | | 2031-0103 | | 6003-0101 | |
|---|---|---|---|---|---|---|---|---|
| | Period 1 | Period 2 | Period 1 | Period 2 | Period 1 | Period 2 | Period 1 | Period 2 |
| 0 | 0 | 0 | 0 | 0 | 0 | 0 | 0 | 0 |
| 1 | 995 | 797 | 1498 | 1317 | 1094 | 840 | 1528 | 1296 |
| 2 | 5514 | 4620 | 7422 | 7154 | 4549 | 4462 | 8617 | 7784 |
| 3 | 14322 | 13802 | 18433 | 18370 | 10513 | 12120 | 23531 | 21747 |
| 4 | 27215 | 28049 | 33967 | 33603 | 19342 | 23519 | 43327 | 43801 |
| 5 | 43537 | 46504 | 52486 | 50460 | 29815 | 37448 | 62700 | 67755 |
| 6 | 58363 | 66269 | 68174 | 68442 | 39357 | 51079 | 76972 | 87223 |
| 7 | 69498 | 83355 | 79546 | 86144 | 46438 | 62230 | 86420 | 102711 |
| 8 | 78151 | 97251 | 88568 | 100655 | 51167 | 71026 | 93589 | 114607 |
| 9 | 84748 | 109173 | 95606 | 112503 | 54568 | 77778 | 99912 | 123747 |
| 10 | 89665 | 119039 | 101047 | 122588 | 57196 | 83434 | 105495 | 131397 |
| 12 | 96302 | 133401 | 109601 | 138339 | 61036 | 92644 | 113716 | 142712 |
| 24 | 109403 | 165304 | 125684 | 179229 | 68617 | 111781 | 132970 | 162063 |

Table 9 shows the Total rhGAA Protein in Plasma PK Summary by Western Blot (Cohort 1)

Table 9.

| Period 1 (Treatment = rhGAA Alone) | | | | | |
|---|---|---|---|---|---|
| **SubjID** | **$C_{max}$ (ng/mL)** | **$T_{max}$ (hr)** | **$AUC_{0-T}$ (ng*hr/mL)** | **$AUC_{0-\infty}$ (ng*hr/mL)** | **T½ (hr)** |
| 2031-0101 | 197632 | 5 | 1573875 | 1842902 | 8.9 |
| 2031-0102 | 240858 | 5 | 2035347 | 2235425 | 6.9 |
| 2031-0103 | 266201 | 5 | 1907938 | 2142094 | 2.7 |
| 6003-0101 | 507039 | 5 | 2513158 | 2523836 | 0.8 |
| **N** | **4** | **4** | **4** | **4** | **4** |
| **Mean** | **302932** | **5.0** | **2007580** | **2186064** | **4.8** |
| **SD** | **138984** | **0.0** | **389191** | **280606** | **3.7** |
| **Min** | **197632** | **5.0** | **1573875** | **1842902** | **0.8** |
| **Median** | **253529** | **5.0** | **1971643** | **2188760** | **4.8** |
| **Max** | **507039** | **5.0** | **2513158** | **2523836** | **8.9** |
| **CV%** | **45.9** | **0.0** | **19.4** | **12.8** | **77.2** |
| **Geometric Mean** | **283118** | **5.0** | **1979695** | **2172404** | **3.4** |

| Period 2 (Treatment = rhGAA + 50 mg AT2220) | | | | | | |
|---|---|---|---|---|---|---|
| **SubjID** | **$C_{max}$ (ng/mL)** | **$T_{max}$ (hr)** | **$AUC_{0-T}$ (ng*hr/mL)** | **$AUC_{0-\infty}$ (ng*hr/mL)** | **$F_{rel}$** | **T½ (hr)** |
| 2031-0101 | 259022 | 6.0 | 2440258 | 2712963 | 1.5 | 6.8 |
| 2031-0102 | 281085 | 5.0 | 3037230 | 3344934 | 1.5 | 6.3 |
| 2031-0103 | 334448 | 6.0 | 2400468 | 2903123 | 1.4 | 4 |
| 6003-0101 | 645181 | 4.0 | 3437632 | 3618220 | 1.4 | 2 |
| **N** | **4** | **4** | **4** | **4** | **4** | **4** |
| **Mean** | **379934** | **5.3** | **2828897** | **3144810** | **1.4** | **4.8** |
| **SD** | **179644** | **1.0** | **499517** | **411935** | | **2.2** |
| **Min** | **259022** | **4.0** | **2400468** | **2712963** | | **2.0** |
| **Median** | **307767** | **5.5** | **2738744** | **3124029** | | **5.2** |
| **Max** | **645181** | **6.0** | **3437632** | **3618220** | | **6.8** |
| **CV%** | **47.3** | **18.2** | **17.7** | **13.1** | | **46.4** |
| **Geometric Mean** | **354035** | **5.2** | **2796515** | **3124625** | | **4.3** |

Results: Cohort 2

Plasma rhGAA activity increases with co-administration of 1-deoxynojirimycin hydrochloride and acid α-glucosidase relative to acid α-glucosidase alone

For co-administration of acid α-glucosidase with 100mg 1-deoxynojirimycin hydrochloride (Period 2) relative to ERT alone (Period 1), a 1.7-fold mean increase in plasma rhGAA activity AUC (Area Under the Curve) was observed, as shown in Table 10A. Table 10A also shows the fold change in rhGAA activity between Periods 1 and 2 in Day 3 and Day 7 muscle biopsies of Cohort 2 subjects. Table 10B shows the plasma PK summary for 1-deoxynojirimycin hydrochloride from Period 2 of Cohorts 1 and 2. Table 10C shows the total rhGAA protein concentrations from Cohorts 1 and 2 as measured by Western blot.

Table 10A. Mean Plasma rhGAA Activity PK Summary for Cohorts 1 (4 subjects) and Cohort 2 (6 subjects), and Mean Muscle Biopsy rhGAA Activity for Cohort 2 (6 subjects):

| Period/ Treatment | Cohort | $C_{max}$[a] (nmol/hr/mL) | $T_{max}$[b] (hr) | $AUC_{0-T}$[a] (hr*nmol/hr/mL) | $AUC_{0-\infty}$[a] (hr*nmol/hr/mL) | AUC Ratio[a] | t½[c] (hr) |
|---|---|---|---|---|---|---|---|
| rhGAA Alone (N=4) | 1 | 16441 (31.6) | 5.0 (4 - 5) | 105834 (30.7) | 107707 (31.1) | - | 3.9 (0.6) |
| rhGAA + 50 mg AT2220 (N=4) | 1 | 19922 (24.4) | 5.0 (4 - 6) | 152207 (21.3) | 157162 (22.4) | 1.5 (13.2) | 4.2 (0.8) |
| rhGAA Alone (N=6) | 2 | 22785 (18.0) | 4.0 (3 - 5) | 142415 (30.4) | 144953 (31.5) | - | 3.8 (0.6) |
| rhGAA + 100 mg AT2220 (N=6) | 2 | 28607 (13.9) | 4.0 (3 - 6) | 229434 (25.3) | 241203 (27.5) | 1.7 (10.1) | 4.9 (0.8) |

[a]Geometric mean (CV%)
[b]Median (range)
[c]Arithmetic mean (SD)

| Treatment | Biopsy Day | rhGAA Activity (pmol/mg/hr) | Fold Change |
|---|---|---|---|
| rhGAA Alone | - | 1538.8 | |
| rhGAA + 100 mg AT2220 | 3 | 2846.1 | 1.3 |
| rhGAA + 100 mg AT2220 | 7 | 1115.0 | 1.2 |
| Follow-up for Baseline | 28 | 634.5 | |

Table 10B. Mean AT2220 PK Summary for Cohorts 1 and 2

| AT2220 PK Summary Table | | | | | | | |
|---|---|---|---|---|---|---|---|
| AT2220 Dose | $C_{max}$[a] (ng/mL) | $T_{max}$[b] (hr) | $AUC_{0-T}$[a] (ng·hr/mL) | $AUC_{0-\infty}$[a] (ng·hr/mL) | $AUC_{0-\infty}$ Ratio | t½[c] (hr) |
| 50 mg (N=4) | 1035 (25.0) | 2.0(1-3) | 6972 (28.1) | 7008(28.2) | - | 3.1(0.2) |
| 100 mg (N=6) | 1863 (13.1) | 3.0(1-4) | 13003 (18.9) | 13137 (19.2) | 1.9 | 3.4(0.3) |

[a]Geometric mean (CV%)
[b]Median (range)
[c]Arithmetic mean (SD)

Table 10C. Cohorts 1 and 2 Plasma rhGAA Protein PK Summary

| Cohorts 1 and 2 Plasma rhGAA Protein PK Summary | | | | | | | |
|---|---|---|---|---|---|---|---|
| **Period/ Treatment** | **Cohort** | **$C_{max}$[a] (nmol/hr/mL)** | **$T_{max}$[b] (hr)** | **$AUC_{0-T}$[a] (hr*nmol/hr/mL)** | **$AUC_{0-T}$[a] Ratio** | **$AUC_{0-\infty}$[a] (hr*nmol/hr/mL)** | **T½[c] (hr)** |
| rhGAA Alone (N=4) | 1 | 283118 (45.9) | 5.0 (5-5) | 1979695 (19.4) | - | 2172404 (12.8) | 4.8 (3.7) |
| rhGAA+50 mgAT2220 (N=4) | 1 | 354035 (47.3) | 5.5 (4-6) | 2796515 (17.7) | 1.4 (9.2) | 3124625 (13.1) | 4.8 (2.2) |
| rhGAA Alone (N=6) | 2 | 399623 (25.8) | 4.0 (4-5) | 2315249 (44.3) | - | 2862876 (86.8) | 3.9 (4.5) |
| rhGAA + 100 mg AT2220 (N=6) | 2 | 430738 (30.5) | 4.0 (4-7) | 3841773 (44.5) | 1.7(18.5) | 5123518 (42.3) | 6.1 (3.2) |
| [a]Geometric mean (CV%)<br>[b]Median (range)<br>[c]Arithmetic mean (SD) | | | | | | | |

For co-administration with 100mg 1-deoxynojirimycin hydrochloride (Period 2) relative to ERT alone (Period 1), individual subject increases in plasma rhGAA activity AUC were 1.5-, 1.5-, 1.6-, 1.7-, 1.8- and 1.9-fold, as shown in Tables 11-16. Tables 11-16 also show the fold change in rhGAA activity between Periods 1 and 2 in Day 3 or Day 7 muscle biopsies of Cohort 2 subjects.

Table 11. rhGAA Activity in Plasma and Muscle for Subject 2028-0201 (Patient F) (Cohort 2)

| **Treatment [total dose (mg), duration of infusion (hr:min), infusion rate (mL/hr)]** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **t½ (hr)** | **$F_{rel}$** |
|---|---|---|---|---|---|---|
| 18.2 mg/kg GAA alone (1850, 4:00, 175) | 20541 | 4.0 | 125868 | 127210 | 3.5 | - |
| 18.2 mg/kg GAA + 100 mg AT2220 (1850, 3:57, 175) | 28053 | 4.0 | 212384 | 222194 | 4.8 | 1.75 |

Body weight: 101.5 kg

| **Treatment** | **Biopsy Day** | **rhGAA Activity (pmol/mg/hr)** | **Fold Change** | **Baseline-subtracted Ratio** | **% Change from Baseline** |
|---|---|---|---|---|---|
| rhGAA Alone | 7 | 756.4 | | | 13.6 |
| rhGAA + 100 mg AT2220 | 7 | 794.1 | 1.0 | 1.4 | 19.2 |
| Follow-up for Baseline | 28 | 666.0 | | | |

Table 12. rhGAA Activity in Plasma and Muscle for Subject 2029-0202 (Patient G) (Cohort 2)

| Treatment [total dose (mg), duration of infusion (hr:min), infusion rate (mL/hr)] | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | t½ (hr) | $F_{rel}$ |
|---|---|---|---|---|---|---|
| 31.3 mg/kg GAA alone (2200, 5:12, 115) | 29167 | 5.0 | 231174 | 240183 | 4.6 | - |
| 31.3 mg/kg GAA + 100 mg AT2220 (2200, 5:24, 115) | 34882 | 6.0 | 321065 | 349917 | 5.9 | 1.46 |

Body weight: 70.4 kg

| Treatment | Biopsy Day | rhGAA Activity (pmol/mg/hr) | Fold Change | Baseline-subtracted Ratio | % Change from Baseline |
|---|---|---|---|---|---|
| rhGAA Alone | 7 | 1019.9 | | | 178 |
| rhGAA + 100 mg AT2220 | 7 | 1608.9 | 1.6 | 1.9 | 339 |
| Follow-up for Baseline | 28 | 366.8 | | | |

Table 13. rhGAA Activity in Plasma and Muscle for Subject 6003-0202 (Patient I) (Cohort 2)

| **Treatment [total dose (mg), duration of infusion (hr:min), infusion rate (mL/hr)]** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **t½ (hr)** | **$F_{rel}$** |
|---|---|---|---|---|---|---|
| 20.0 mg/kg GAA alone (2100, 3:55, 175) | 20799 | 4.0 | 117942 | 119400 | 3.6 | - |
| 20.0 mg/kg GAA + 100 mg AT2220 (2100, 3:58, 175) | 29775 | 4.0 | 185508 | 190894 | 4.2 | 1.60 |

Body weight: 105.1 kg

| **Treatment** | **Biopsy Day** | **rhGAA Activity (pmol/mg/hr)** | **Fold Change** | **Baseline-subtracted Ratio** | **% Change from Baseline** |
|---|---|---|---|---|---|
| rhGAA Alone | 7 | 844.7 | | | - |
| rhGAA + 100 mg AT2220 | 7 | 942.0 | 1.1 | - | - |
| Follow-up for Baseline | 28 | N/A | | | |

Table 14. rhGAA Activity in Plasma and Muscle for Subject 2029-0203 (Patient H) (Cohort 2)

| Treatment [total dose (mg), duration of infusion (hr:min), infusion rate (mL/hr)] | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | t½ (hr) | $F_{rel}$ |
|---|---|---|---|---|---|---|
| 20.6 mg/kg GAA alone (1850, 3:42, 175) | 17907 | 4.0 | 107204 | 110173 | 4.6 | - |
| 20.6 mg/kg GAA + 100 mg AT2220 (1850, 3:44, 175) | 23243 | 4.0 | 161694 | 166686 | 4.4 | 1.51 |

Body weight: 89.7 kg

| Treatment | Biopsy Day | rhGAA Activity (pmol/mg/hr) | Fold Change | Baseline-subtracted Ratio | % Change from Baseline |
|---|---|---|---|---|---|
| rhGAA Alone | 3 | 1347.7 | | | 54.8 |
| rhGAA + 100 mg AT2220 | 3 | 1103.2 | 0.8 | 0.5 | 26.7 |
| Follow-up for Baseline | 28 | 870.7 | | | |

Table 15. rhGAA Activity in Plasma and Muscle for Subject 6003-0203 (Patient J) (Cohort 2)

| **Treatment [total dose (mg), duration of infusion (hr:min), infusion rate (mL/hr)]** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **t½ (hr)** | **$F_{rel}$** |
|---|---|---|---|---|---|---|
| 19.9 mg/kg GAA alone (1450, 3:25, 175) | 23779 | 3.0 | 153974 | 155601 | 3.4 | - |
| 19.9 mg/kg GAA + 100 mg AT2220 (1450, 3:25, 175) | 26219 | 3.0 | 269271 | 292137 | 5.8 | 1.88 |

Body weight: 73.0 kg

| **Treatment** | **Biopsy Day** | **rhGAA Activity (pmol/mg/hr)** | **Fold Change** | **Baseline-subtracted Ratio** | **% Change from Baseline** |
|---|---|---|---|---|---|
| rhGAA Alone | 3 | 3908.7 | | | - |
| rhGAA + 100 mg AT2220 | 3 | 5296.6 | 1.4 | - | - |
| Follow-up for Baseline | 28 | N/A | | | |

Table 16. rhGAA Activity in Plasma and Muscle for Subject 2001-0201 (Patient E) (Cohort 2)

| Treatment [total dose (mg), duration of infusion (hr:min), infusion rate (mL/hr)] | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | t½ (hr) | $F_{rel}$ |
|---|---|---|---|---|---|---|
| GAA alone | 26371 | 4.0 | 147283 | 148324 | 3.13 | - |
| GAA + 100 mg AT2220 | 30869 | 4.0 | 264841 | 272353 | 4.07 | 1.84 |

Body weight: 106.9 kg

| Treatment | Biopsy Day | rhGAA Activity (pmol/mg/hr) | Fold Change | Baseline-subtracted Ratio | % Change from Baseline |
|---|---|---|---|---|---|
| rhGAA Alone | 3 | 1355.4 | | | - |
| rhGAA + 100 mg AT2220 | 3 | 2138.5 | 1.6 | - | - |
| Follow-up for Baseline | 28 | N/A | | | |

Table 17 below shows the cumulative AUCs for rhGAA activity in plasma of cohort 2.

Table 17.

| Time (hr) | 2028-0201 | | 2029-0202 | | 6003-0202 | | 2029-0203 | | 6003-0203 | | 2001-0201 | |
|---|---|---|---|---|---|---|---|---|---|---|---|---|
| | Period 1 | Period 2 | Period 1 | Period 2 | Period 1 | Period 2 | Period 1 | Period 2 | Period 1 | Period 2 | Period 1 | Period 2 |
| 0 | 0 | 0 | 0 | 0 | 0 | 0 | 0 | 0 | 0 | 0 | 0 | 0 |
| 1 | 1472 | 1769 | 1443 | 1610 | 1034 | 1068 | 1220 | 1337 | 1671 | 1659 | 1386 | 1454 |
| 2 | 7965 | 8315 | 7828 | 9621 | 7705 | 7937 | 7214 | 7450 | 10499 | 10615 | 7949 | 8983 |
| 3 | 22987 | 22573 | 22121 | 26716 | 22685 | 23428 | 20011 | 20154 | 29546 | 31020 | 22185 | 26097 |
| 4 | 43259 | 46080 | 43930 | 50207 | 42426 | 48006 | 36988 | 39704 | 52442 | 56689 | 44429 | 52571 |
| 5 | 62103 | 72142 | 70971 | 78427 | 59273 | 72180 | 52355 | 60508 | 72064 | 79937 | 67125 | 80667 |
| 6 | 77708 | 92769 | 98881 | 111294 | 71064 | 90597 | 63550 | 77746 | 88561 | 101224 | 83577 | 104121 |
| 7 | 89164 | 109551 | 122363 | 141376 | 80584 | 107070 | 72339 | 92388 | 102076 | 122069 | 96025 | 124866 |
| 8 | 96948 | 124505 | 140329 | 164267 | 87328 | 120678 | 78879 | 103874 | 112225 | 141195 | 105784 | 143990 |
| 9 | 102833 | 137830 | 155433 | 184205 | 92657 | 132007 | 83646 | 113265 | 120241 | 157686 | 112846 | 160285 |
| 10 | 107669 | 150103 | 168382 | 203675 | 97345 | 141786 | 87638 | 121326 | 126846 | 173053 | 118271 | 174339 |
| 12 | 114263 | 168912 | 187295 | 235455 | 104183 | 155687 | 93505 | 133617 | 136582 | 199017 | 126701 | 198053 |
| 24 | 125868 | 212384 | 231174 | 321065 | 117942 | 185508 | 107204 | 161694 | 153974 | 269271 | 147283 | 264841 |

Table 18A shows rhGAA activity from muscle biopsies taken on Days 3 or 7 of Periods 1 and 2, plus an optional biopsy at follow up for cohort 2 subject.

Table 18A. rhGAA Activity in Muscle (Cohort 2)

| SubjID/Period | Biopsy Day | % Change from Baseline | Baseline-subtracted Ratio | Change from rhGAA Alone |
|---|---|---|---|---|
| 6003-0203* | 3 | - | - | **1.4** |
| 2001-0201* | 3 | - | - | **1.6** |
| 2029-0202/P1 | 7 | 178.1 | | **-** |
| 2029-0202/P2 | 7 | 338.6 | 1.9 | **1.6** |
| 2029-0203/P1 | 3 | 54.8 | | **-** |
| 2029-0203/P2 | 3 | 26.7 | 0.5 | **0.8** |
| 6003-0202* | 7 | - | - | **1.1** |
| 2028-0201/P1 | 7 | 13.6 | | **-** |
| 2028-0201/P2 | 7 | 19.2 | 1.4 | **1.0** |
| * Baseline not available | | | | |

Table 18B shows individual muscle 1-DNJ-HCL (AT2220) concentrations taken on Days 3 or 7 of Cohorts 1 and 2.

Table 18B. Individual Muscle 1-DNJ-HCL (AT2220) Concentrations

| AT2220-010 Muscle AT2220 Cone for Cohort 2 | | | | |
|---|---|---|---|---|
| **SubjID** | **Dose (mg)** | **Period** | **Study Day** | **Muscle AT2220 Conc (ng/g)[a]** |
| Patient A | 50 mg | Period 2 | Day 7 | 11.8 |
| Patient B | 50 mg | Period 2 | Day 7 | 10.0 |
| Patient C | 50 mg | Period 2 | Day 7 | 13.1 |
| Patient D | 50 mg | Period 2 | Day 7 | BLQ |
| Patient E | 100 mg | Period 2 | Day 3 | 17.8 |
| Patient F | 100 mg | Period 2 | Day 7 | B LQ[b] |
| Patient F | 0 mg | Follow up | Baseline | BLQ |
| Patient G | 100 mg | Period 2 | Day 7 | BLQ |
| Patient G | 0 mg | Follow up | Baseline | BLQ |
| Patient H | 100 mg | Period 2 | Day 3 | BLQ[b] |
| Patient H | 0 mg | Follow up | Baseline | BLQ |
| Patient I | 100 mg | Period 2 | Day 7 | BLQ[b] |
| Patient J | 100 mg | Period 2 | Day 3 | BLQ[c] |
| [a]LLOQ = 8.0 ng/g<br>[b]Due to small muscle volume, sample was diluted 2-fold, therefore LLOQ = 16.0 ng/g<br>[c]Due to small muscle volume, sample was diluted 3-fold, therefore LLOQ = 32.0 ng/g | | | | |

Summary of Results

Single doses of 50 mg and 100 mg 1-DNJ-HCl (AT2220) have been found safe and well-tolerated in patients with Pompe Disease. Only mild, transient adverse events (AE's) have been reported, none of which were related to AT2220 (representative adverse events are described below). One serious AE was citalopram-induced QTc prolongation. The QTc prolongation attenuated following citalopram dose reduction. Generally, urine hex 4 levels either did not change from baseline, or did not show any consistent trend following a single dose of AT2220 (Fig. 7). Additionally, CPK levels did not appreciably change from baseline in Cohorts 1 and 2 (Fig. 8).

Plasma rhGAA activity AUC increased for all patients for both co-administered doses relative to alglucosidase alfa alone. Increases in AUC were primarily driven by prolonged plasma half-life due to increases in rhGAA activity at

post-Tmax time points (Table 10A, Fig. 2A and Fig. 4). The increases in plasma rhGAA activity AUC suggest an increase in stabilized rhGAA uptake for tissue distribution.

Muscle biopsies were taken on Day 7 for all four Cohort 1 patients, and on Day 3 or Day 7 for each of 3 of the 6 Cohort 2 patients. Three patients from Cohort 2 had an optional Day 30 muscle biopsy, that was used as a baseline for those patients. Of the Cohort 1 patients, following co-administration of 50 mg AT2220, one had a 40% increase in muscle rhGAA activity, two showed no change, and one had a 30% decrease in rhGAA activity relative to rhGAA alone. Of the Cohort 2 patients, following co-administration of 100 mg AT2220, two patients had 60% and 40% increases in rhGAA activity relative to rhGAA alone, but one was decreased by 20% from biopsies taken on Day 3. From biopsies taken on Day 7, two were increased by 60% and 10%, and one showed no change in rhGAA activity.

The pharmacokinetics of plasma AT2220 are approximately linear for the 50 mg and 100 mg doses evaluated at this point in the study. An approximate 2-fold increase in Cmax and AUC is observed with dose (Table 10B, Fig. 5). The rate of absorption (Tmax) is 2 to 3 hours indicating all bioavailable drug has been absorbed early on during the infusion of rhGAA. Muscle AT2220 concentrations from Day 3 or Day 7 biopsies were either below or near the lower limit of quantitation of 8 ng/g (Table 18B).

Total plasma rhGAA protein by Western blot followed a similar trend as plasma rhGAA activity in terms of AT2220 dose-related increases (Fig. 6, Table 10C).

Conclusions

1-DNJ-HCl was safe and well-tolerated at both 50 mg and 100 mg dose levels evaluated to date.

Plasma rhGAA activity increased 20% to 40% and 50% to 90% following single dose of 50 mg and 100 mg 1-DNJ-HCl, respectively.

At the 50 mg dose level, 1 of 4 patients had increased rhGAA activity in muscle; however, at 100 mg 1-DNJ-HCl, 4 of 6 patients had up to 60% increases in rhGAA activity in muscle.

Plasma 1-DNJ-HCl demonstrated approximately linear PK for the 2 doses evaluated to date.

1-DNJ-HCl concentrations in muscle were either below or just above the lower limit of quantification from Day 3 or 7 biopsies suggesting 1-DNJ-HCl may not accumulate following multiple dosing every 14 days.

Plasma total rhGAA protein PK followed a similar trend to rhGAA activity PK.

Adverse Events

20 adverse events (AEs) have been reported, one of which was serious. Representative adverse events are shown in Tables 19 and 20. The serious AE, prolonged QTc interval from 473 to 493 msec, which occurred after the screening visit, but prior to dosing, was moderate in severity, and was considered unrelated to study drug by the investigator. All other AEs were mild in severity, all considered unrelated to study drug, and resolved without treatment. Urine hexose tetrasaccharide A (urine Hex 4) and serum CPK levels are presented for each patient in Figures 7 and 8.

Table 19. Cohort 1: Adverse Events

| Investigator No./Patient No. | Adverse Event | Start Date | Stop Date | Outcome | Any Treatment Required? | Relation to Study Drug? | Was Event Serious? | Intensity |
|---|---|---|---|---|---|---|---|---|
| **20310101** | SINUS BLOCKED WITH RUNNY NOSE | 31JAN2012 | 04FEB2012 | Recovered/ Resolved | None | Unrelated | No | Mild |
| | TIRED, MORE THAN USUAL | 31JAN2012 | 04FEB2012 | Recovered/ Resolved | None | Unrelated | No | Mild |
| **20310102** | BILATERAL THIGH PAIN | 06JAN2012 | 31JAN2012 | Recovered/ Resolved | None | Unrelated | No | Mild |
| | FREQUENCY OF URINATION INCREASED TO APPROX HOURLY. | 06JAN2012 | 09JAN2012 | Recovered/ Resolved | None | Unrelated | No | Mild |
| | LONGER SLEEP INCREASED NAP FREQUENCY | 06JAN2012 | 09JAN2012 | Recovered/ Resolved | None | Unrelated | No | Mild |
| | RLEG SORE "SHOOTING PAIN" WHEN PRESSURE APPLIED TO SITE | 10JAN2012 | | Recovering/ Resolving | Concomitant Medications | Unrelated | No | Mild |
| | RIGHT INTERIOR SIDE OF MOUTH FELT LIKE TISSUE WAS CHEWED ON | 05JAN2012 | 09JAN2012 | Recovered/ Resolved | None | Unrelated | No | Mild |
| | UPPER RESPIRATO RY/FLU LIKE SYMPTOMS | 06JAN2012 | 09JAN2012 | Recovered/ Resolved | None | Unrelated | No | Mild |
| **20310103** | PAIN IN RIGHT THIGH | 05JAN2012 | 20JAN2012 | Recovered/ Resolved | Concomitant Medications | Unrelated | No | Mild |
| **60030101** | ACHING FEET | 25NOV2011 | 25NOV2011 | Recovered/ Resolved | Concomitant Medications | Unrelated | No | Mild |

- Safety data reviewed for 4 subjects
- No SAE or drug-related AEs
- Urine Hex4: no drug related elevations
- ALT/AST: No drug related elevations
- CPK: no drug related elevations beyond subjects' baseline values range - no clinically significant changes
- Muscle strength: No change between Periods 1 and 2
- Follow-up occurs 24 - 30 days after Period 2, Visit 4

    - 3 subjects showed elevation in Hex4 and CPK levels
    - Additional follow-up showed all 3 subjects had Hex4 and CPK levels within baseline range
    - changes were consistent with background noise

Table 20. Cohort 2: Adverse Events

| Investigator No./Patient No. | Adverse Event | Start Date | Stop Date | Outcome | Any Treatment Required? | Relation to Study Drug? | Was Event Serious? | Intensity |
|---|---|---|---|---|---|---|---|---|
| **60030202** | None | N/A | N/A | N/A | N/A | N/A | N/A | N/A |
| **20280201** | RIGHT LEG PAIN POST MUSCLE BIOPSY | 21MAR2012 | 22MAR20 12 | Recovered/ Resolved | None | Unrelated | No | Mild |
| **20290202** | None | N/A | N/A | N/A | N/A | N/A | N/A | N/A |
| **20290203** | ERYTHEMA AT PORT SITE SPREADING TO HALFWAY UP NECK AND MID-CHEST DUE TO ALLERGIC REACTION TO TAPE | 05APR2012 | 07APR2012 | Recovered/ Resolved | None | Unrelated | No | Mild |
| | TENDERNESS AT MUSCLE BIOPSY SITE RIGHT LEG | 06APR2012 | 19APR2012 | Recovered/ Resolved | None | Unrelated | No | Mild |
| | BRUISING AT MUSCLE BIOPSY SITE RIGHT LEG | 06APR2012 | 17APR2012 | Recovered/ Resolved | None | Unrelated | No | Mild |
| | ERYTHEMA AT PORT SITE DUE TO ALLERGIC REACTION TO TAPE | 19APR2012 | 21 APR2012 | Recovered/ Resolved | None | Unrelated | No | Mild |
| **60030203** | None | N/A | N/A | N/A | N/A | N/A | N/A | N/A |

- Safety data reviewed for 5 subjects
- No SAE or drug-related AEs
- Hex4: no drug related elevations
- ALT/AST: No drug related elevations
- CPK: no drug related elevations beyond subjects' baseline range
- Muscle strength: No change between Periods 1 and 2

**EXAMPLE 3: Dosing Regimen for the Treatment of Pompe Disease using 1-Deoxynojirimycin Hydrochloride and Alglucosidase Alfa -- Cohorts 3 and 4**

One objective of the study was to evaluate the safety, effectiveness, and pharmacodynamics of dose regimens comprising co-administering 1-deoxynojirimycin hydrochloride and alglucosidase alfa in patients with Pompe disease.

Another objective of the study was to assess the effects of various doses of 1-deoxynojirimycin hydrochloride on the GAA activity. This was evaluated by measuring the GAA enzyme activity and protein levels in skeletal muscle at Day 3 and/or Day 7 following a single intravenous infusion with alglucosidase alfa alone and after pre-administration of single ascending oral doses of 1-deoxynojirimycin hydrochloride.

***Methods.*** The study was conducted essentially according to the methods described in Example 1. Cohort 3 comprised 6 subjects. Cohort 4 comprised 7 subjects. Each subject received alglucosidase alfa alone as an intravenous infusion in Period 1 and a single 250 mg dose (Cohort 3) or 600 mg (Cohort 4) of 1-deoxynojirimycin hydrochloride one hour before initiation of an intravenous infusion of alglucosidase alfa in Period 2.

***Results: Cohort 3***

Plasma and muscle rhGAA activity increases with co-administration of 1-deoxynojirimycin hydrochloride and acid α-glucosidase relative to acid α-glucosidase alone

For co-administration of acid α-glucosidase with 250mg 1-deoxynojirimycin hydrochloride (Period 2) relative to ERT alone (Period 1), a 2.0-fold mean increase in plasma rhGAA activity was observed, as shown in Table 21.

Table 21. Plasma rhGAA Activity PK Summary for Cohorts 1, 2, and 3.

| Period/Treatment | Cohort | Period | $C_{max}$[a] (nmol/hr/mL) | $T_{max}$[b] (hr) | $AUC_{0-T}$[a] (hr*nmol/hr/ml) | $AUC_{0-\infty}$[a] (hr*nmol/hr/mL) | AUC Ratio[a] | t½[c](hr) |
|---|---|---|---|---|---|---|---|---|
| rhGAA Alone (N=4) | 1 | 1 | 16441 (31.6) | 5.0 (4-5) | 105834 (30.7) | 107707 (31.1) | | 3.9 (0.6) |
| rhGAA+50 mg AT2220 (N=4) | 1 | 2 | 19922 (24.4) | 5.0 (4-6) | 152207 (21.3) | 157162 (22.4) | 1.5 (13.2) | 4.2 (0.8) |
| rhGAA Alone (N=6) | 2 | 1 | 22785 (18,0) | 4.0 (3-5) | 142415 (30.4) | 144953 (31.5) | | 3.8 (0,6) |
| rhGAA + 100 mg AT2220 (N=6) | 2 | 2 | 28607 (13.9) | 4.0 (3-6) | 229434 (25.3) | 241203 (27.5) | 1.7 (10.1) | 4.9 (0.8) |
| rhGAA Alone (N=6) | 3 | 1 | 18986 (18.7) | 4.0 (4-4) | 107774 (19.7) | 109165 (20.0) | | 3.6 (0.5) |
| rhGAA + 250 mg AT2220 (N=6) | 3 | 2 | 22651 (9.0) | 4.0 (4-4) | 201224 (8,8) | 214751 (9.5) | 2.0 (19.7) | 5.3 (1,1) |

Geometric mean (CV%)
[b]Median (range)
[c]Arithmetic mean (SD)

For co-administration of acid α-glucosidase with 250mg 1-deoxynojirimycin hydrochloride (Period 2) relative to ERT alone (Period 1), increases in plasma rhGAA activity of cohort 3 subjects are shown in Tables 22-35. These tables also show the increase in rhGAA in muscle tissue following co-administration of acid α-glucosidase with 250mg 1-deoxynojirimycin hydrochloride (Period 2) relative to ERT alone (Period 1).

Table 22. rhGAA Activity in Plasma for Subject 2027-0301.

| **Treatment (Total Dose [mg], Duration of Infusion [hr: min])** | **SubjID** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **P2/P1 $AUC_{0-\infty}$ Ratio** | **t½ (hr)** |
|---|---|---|---|---|---|---|---|
| 20.0 mg/kg rhGAA Alone (1540, 3:41) | 2027-0301 | 22352 | 4.0 | 112800 | 113502 | - | 3.2 |
| 20.0 mg/kg rhGAA + 250 mg AT2220 (1540, 3:55) | 2027-0301 | 23309 | 4.0 | 187370 | 192337 | 1.7 | 4.0 |
| Body Weight = 77.0 kg | | | | | | | |

Table 23. rhGAA Activity in Plasma for Subject 2029-0304.

| **Treatment (Total Dose [mg], Duration of Infusion [hrmin])** | **SubjID** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **P2/P1 $AUC_{0-\infty}$ Ratio** | **t½ (hr)** |
|---|---|---|---|---|---|---|---|
| 19.9 mg/kg rhGAA Alone (1900, 3:44) | 2029-0304 | 23813 | 4.0 | 134941 | 136708 | - | 3.7 |
| 19.9 mg/kg rhGAA + 250 mg AT2220 (1900,4:02) | 2029-0304 | 23628 | 4.0 | 209939 | 225285 | 1.6 | 5.7 |
| Body Weight = 95.6 kg | | | | | | | |

Table 24. rhGAA Activity in Plasma for Subject 2029-0305.

| **Treatment (Total Dose [mg], Duration of infusion [hr: min])** | **SubjID** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **P2/P1 $AUC_{0-\infty}$ Ratio** | **t½ (hr)** |
|---|---|---|---|---|---|---|---|
| 20.5 mg/kg rhGAA Alone (1400, 3:44) | 2029-0305 | 19996 | 4.0 | 115349 | 117667 | - | 4.3 |

(continued)

| Treatment (Total Dose [mg], Duration of infusion [hr: min]) | SubjID | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | P2/P1 $AUC_{0-\infty}$ Ratio | t½ (hr) |
|---|---|---|---|---|---|---|---|
| 20.5 mg/kg rhGAA + 250 mg AT2220 (1400, 3:49) | 2029-0305 | 25898 | 4.0 | 233726 | 248494 | 2.1 | 5.3 |
| Body Weight = 68.3 kg | | | | | | | |

Table 25. rhGAA Activity in Plasma for Subject 2029-0306.

| Treatment (Total Dose [mg], Duration of Infusion [hr:min]) | SubjID | $C_{max}$ (nmol/hr/mL) | $T_{max}$ (hr) | $AUC_{0-T}$ (hr*nmol/hr/ml) | $AUC_{0-\infty}$ (hr*nmol/hr/mL) | P2/P1 $AUC_{0-\infty}$ Ratio | t½ (hr) |
|---|---|---|---|---|---|---|---|
| 20.4 mg/kg rhGAA Alone (1300,3:43) | 2029-0306 | 14548 | 4.0 | 97198 | 98713 | - | 3.7 |
| 20.4 mg/kg rhGAA + 250 mg AT2220 (1300, 3:59) | 2029-0306 | 21631 | 4.0 | 189711 | 215411 | 2.2 | 7.2 |
| Body Weight = 63.8 kg | | | | | | | |

Table 26. rhGAA Activity in Plasma for Subject 2029-0307.

| Treatment (Total Dose [mg], Duration of Infusion [hr:min]) | SubjID | $C_{max}$ [nmol/hr/mL) | $T_{max}$ (hr) | $AUC_{0-T}$ (hr*nmol/hr/mL) | $AUC_{0-\infty}$ (hr*nmo [/hr/mL) | P2/P1 $AUC_{0-\infty}$ Ratio | t½ (hr) |
|---|---|---|---|---|---|---|---|
| 19.9 mg/kg rhGAA Alone (1200, 3:43) | 2029-0307 | 15758 | 4.0 | 73754 | 74024 | - | 2.9 |
| 19.9 mg/kg rhGAA + 250 mg AT2220 (1200, 3:43) | 2029-0307 | 21983 | 4.0 | 189648 | 196051 | 2.6 | 4.3 |
| Body Weight = 60.4 kg | | | | | | | |

Table 27. rhGAA Activity in Plasma for Subject 6003-0304.

| Treatment (Total Dose [mg], Duration of Infusion [hr:min]) | SubjID | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | P2/P1 $AUC_{0-\infty}$ Ratio | t½ (hr) |
|---|---|---|---|---|---|---|---|
| 20.2 mg/kg rhGAA Alone (1200,4:00) | 6003-0304 | 19195 | 4.0 | 124504 | 126855 | - | 3.8 |
| 20.2 mg/kg rhGAA + 250 mg AT2220 (1200, 3:50) | 6003-0304 | 19912 | 4.0 | 200698 | 215705 | 1.7 | 5.5 |
| Body Weight = 59.5 kg | | | | | | | |

Table 28. PK Table: individual subjects and statistical summary for cohort 3.

| Cohort | Period | Treatment | SubjID | $C_{max}$ (nmol/hr/m L) | $T_{max}$ (hr) | $AUC_{0-T}$ (hr*nmol/hr/mL) | $AUC_{0-\infty}$ (hr*nmol/hr/mL) | P2/P1 $AUC_{0-\infty}$ Ratio | t½ (hr) |
|---|---|---|---|---|---|---|---|---|---|
| Cohort 3 | Period 1 | rhGAA Alone | 2027-0301 | 22352 | 4.0 | 112800 | 113502 | - | 3.2 |
| | | | 2029-0304 | 23813 | 4.0 | 134941 | 136708 | - | 3.7 |
| | | | 2029-0305 | 19996 | 4.0 | 115349 | 117667 | - | 4.3 |
| | | | 2029-0306 | 14548 | 4.0 | 97198 | 98713 | - | 3.7 |
| | | | 2029-0307 | 15758 | 4.0 | 73754 | 74024 | - | 2.9 |
| | | | 6003-0304 | 19195 | 4.0 | 124504 | 126855 | - | 3.8 |
| | | | **N** | **6** | **6** | **6** | **6** | **-** | **6** |
| | | | **Mean** | **19277** | **4.0** | **109758** | **111245** | **-** | **3.6** |
| | | | **SD** | **3614** | **0.0** | **21662** | **22275** | **-** | **0.5** |
| | | | **Min** | **14548** | **4.0** | **73754** | **74024** | **-** | **2.9** |
| | | | **Median** | **19596** | **4.0** | **114075** | **115585** | **-** | **3.7** |
| | | | **Max** | **23813** | **4.0** | **134941** | **136708** | **-** | **4.3** |
| | | | **CV%** | **18.7** | **0.0** | **19.7** | **20.0** | **-** | **13.6** |
| | | | **Geometric Mean** | **18986** | **4.0** | **107774** | **109165** | | **3.6** |

| Cohort | Period | Treatment | SubjID | $C_{max}$ (nmol/hr/mL) | $T_{max}$ (hr) | $AUC_{0-T}$ (hr*nmol/hr/mL) | $AUC_{0-\infty}$ (Hr*nmol/hr/mL) | P2/P1 $AUC_{0-\infty}$ Ratio | t½ (hr) |
|---|---|---|---|---|---|---|---|---|---|
| Cohort 3 | Period 2 | rhGAA + 250 mg AT2220 | 2027-0301 | 23309 | 4.0 | 187370 | 192337 | 1.7 | 4.0 |
| | | | 2029-0304 | 23628 | 4.0 | 209939 | 225285 | 1.6 | 5.7 |
| | | | 2029-0305 | 25898 | 4.0 | 233726 | 248494 | 2.1 | 5.3 |
| | | | 2029-0306 | 21631 | 4.0 | 189711 | 215411 | 2.2 | 7.2 |
| | | | 2029-0307 | 21983 | 4.0 | 189648 | 196051 | 2.6 | 4.3 |
| | | | 6003-0304 | 19912 | 4.0 | 200698 | 215705 | 1.7 | 5.5 |
| | | | **N** | **6** | **6** | **6** | **6** | **6** | **6** |
| | | | **Mean** | **22727** | **4.0** | **201849** | **215547** | **2.0** | **5.3** |
| | | | **SD** | **2044** | **0.0** | **17826** | **20490** | **0.39** | **1.1** |
| | | | **Min** | **19912** | **4.0** | **187370** | **192337** | **1.6** | **4.0** |
| | | | **Median** | **22646** | **4.0** | **195205** | **215558** | **1.9** | **5.4** |
| | | | **Max** | **25898** | **4.0** | **233726** | **248494** | **2.6** | **7.2** |
| | | | **CV%** | **9.0** | **0.0** | **8.8** | **9.5** | **19.7** | **21.4** |

(continued)

| Cohort | Period | Treatment | SubjID | $C_{max}$ (nmol/hr/mL) | $T_{max}$ (hr) | $AUC_{0-T}$ (hr*nmol/hr/mL) | $AUC_{0-\infty}$ (Hr*nmol/hr/mL) | P2/P1 $AUC_{0-\infty}$ Ratio | t½ (hr) |
|---|---|---|---|---|---|---|---|---|---|
| | | | Geometric Mean | 22651 | 4.0 | 201224 | 214751 | 2.0 | 5.2 |

Table 29. Cumulative Plasma rhGAA Activity AUCs for cohort 3.

| | 2027-0301 | | 2029-0304 | | 2029-0305 | | 2029-0306 | | 2029-0307 | | 6003-0304 | |
|---|---|---|---|---|---|---|---|---|---|---|---|---|
| **Time (hr)** | **Period 1** | **Period 2** | **Period 1** | **Period 2** | **Period 1** | **Period 2** | **Period 1** | **Period 2** | **Period 1** | **Period 2** | **Period 1** | **Period 2** |
| 0 | 0 | 0 | 0 | 0 | 0 | 0 | 0 | 0 | 0 | 0 | 0 | 0 |
| 1 | 1298 | 1133 | 1267 | 1143 | 1220 | 1393 | 792 | 1010 | 1062 | 1215 | 909 | 1301 |
| 2 | 7879 | 7326 | 8113 | 7960 | 7497 | 8969 | 5424 | 5326 | 5868 | 8077 | 5949 | 6683 |
| 3 | 21394 | 21155 | 22525 | 22418 | 21222 | 26305 | 15301 | 15140 | 16684 | 23310 | 16839 | 18909 |
| 4 | 40802 | 41579 | 43265 | 43017 | 39887 | 50408 | 28612 | 32464 | 31637 | 43887 | 33196 | 36732 |
| 5 | 59642 | 64183 | 63849 | 64625 | 56227 | 74955 | 41909 | 51551 | 44153 | 63736 | 50697 | 55908 |
| 6 | 72995 | 84032 | 79311 | 84019 | 68144 | 95856 | 53007 | 67542 | 52334 | 80847 | 64811 | 72918 |
| 7 | - | 100016 | 91047 | 100716 | 77913 | 114242 | 62208 | 81827 | 58134 | 96913 | 75545 | 88421 |
| 8 | 89787 | 113607 | 100193 | 115598 | 85835 | 130563 | 69700 | 94724 | 62129 | 111305 | 84044 | 102543 |
| 9 | 95144 | 124902 | 107586 | 129048 | 91696 | 144498 | 75249 | 106837 | 64856 | 122982 | 91391 | 115053 |
| 10 | 99116 | 134855 | 113075 | 140047 | 96015 | 156950 | 79526 | 117553 | 66898 | 133094 | 97158 | 126666 |
| 12 | 104289 | 151892 | 120450 | 158424 | 102185 | 177830 | 85692 | 134802 | 69635 | 150378 | 105787 | 146551 |
| 24 | 112800 | 187370 | 134941 | 209939 | 115349 | 233726 | 97198 | 189711 | 73754 | 189648 | 124504 | 200698 |

Table 30. Cumulative Plasma rhGAA Activity AUCs 0-Tmax and Tmax-12h for cohorts 1-3.

**Cohort 1: Period 1 rhGAA Alone, Period 2 rhGAA + 50 mg AT2220**

| Time (hr) | 2031-0101 | | | | 2031-0102 | | | | 2031-0103 | | | | 6003-0101 | | | |
|---|---|---|---|---|---|---|---|---|---|---|---|---|---|---|---|---|
| | Period 1 | Period 2 | Difference | % Change | Period 1 | Period 2 | Difference | % Change | Period 1 | Period 2 | Difference | % Change | Period 1 | Period 2 | Difference | % Change |
| $T_{max}$ | 5.0 | 5.0 | 0.0 | 0.0 | 5.0 | 6.0 | 1.0 | 20 | 5.0 | 5.0 | 0.0 | 0.0 | 4.0 | 4.0 | 0.0 | 0.0 |
| $AUC_{0\text{-}Tmax}$ | 43537 | 46504 | 2967 | 6.8 | 52486 | 68442 | 15956 | 30.4 | 29815 | 37448 | 7632 | 25.6 | 43327 | 43801 | 474 | 1.1 |
| $AUC_{Tmax\text{-}12h}$ | 96302 | 133401 | 37099 | 38.5 | 109601 | 138339 | 28739 | 26.2 | 61036 | 92644 | 31609 | 51.8 | 113716 | 142712 | 28997 | 25.5 |

**Cohort 2: Period 1 rhGAA Alone, Period 2 rhGAA + 100 mg AT2220**

| Time (hr) | 2028-0201 | | | | 2029-0202 | | | | 6003-0202 | | | | 2029-0203 | | | | 6003-0203 | | | | 2001-0201 | | | |
|---|---|---|---|---|---|---|---|---|---|---|---|---|---|---|---|---|---|---|---|---|---|---|---|---|
| | Period 1 | Period 2 | Difference | % Change | Period 1 | Period 2 | Difference | % Change | Period 1 | Period 2 | Difference | % Change | Period 1 | Period 2 | Difference | % Change | Period 1 | Period 2 | Difference | % Change | Period 1 | Period 2 | Difference | % Change |
| $T_{max}$ | 4.0 | 4.0 | 0.0 | 0.0 | 5.0 | 6.0 | 1.0 | 20.0 | 4.0 | 4.0 | 0.0 | 0.0 | 4.0 | 4.0 | 0.0 | 0.0 | 3.0 | 3.0 | 0.0 | 0.0 | 4.0 | 4.0 | 0.0 | 0.0 |
| $AUC_{0\text{-}Tmax}$ | 43259 | 46080 | 2821 | 6.5 | 70971 | 111294 | 40323 | 56.8 | 42426 | 48006 | 5580 | 13.2 | 36988 | 39704 | 2716 | 7.3 | 29546 | 31020 | 1474 | 5.0 | 44429 | 52571 | 8141 | 18.3 |
| $AUC_{Tmax\text{-}12h}$ | 114263 | 168912 | 54649 | 47.8 | 187295 | 235455 | 48160 | 25.7 | 104183 | 155687 | 51504 | 49.4 | 93505 | 133617 | 40112 | 42.9 | 136582 | 199017 | 62435 | 45.7 | 126701 | 198053 | 71352 | 56.3 |

**Cohort 3: Period 1 rhGAA Alone, Period 2 rhGAA + 250 mg AT2220**

| Time (hr) | 2027-0301 | | | | 2029-0304 | | | | 2029-0305 | | | | 2029-0306 | | | | 2029-0307 | | | | 6003-0304 | | | |
|---|---|---|---|---|---|---|---|---|---|---|---|---|---|---|---|---|---|---|---|---|---|---|---|---|
| | Period 1 | Period 2 | Difference | % Change | Period 1 | Period 2 | Difference | % Change | Period 1 | Period 2 | Difference | % Change | Period 1 | Period 2 | Difference | % Change | Period 1 | Period 2 | Difference | % Change | Period 1 | Period 2 | Difference | % Change |
| $T_{max}$ | 4.0 | 4.0 | 0.0 | 0.0 | 4.0 | 4.0 | 0.0 | 0.0 | 4.0 | 4.0 | 0.0 | 0.0 | 4.0 | 4.0 | 0.0 | 0.0 | 4.0 | 4.0 | 0.0 | 0.0 | 4.0 | 4.0 | 0.0 | 0.0 |
| $AUC_{0\text{-}Tmax}$ | 40802 | 41579 | 777 | 1.9 | 43265 | 43017 | -248 | -0.6 | 39887 | 50408 | 10521 | 26.4 | 28612 | 32464 | 3852 | 13.5 | 31637 | 43887 | 12250 | 38.7 | 33196 | 36732 | 3537 | 10.7 |
| $AUC_{Tmax\text{-}12h}$ | 104289 | 151892 | 47603 | 45.6 | 120450 | 158424 | 37974 | 31.5 | 102185 | 177830 | 75644 | 74.0 | 85692 | 134802 | 49110 | 57.3 | 69635 | 150378 | 80743 | 116.0 | 105787 | 146551 | 40764 | 38.5 |

Table 31. Mean (SD) Plasma AT2220 Profile for Cohorts 1 N=4 2 (N=6), and 3 (N=6).

| AT2220 PK Summary Table | | | | | | |
|---|---|---|---|---|---|---|
| **AT2220 Dose** | **$C_{max}$[a] (ng/mL)** | **$T_{max}$[b] (hr)** | **$AUC_{0-T}$[a] (ng·hr/mL)** | **$AUC_{0-\infty}$[a] (ng.hr/mL)** | **$AUC_{0-\infty}$ Ratio** | **t½[c] (hr)** |
| 50 mg (N=4) | 1035 (25.0) | 2.0 (1 - 3) | 6972 (28.1) | 7008 (28.2) | - | 3.1 (0.2) |
| 100 mg (N=6) | 1863 (13.1) | 3.0 (1 - 4) | 13003 (18.9) | 13137 (19.2) | 1.9 | 3.4 (0.3) |
| 250 mg (N=6) | 3150 (32.5) | 2.0 (2 - 3) | 21760 (19.8) | 22083 (19.2) | 3.2 | 3.7 0.6 |

[a]Geometric mean (CV%)
[b]Median (range)
[c]Arithmetic mean (SD)

Table 32. Plasma AT2220 PK Summary Table for Cohort 3.

| AT2220-010 Cohort 3 Plasma AT2220 PK Parameters | | | | | | | |
|---|---|---|---|---|---|---|---|
| **Cohort** | **AT2220 Dose** | **SubjID** | **$C_{max}$ (ng/mL)** | **$T_{max}$ (hr)** | **$AUC_{0-T}$ (ng*hr/mL)** | **$AUC_{0-\infty}$ (ng*hr/mL)** | **t½ (hr)** |
| Cohort 3 | 250 mg | 2027-0301 | 3620 | 2.0 | 22230 | 22345 | 3.0 |
| | | 2029-0304 | 2350 | 2.0 | 18321 | 18711 | 4.1 |
| | | 2029-0305 | 4220 | 2.0 | 24278 | 24558 | 3.8 |
| | | 2029-0306 | 2090 | 3.0 | 18707 | 19329 | 4.6 |
| | | 2029-0307 | 2740 | 2.0 | 19344 | 19534 | 3.4 |
| | | 6003-0304 | 4750 | 3.0 | 29670 | 29916 | 3.4 |
| | | **N** | **6** | **6** | **6** | **6** | **6** |
| | | **Mean** | **3295** | **2.3** | **22092** | **22399** | **3.7** |
| | | **SD** | **1071** | **0.5** | **4369** | **4299** | **0.6** |
| | | **Min** | **2090** | **2.0** | **18321** | **18711** | **3.0** |
| | | **Median** | **3180** | **2.0** | **20787** | **20940** | **3.6** |
| | | **Max** | **4750** | **3.0** | **29670** | **29916** | **4.6** |
| | | **CV%** | **32.5** | **22.1** | **19.8** | **19.2** | **15.4** |
| | | **Geometric Mean** | **3150** | **2.3** | **21760** | **22083** | **3.7** |

- Mean Cmax and AUC increased in an approximately dose-proportional manner (1.8- and 1.9-fold, respectively) from 50 mg to 100 mg.
- However, Cmax and AUC increased in a less than dose-proportional manner to 250 mg (3.0- and 3.2-fold, respectively).

Table 33. Muscle rhGAA Activity Summary of Cohorts 1-3.

| AT2220-010 Muscle rhGAA Activity All Cohorts | | | | | | | | |
|---|---|---|---|---|---|---|---|---|
| **SubjID** | **Cohort** | **Period** | **Treatment** | **Biopsy Day** | **Muscle rhGAA Activity (pmol/mg/hr)** | **P2/P1 Ratio** | **% Change from Period 1** | **% Change from Baseline** |
| 2031-0101 | Cohort 1 | Period 1 | rhGAA Alone | 7 | 1352.8 | | | |
| 2031-0101 | Cohort 1 | Period 2 | rhGAA + 50 mg AT2220 | 7 | 1398.5 | 1.0 | 3.4 | |
| 2031-0102 | Cohort 1 | Period 1 | rhGAA Alone | 7 | 1010.5 | | | |
| 2031-0102 | Cohort 1 | Period 2 | rhGAA + 50 mp AT2220 | 7 | 956.3 | 0.95 | -5.4 | |
| 2031-0103 | Cohort 1 | Period 1 | rhGAA Alone | 7 | 988.4 | | | |
| 2031-0103 | Cohort 1 | Period 2 | rhGAA + 50 mg AT2220 | 7 | 1383.1 | 1.4 | 39.9 | |
| 6003-0101 | Cohort 1 | Period 1 | rhGAA Alone | 7 | 1620.4 | | | |
| 6003-0101 | Cohort 1 | Period 2 | rhGAA + 50 mg AT2220 | 7 | 1109.0 | 0.68 | -31.6 | |
| 2028-0201 | Cohort 2 | Period 1 | rhGAA Alone | 7 | 756.4 | | | 13.6 |
| 2028-0201 | Cohort 2 | Period 2 | rhGAA + 100 mg AT2220 | 7 | 794.1 | 1.0 | 5.0 | 19.2 |
| 2028-0201 | Cohort 2 | Follow up | Baseline | 28 | 666.0 | | | |
| 2029-0202 | Cohort 2 | Period 1 | rhGAA Alone | 7 | 1019.9 | | | 178 |
| 2029-0202 | Cohort 2 | Period 2 | rhGAA + 100 mg AT2220 | 7 | 1608.9 | 1.6 | 57.8 | 339 |
| 2029-0202 | Cohort 2 | Follow up | Baseline | 28 | 366.8 | | | |
| 2029-0203 | Cohort 2 | Period 1 | rhGAA Alone | 3 | 1347.7 | | | 54.8 |
| 2029-0203 | Cohort 2 | Period 2 | rhGAA + 100 mg AT2220 | 3 | 1103.2 | 0.82 | -18.1 | 26.7 |
| 2029-0203 | Cohort 2 | Follow up | Baseline | 28 | 870.7 | | | |
| 6003-0202 | Cohort 2 | Period 1 | rhGAA Alone | 7 | 844.7 | | | |
| 6003-0202 | Cohort 2 | Period 2 | rhGAA + 100 mg AT2220 | 7 | 942.0 | 1.1 | 11.5 | |
| 6003-0203 | Cohort 2 | Period 1 | rhGAA Alone | 3 | 3908.7 | | | |

(continued)

| AT2220-010 Muscle rhGAA Activity All Cohorts | | | | | | | | |
|---|---|---|---|---|---|---|---|---|
| SubjID | Cohort | Period | Treatment | Biopsy Day | Muscle rhGAA Activity (pmol/mg/hr) | P2/P1 Ratio | % Change from Period 1 | % Change from Baseline |
| 6003-0203 | Cohort 2 | Period 2 | rhGAA + 100 mg AT2220 | 3 | 5296.6 | 1.4 | 35.5 | |
| 2001-0201 | Cohort 2 | Period 1 | rhGAA Alone | 3 | 1355.4 | | | |
| 2001-0201 | Cohort 2 | Period 2 | rhGAA + 100 mg AT2220 | 3 | 2138.5 | 1.6 | 57.8 | |
| 2027-0301 | Cohort 3 | Period 1 | rhGAA Alone | 7 | BLQ | | | |
| 2027-0301 | Cohort 3 | Period 2 | rhGAA + 250 mg AT2220 | 7 | 2090.7 | ∞ | ∞ | |
| 2029-0304 | Cohort 3 | Period 1 | rhGAA Alone | 7 | 1350.2 | | | |
| 2029-0304 | Cohort 3 | Period 2 | rhGAA + 250 mg AT2220 | 7 | 1322.0 | 1.0 | -2.1 | |
| 2029-0305 | Cohort 3 | Period 1 | rhGAA Alone | 7 | 937.9 | | | |
| 2029-0305 | Cohort 3 | Period 2 | rhGAA + 250 mg AT2220 | 7 | 1746.6 | 1.9 | 86.2 | |
| 2029-0306 | Cohort 3 | Period 1 | rhGAA Alone | 3 | 2934.9 | | | 1100 |
| 2029-0306 | Cohort 3 | Period 2 | rhGAA + 250 mg AT2220 | 3 | 2819 | 1.0 | -3.9 | 1053 |
| 2029-0306 | Cohort 3 | Follow up | Baseline | 28 | 244.5 | | | |
| 2029-0307 | Cohort 3 | Period 1 | rhGAA Alone | 3 | 2228.9 | | | |
| 2029-0307 | Cohort 3 | Period 2 | rhGAA + 250 mg AT2220 | 3 | 1883.9 | 0.85 | -15.5 | |
| 6003-0304 | Cohort 3 | Period 1 | rhGAA Alone | 3 | 2755 | | | |
| 6003-0304 | Cohort 3 | Period 2 | rhGAA + 250 mg AT2220 | 3 | 3896.9 | 1.4 | 41.4 | |
| **Mean** | **Cohort 1** | | | **Day 7** | **N=4** | **1.0** | **1.6** | |
| **Mean** | **Cohort 2** | | | **Day 3** | **N=3** | **1.3** | **25.0** | |

(continued)

| AT2220-010 Muscle rhGAA Activity All Cohorts | | | | | | | | |
|---|---|---|---|---|---|---|---|---|
| **SubjID** | **Cohort** | **Period** | **Treatment** | **Biopsy Day** | **Muscle rhGAA Activity (pmol/mg/hr)** | **P2/P1 Ratio** | **% Change from Period 1** | **% Change from Baseline** |
| **Mean** | **Cohort 2** | | | **Day 7** | **N=3** | **1.2** | **24.8** | |
| **Mean** | **Cohort 3** | | | **Day 3** | **N=3** | **1.1** | **7.3** | |
| **Mean** | **Cohort 3** | | | **Day 7** | **N=2** | **1.4** | **42.1** | |

Table 34. AT2220 Concentrations in muscle from biopsies taken 3 or 7 days post dose in cohorts 1-3.

| AT2220-010 Muscle AT2220 Conc for Cohorts 1, 2, and 3 | | | | | |
|---|---|---|---|---|---|
| **SubjID** | **Cohort** | **Dose (mg)** | **Period** | **Study Day** | **Muscle AT2220 Conc (ng/g)[a]** |
| 2031-0101 | 1 | 50 mg | Period 2 | Day 7 | 11.8 |
| 2031-0102 | 1 | 50 mg | Period 2 | Day 7 | 10.0 |
| 2031-0103 | 1 | 50 mg | Period 2 | Day 7 | 13.1 |
| 6003-0101 | 1 | 50 mg | Period 2 | Day 7 | BLQ[b] |
| 2001-0201 | 2 | 100 mg | Period 2 | Day 3 | 17.8 |
| 2028-0201 | 2 | 100 mg | Period 2 | Day 7 | BLQ[b] |
| 2028-0201 | 2 | 0 mg | Follow up | Baseline | BLQ |
| 2029-0202 | 2 | 100 mg | Period 2 | Day 7 | BLQ |
| 2029-0202 | 2 | 0 mg | Follow up | Baseline | BLQ |
| 2029-0203 | 2 | 100 mg | Period 2 | Day 3 | BLQ[c] |
| 2029-0203 | 2 | 0 mg | Follow up | Baseline | BLQ |
| 6003-0202 | 2 | 100 mg | Period 2 | Day 7 | BLQ[b] |
| 6003-0203 | 2 | 100 mg | Period 2 | Day 3 | BLQ[c] |
| 2027-0301 | 3 | 250 mg | Period 2 | Day 7 | ISV[d] |
| 2029-0304 | 3 | 250 mg | Period 2 | Day 7 | 28.1 |
| 2029-0305 | 3 | 250 mg | Period 2 | Day 7 | 27.9 |
| 2029-0306 | 3 | 250 mg | Period 2 | Day 3 | BLQ |
| 2029-0306 | 3 | 0 mg | Follow up | Baseline[e] | BLQ[c] |
| 2029-0307 | 3 | 250 mg | Period 2 | Day 3 | 54.4 |
| 6003-0304 | 3 | 250 mg | Period 2 | Day 3 | 61.9 |

[a]LLOQ = 8.0 ng/g
[b]Due to small muscle volume, sample was diluted 2-fold, therefore LLOQ = 16.0 ng/g
[c]Due to small muscle volume, sample was diluted 3-fold, therefore LLOQ = 32.0 ng/g
[d]No result due to insufficient sample for assay
[e]29 days since AT2220 administration

- Preliminary muscle AT2220 concentrations are available from biopsies taken 3 or 7 days post dose and at follow up for baseline
- 4 of 6 Cohort 3 subjects had a quantifiable AT2220 concentration in muscle.
- Dose-related increases in Cohort 3 muscle AT2220 concentrations are observed.
- Increased Cohort 3 muscle AT2220 concentrations are also study day related.

Table 35. rhGAA Activity in PBMCs: Summary of Cohorts 1-3.

| Arithmetic Mean | | | | |
|---|---|---|---|---|
| **Cohort** | **N** | **Time** | **Period 2 / Period 1 Ratio** | **% Change from Period 1** |
| Cohort 1 | 4 | 0 | 2.6 | 157 |
| Cohort 1 | 4 | 4h | 0.8 | -17.5 |
| Cohort 1 | 4 | Day 2 | 0.9 | -13.9 |
| Cohort 1 | 4 | Day 7 | 1.0 | 4.1 |
| Cohort 2 | 6 | 0 | 0.9 | -12.4 |
| Cohort 2 | 6 | 4h | 0.9 | -8.4 |
| Cohort 2 | 6 | Day 2 | 1.8 | 81.0 |
| Cohort 2 | 3 | Day 3 | 0.6 | -36.7 |
| Cohort 2 | 3 | Day 7 | 0.9 | -5.9 |
| Cohort 3 | 6 | 0 | 2.1 | 107 |
| Cohort 3 | 6 | 4h | 11.3 | 1028 |
| Cohort 3 | 6 | Day 2 | 1.5 | 47.5 |
| Cohort 3 | 3 | Day 3 | 3.4 | 240 |
| Cohort 3 | 3 | Day 7 | 1.5 | 49.0 |

Results: Cohort 4

Plasma and muscle rhGAA activity increases with co-administration of 1-deoxynojirimycin hydrochloride and acid α-glucosidase relative to acid α-glucosidase alone

For co-administration of acid α-glucosidase with 600mg 1-deoxynojirimycin hydrochloride (Period 2) relative to ERT alone (Period 1), increases in plasma rhGAA activity of cohort 4 subjects are shown in Tables 36-38. These tables also show the increase in rhGAA in muscle tissue following co-administration of acid α-glucosidase with 600mg 1-deoxynojirimycin hydrochloride (Period 2) relative to ERT alone (Period 1).

Table 36. PK Table: individual subjects and statistical summary of plasma rhGAA activity in cohort 4.

| Treatment | Period | SubjID | Cohort | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | P2/P1 $AUC_{0-\infty}$ Ratio | t½ (hr) |
|---|---|---|---|---|---|---|---|---|---|
| rhGAA Alone | Period 1 | 2033-0402 | Cohort 4 | 29604 | 4.0 | 196222 | 203729 | - | 4.7 |
| | | 2304-0401 | Cohort 4 | 23192 | 4.0 | 157216 | 159700 | - | 3.9 |
| | | 2304-0402 | Cohort 4 | 11314 | 3.0 | 59064 | 59320 | - | 3.1 |
| | | 2008-0101 | Cohort 4 | 15760 | 4.0 | 105281 | 106938 | - | 3.6 |
| | | 2030-0401 | Cohort 4 | 25506 | 4.0 | 169773 | 175606 | - | 4.7 |
| | | 2304-0403 | Cohort 4 | 18693 | 4.0 | 103687 | 103890 | - | 2.6 |
| | | 6003-0405 | Cohort 4 | 13337 | 6.0 | 91510 | 93071 | - | 3.5 |
| | | | **N** | 7 | 7 | **7** | 7 | 7 | **7** |
| | | | **Mean** | **19629** | **4.1** | **126108** | **128893** | - | **3.7** |
| | | | **SD** | **6724** | **0.9** | **49022** | **51579** | - | **0.8** |
| | | | **Min** | **11314** | **3.0** | **59064** | **59320** | - | **2.6** |
| | | | **Median** | **18693** | **4.0** | **105281** | **106938** | - | **3.6** |
| | | | **Max** | **29604** | **6.0** | **196222** | **203729** | - | **4.7** |
| | | | **CV%** | **34.3** | **21.7** | **38.9** | **40.0** | - | **20.9** |
| | | | **Geometric Mean** | **18628** | **4.1** | **117489** | **119620** | - | **3.7** |
| **Treatment** | **Period** | **SubjID** | **Cohort** | **$C_{max}$ (nmol/hr/mL)** | **$T_{max}$ (hr)** | **$AUC_{0-T}$ (hr*nmol/hr/mL)** | **$AUC_{0-\infty}$ (hr*nmol/hr/mL)** | **P2/P1 $AUC_{0-\infty}$ Ratio** | **t½ (hr)** |
| rhGAA + 600 mg AT2220 | Period 2 | 2033-0402 | Cohort 4 | 30226 | 5.0 | 297583 | 341719 | 1.7 | 7.3 |
| | | 2304-0401 | Cohort 4 | 31820 | 4.0 | 359201 | 427452 | 2.7 | 8.5 |
| | | 2304-0402 | Cohort 4 | 15145 | 4.0 | 152737 | 167209 | 2.8 | 6.3 |
| | | 2008-0101 | Cohort 4 | 17421 | 5.0 | 190273 | 215315 | 2.0 | 6.7 |
| | | 2030-0401 | Cohort 4 | 34985 | 4.0 | 345028 | 390285 | 2.2 | 7.0 |

(continued)

| Treatment | Period | SubjID | Cohort | $C_{max}$ (nmol/hr/mL) | $T_{max}$ (hr) | $AUC_{0-T}$ (hr*nmol/hr/mL) | $AUC_{0-\infty}$ (hr*nmol/hr/mL) | P2/P1 $AUC_{0-\infty}$ Ratio | t½ (hr) |
|---|---|---|---|---|---|---|---|---|---|
| | | 2304-0403 | Cohort 4 | 24031 | 3.0 | 203896 | 208703 | 2.0 | 4.0 |
| | | 6003-0405 | Cohort 4 | 13704 | 6.0 | 141283 | 157166 | 1.7 | 6.2 |
| | | | **N** | **7** | **7** | **7** | **7** | **7** | **7** |
| | | | **Mean** | **23905** | **4.4** | **241429** | **272550** | **2.2** | **6.6** |
| | | | **SD** | **8643** | **1.0** | **90992** | **111358** | **0.4** | **1.4** |
| | | | **Min** | **13704** | **3.0** | **141283** | **157166** | **1.7** | **4.0** |
| | | | **Median** | **24031** | **4.0** | **203896** | **215315** | **2.0** | **6.7** |
| | | | **Max** | **34985** | **6.0** | **359201** | **427452** | **2.8** | **8.5** |
| | | | **CV%** | **36.2** | **22.0** | **37.7** | **40.9** | **20.8** | **20.9** |
| | | | **Geometric Mean** | **22505** | **4.3** | **226809** | **253526** | **2.1** | **6.4** |

Table 37. PK Summary Table: All Cohorts (1-4).

| Period/Treatment | Cohort | Period | $C_{max}{}^{a}$ (nmol/hr/mL) | $T_{max}{}^{b}$(hr) | $AUC_{0\text{-}T}{}^{a}$ (hr*nmol/hr/mL) | $AUC_{0\text{-}\infty}{}^{a}$ (hr*nmol/hr/mL) | AUC Ratio[a] | C½[c] (hr) |
|---|---|---|---|---|---|---|---|---|
| rhGAA Alone (N=4) | 1 | 1 | 16441 (31.6) | 5.0 (4-5) | 105834 (30.7) | 107707 (31.1) | - | 3.9 (0.6) |
| rhGAA + 50 mg AT2220 (N=4) | 1 | 2 | 19922 (24.4) | 5.0 (4-6) | 152207 (21.3) | 157162 (22.4) | 1.5 (13.2) | 4.2 (0.8) |
| rhGAA Alone (N=6) | 2 | 1 | 22785 (18.0) | 4.0 (3-5) | 142415 (30.4) | 144953 (31.5) | - | 3.8 (0.6) |
| rhGAA + 100 mg AT2220 (N=6) | 2 | 2 | 28607 (13.9) | 4.0 (3-6) | 229434 (25.3) | 241203 (27.5) | 1.7 (10.1) | 4.9 (0.8) |
| rhGAA Alone (N=6) | 3 | 1 | 18986 (18.7) | 4.0 (4-4) | 107774 (19.7) | 109165 (20.0) | - | 3.6 (0.5) |
| rhGAA + 250 mg AT2220 (N=6) | 3 | 2 | 22651 (9.0) | 4.0 (4-4) | 201224 (8.8) | 214751 (9.5) | 2.0 (19.7) | 5.3 (1.1) |
| rhGAA Alone (N=7) | 4 | 1 | 18628 (34.3) | 4.0 (3-6) | 117489 (38.9) | 119620 (40.0) | - | 3.7 (0.8) |
| rhGAA + 600 mg AT2220 (N=7) | 4 | 2 | 22505 (36.2) | 4.0 (3-6) | 226809 (37.7) | 253526 (40.9) | 2.1 (20.8) | 6.6 (1.4) |

[a]Geomatric mean (CV%)
[b]Median (range)
[c]Arithmetic mean (SD)

Table 38. AT2220-010 Muscle rhGAA Activity Summary of Cohort 4 Subjects.

| SubjID | Cohort | Period | Treatment | Biopsy Day | Muscle rhGAA Activity (pmol/mg/hr) | P2/P1 Ratio | Tx/BSL Ratio |
|---|---|---|---|---|---|---|---|
| 2033-0402 | Cohort 4 | Period 1 | rhGAA Alone | 3 | 1020.0 | | 2.2 |
| 2033-0402 | Cohort 4 | Period 2 | rhGAA + 600 mg AT2220 | 3 | 3052.7 | 3.0 | 6.7 |
| 2033-0402 | Cohort 4 | Follow up | Baseline | 28 | 454.8 | | |
| 2304-0401 | Cohort 4 | Period 1 | rhGAA Alone | 7 | 1179.6 | | |
| 2304-0401 | Cohort 4 | Period 2 | rhGAA + 600 mq AT2220 | 7 | 1261.2 | 1.1 | |
| 2304-0402 | Cohort 4 | Period 1 | rhGAA Alone | 7 | 901.6 | | 1.2 |
| 2304-0402 | Cohort 4 | Period 2 | rhGAA + 600 mg AT2220 | 7 | 991.1 | 1.1 | 1.3 |
| 2304-0402 | Cohort 4 | Follow up | Baseline | 28 | 765.1 | | |
| 2008-0101 | Cohort 4 | Period 1 | rhGAA Alone | 3 | 2262.2 | | 2.2 |
| 2008-0101 | Cohort 4 | Period 2 | rhGAA + 600 mg AT2220 | 3 | 3785.9 | 1.7 | 3.6 |
| 2008-0101 | Cohort 4 | Follow up | Baseline | Follow-up | 1047.3 | | |
| 2030-0401 | Cohort 4 | Period 1 | rhGAA Alone | 3 | a | | |
| 2030-0401 | Cohort 4 | Period 2 | rhGAA + 600 mg AT2220 | 3 | 1722.3 | N/A | |
| 2304-0403 | Cohort 4 | Period 1 | rhGAA Alone | 3 | b | | N/A |
| 2304-0403 | Cohort 4 | Period 2 | rhGAA + 600 mg AT2220 | 3 | b | N/A | N/A |
| 2304-0403 | Cohort 4 | Follow up | Baseline | Follow-up | b | | |
| 6003-0405 | Cohort 4 | Period 1 | rhGAA Alone | 7 | 723.6 | | |
| 6003-0405 | Cohort 4 | Period 2 | rhGAA + 600 mg AT2220 | 7 | 1021.3 | 1.4 | |

[a] Muscle activty could not be accurately determined due to low protein levels from a presumed fatty tissue sample
[b] Biopsy samples mislabed at the site; could not be confirmed

Summary of Results

For people with Pompe disease, deficient GAA enzyme leads to the accumulation of glycogen in tissues affected by disease (primarily muscle). Preclinical data (Khanna et al. PLoS ONE (2012) 7(7): e40776. doi:10.1371/journal.pone.0040776) demonstrated that AT2220 in combination with ERT enhances rhGAA enzyme activity, reduces glycogen accumulation, and potentially mitigates ERT-related immunogenicity in a mouse model of Pompe disease. In the present study described by Examples 1-3, co-administration of AT2220 to Pompe patients increased rhGAA enzyme activity and enhanced rhGAA enzyme uptake into muscle tissue compared to ERT alone.

The study described by Examples 1-3 is a Phase 2 open-label, multi-center study to evaluate the safety and

PK effects of four increasing oral doses of AT2220 (50 mg (Cohort 1), 100 mg (Cohort 2), 250 mg (Cohort 3), or 600 mg (Cohort 4)) co-administered with ERT (Myozyme®/Lumizyme®) versus ERT alone in males and females with Pompe disease. The study enrolled male and female patients who had been on a stable dose and regimen of ERT for at least three months. All patients were given a regularly scheduled ERT infusion. One hour prior to the initiation of the next ERT infusion, patients received a single oral dose of AT2220. Plasma rhGAA activity and protein levels were evaluated during each infusion. Each patient underwent muscle biopsies three or seven days after each infusion to measure tissue GAA enzyme activity with and without the chaperone, as well as to measure the level of AT2220 in the muscle.

Safety: Single doses of AT2220 co-administered with ERT were well-tolerated, with no drug-related adverse events reported. In addition, AT2220 was cleared from muscle to near-undetectable levels by Day 7 in all four cohorts.

Recombinant Human GAA (rhGAA) Enzyme Activity in Plasma: 24-hour plasma pharmacokinetics (PK) was measured during and after each infusion. Plasma rhGAA activity increased in 23 out of 23 patients (100%) following co-administration and the increases were dose-related. These data suggest that co-administration increases the amount of stabilized, properly folded, and active rhGAA enzyme available for uptake into tissue. Table 39 and Figure 21 show a summary of rhGAA enzyme activity in plasma area under curve (AUC) for cohorts 1-4 described by Examples 1-3.

Table 39. rhGAA enzyme activity in plasma area under curve (AUC) for cohorts 1-4.

| rhGAA Enzyme Activity in Plasma Area Under Curve (AUC) ERT + AT2220 vs. ERT Alone | |
|---|---|
| **Cohort** | **% Increase vs. ERT Alone** |
| 1 (n=4) | 50% |
| 2 (n=6) | 70% |
| 3 (n=6) | 100% |
| 4 (n=7) | 110% |

**Enzyme Activity in Muscle:** Muscle biopsies were taken to measure GAA enzyme uptake into muscle tissue, with and without AT2220. In Cohort 1, all 4 patients had muscle biopsies on Day 7. In Cohorts 2-4, muscle biopsies were taken on Day 3 for half the patients, and on Day 7 for the other half of patients.

In Cohort 1, no consistent change in GAA enzyme activity was observed at day 7. In Cohorts 2, 3, and 4 the results show that more enzyme is taken up into muscle tissue following AT2220 co-administration compared to ERT alone. The effect was most pronounced at the highest (600 mg) dose of AT2220. Table 40 and Figure 22 show a summary of GAA enzyme activity in muscle at day 3 for cohorts 2-4 described by Examples 1-3.

Table 40. GAA enzyme activity in muscle at day 3 for cohorts 2-4.

| ***GAA Enzyme Activity in Muscle at Day 3*** | |
|---|---|
| ***ERT + AT2220 vs. ERT Alone*** | |
| **Cohort** | **% Increase vs. ERT Alone** |
| 2 (n=3) | 25% |
| 3 (n=3) | 7% |
| 4 (n=2) | 133% |

At Day 3 the GAA enzyme activity in muscle following co-administration compared to ERT alone in patients with evaluable biopsies increased by the following: 25% in Cohort 2 (n=3), 7% in Cohort 3 (n=3), and 133% in Cohort 4 (n=2). At Day 7 the GAA enzyme activity in muscle was lower relative to Day 3, as expected based on the cellular half-life of the enzyme. However, following co-administration compared to ERT alone in patients with evaluable biopsies the following increases were sustained: 20% in Cohort 2 (n=3), 40% in Cohort 3 (n=2), and 20% in Cohort 4 (n=3).

**Effect of AT2220 on ERT-Related Immunogenicity Measured ex vivo:** By stabilizing the folded and active form of the rhGAA enzyme, AT2220 may mitigate ERT-induced immunogenicity since unfolded and aggregated proteins are generally more antigenic than properly folded proteins. Recent published studies show that approximately 40% of the administered ERT can be captured by circulating antibodies and infusion associated reactions occur in approximately 50% of Pompe patients receiving ERT infusions (Banati et al., Muscle Nerve. 2011 Nov;44(5):720-6). Initial ex vivo studies using T cells derived from blood from 50 healthy donors demonstrated that the addition of AT2220 may significantly reduce the immunogenicity of Myozyme® and Lumizyme®. The studies utilized Antitope Ltd.'s EpiScreen™ assay and are being repeated in samples from the Pompe patients in the study described by Examples 1-3.

**EXAMPLE 4: Skeletal muscle distribution and half-life of N-butyl-DNJ (AT2221) is similar to 1-DNJ (AT2220)**

Eight-week old wild type C57BL/6 mice where administered an oral dose of 100mg/kg of 1-DNJ or N-butyl-DNJ. Plasma and tissue samples were taken at 0.5, 2, 4, 24, 48, 72, 96, 120, 144, & 168 hours post administration and the presence of drug was analyzed. The concentration of drug in plasma is expressed as ng/ml. The concentration of drug in tissue samples is expressed as ng/g.

As shown in Figure 23, the skeletal muscle distribution and half-life of N-butyl-DNJ (AT2221) is similar to 1-DNJ (AT2220). The Cmax of AT2220 was 120 uM. The Cmax of AT2221 was 140 uM.

**EXAMPLE 5: N-butyl-DNJ (AT2221) and 1-DNJ (AT2220) have a similar effect on Phramacokinetics of rhGAA.**

Eight week old GAA KO mice were administered rhGAA (10 mg/kg IV). Oral AT2220 or AT2221 (100 mg/kg) was administered 30 min prior to GAA (Myozyme); Plasma samples were taken at pre-dose, 0.08, 0.25, 0.50, 0.75,1, 2, 4, 8, and 24 hours after administration of GAA and the enzyme activity was determined.

As shown in Figure 24, AT2220 and AT2221 increased the circulating half-life of rhGAA by at least ~2-fold. N-butyl-DNJ (AT2221) and 1-DNJ (AT2220) have a similar effect on Phramacokinetics of rhGAA.

A Western blot of recombinant GAA in plasma at 2, 8, and 24 hours after i.v. administration of GAA is shown in Figure 25 for mice administered rhGAA with or without AT2220 or AT2221.

**EXAMPLE 6: Coadministration of DNJ or NB-DNJ with rhGAA has a similar effect on glycogen reduction**

Twelve week old GAA KO mice were administered 20 mg/kg i.v. recombinant human GAA (Myozyme) every other week for 8 weeks. An oral dose of AT2220 or AT2221 (30 mg/kg) was administered 30 minutes prior to rhGAA (Myozyme). Tissues were collected 21 days after the last dose of rhGAA and the level of glycogen (GAA substrate) was measured.

As shown in Figure 26, coadministration of DNJ or NB-DNJ with rhGAA has a similar effect on glycogen reduction. n=5-mice/group; $*p<0.05$ vs. untreated t-test; $\#p<0.05$ vs. Myozyme alone t-test; Dotted line indicates wild type glycogen levels. Cmax ~40 μM following administration of 30 mg/kg AT2220 or AT2221; equivalent to approximately 600 mg in humans.

## Claims

1. 1-Deoxynojirimycin (1-DNJ), n-butyl deoxynojirimycin (NB-DNJ), or a pharmaceutically acceptable salt thereof and an effective amount of human recombinant acid α-glucosidase enzyme replacement therapy for use in a method for the treatment of Pompe disease in a human subject; wherein:
   the 1-DNJ, the NB-DNJ, or the pharmaceutically acceptable salt thereof is administered in an amount of 250 mg or 600 mg and is administered from 4 hours prior to, to immediately before the administration of the human recombinant acid α-glucosidase enzyme replacement therapy.

2. A kit for the treatment of Pompe disease in a human subject, the kit comprising 250 mg or 600 mg of 1-deoxynojirimycin (1-DNJ), n-butyl deoxynojirimycin (NB-DNJ), or a pharmaceutically acceptable salt thereof, and an effective amount of human recombinant acid α-glucosidase enzyme replacement therapy, wherein the 1-DNJ, NB-DNJ, or pharmaceutically acceptable salt thereof is administered from 4 hours prior to, to immediately before the administration of the human recombinant acid α-glucosidase enzyme replacement therapy.

3. 1-DNJ, NB-DNJ, or a pharmaceutically acceptable salt thereof, and human recombinant acid α-glucosidase enzyme replacement therapy for use according to claim 1, wherein the 1-DNJ, NB-DNJ, or pharmaceutically acceptable salt thereof is administered about 1 hour before the administration of the human recombinant acid α-glucosidase enzyme replacement therapy.

4. The kit for use according to claim 2, wherein the 1-DNJ, NB-DNJ, or pharmaceutically acceptable salt thereof is administered about 1 hour before the administration of the human recombinant acid α-glucosidase enzyme replacement therapy.

5. 1-DNJ, NB-DNJ, or a pharmaceutically acceptable salt thereof, and human recombinant acid α-glucosidase enzyme replacement therapy for use according to claim 1, wherein the patient fasts for a period of time beginning 0.5 to 4 hours prior to and ending 0.5 to 4 hours following administration of the 1-DNJ, NB-DNJ, or pharmaceutically acceptable salt thereof.

6. 1-DNJ, NB-DNJ, or a pharmaceutically acceptable salt thereof, and human recombinant acid α-glucosidase enzyme replacement therapy for use according to claim 5, wherein the patient fasts for at least 2 hours prior to and at least 2 hours following administration of the 1-DNJ, NB-DNJ, or pharmaceutically acceptable salt thereof.

7. 1-DNJ, NB-DNJ, or a pharmaceutically acceptable salt thereof, and human recombinant acid α-glucosidase enzyme replacement therapy for use according to claim 1, wherein the 1-DNJ, NB-DNJ, or pharmaceutically acceptable salt thereof is administered about 2 hours prior to the administration of the human recombinant acid α-glucosidase enzyme replacement therapy.

8. 1-DNJ, NB-DNJ, or a pharmaceutically acceptable salt thereof, and human recombinant acid α-glucosidase enzyme replacement therapy for use according to claim 6, wherein the 1-DNJ, NB-DNJ, or pharmaceutically acceptable salt thereof is administered about 1 hour prior to the administration of the human recombinant acid α-glucosidase enzyme replacement therapy.

9. 1-DNJ, NB-DNJ, or a pharmaceutically acceptable salt thereof, and human recombinant acid α-glucosidase enzyme replacement therapy for use according to claim 1, wherein the human recombinant acid α-glucosidase enzyme replacement therapy is alglucosidase alfa.

10. 1-DNJ, NB-DNJ, or a pharmaceutically acceptable salt thereof, and human recombinant acid α-glucosidase enzyme replacement therapy for use according to claim 1, wherein the patient is administered a second dose of the 1-DNJ, NB-DNJ, or pharmaceutically acceptable salt thereof between the administration of the human recombinant acid α-glucosidase enzyme replacement therapy and about 4 hours thereafter.

11. 1-DNJ, NB-DNJ, or a pharmaceutically acceptable salt thereof and human recombinant acid α-glucosidase enzyme replacement therapy for use according to any one of claims 3, 7, or 8, wherein the 1-DNJ, NB-DNJ or pharmaceutically acceptable salt thereof and human recombinant acid α-glucosidase enzyme replacement therapy are administered every 1 to 4 weeks.

12. 1-DNJ, NB-DNJ, or a pharmaceutically acceptable salt thereof, and human recombinant acid α-glucosidase enzyme replacement therapy for use according to claim 11, wherein the 1-DNJ, NB-DNJ, or pharmaceutically acceptable salt thereof and human recombinant acid α-glucosidase enzyme replacement therapy are administered every 2 weeks.

**Patentansprüche**

1. 1-Deoxynojirimycin (1-DNJ), n-Butyldeoxynojirimycin (NB-DNJ) oder ein pharmazeutisch annehmbares Salz davon und eine wirksame Menge an humaner rekombinanter saurer α-Glucosidase-Enzymersatztherapie zur Verwendung in einem Verfahren zur Behandlung von Morbus Pompe bei einem Menschen; wobei:
das 1-DNJ, das NB-DNJ oder das pharmazeutisch annehmbare Salz davon in einer Menge von 250 mg oder 600 mg verabreicht wird und von 4 Stunden vor bis unmittelbar vor der Verabreichung der humanen rekombinanten sauren α-Glucosidase-Enzymersatztherapie verabreicht wird.

2. Kit für die Behandlung von Morbus Pompe bei einem Menschen, wobei der Kit 250 mg oder 600 mg 1-Desoxynojirimycin (1-DNJ), n-Butyldeoxynojirimycin (NB-DNJ) oder ein pharmazeutisch annehmbares Salz davon und eine wirksame Menge an humaner rekombinanter saurer α-Glucosidase-Enzymersatztherapie enthält, wobei das 1-DNJ, das NB-DNJ oder das pharmazeutisch annehmbare Salz davon von 4 Stunden vor bis unmittelbar vor der Verabreichung der humanen rekombinanten sauren α-Glucosidase-Enzymersatztherapie verabreicht wird.

3. 1-DNJ, NB-DNJ oder ein pharmazeutisch annehmbares Salz davon und humane rekombinante saure α-Glucosidase-Enzymersatztherapie zur Verwendung nach Anspruch 1, wobei das 1-DNJ, das NB-DNJ oder das pharmazeutisch annehmbare Salz davon etwa 1 Stunde vor der Verabreichung der humanen rekombinanten sauren α-Glucosidase-Enzymersatztherapie verabreicht wird.

4. Kit zur Verwendung nach Anspruch 2, wobei das 1-DNJ, das NB-DNJ oder das pharmazeutisch annehmbare Salz davon etwa 1 Stunde vor der Verabreichung der humanen rekombinanten sauren α-Glucosidase-Enzymersatztherapie verabreicht wird.

5. 1-DNJ, NB-DNJ oder ein pharmazeutisch annehmbares Salz davon und humane rekombinante saure α-Glucosidase-Enzymersatztherapie zur Verwendung nach Anspruch 1, wobei der Patient für einen Zeitraum fastet, der 0,5 bis 4 Stunden vor und 0,5 bis 4 Stunden nach Verabreichung des 1-DNJ, NB-DNJ oder eines pharmazeutisch annehmbaren Salzes davon beginnt bzw. endet.

6. 1-DNJ, NB-DNJ oder ein pharmazeutisch annehmbares Salz davon und humane rekombinante saure α-Glucosidase-Enzymersatztherapie zur Verwendung nach Anspruch 5, wobei der Patient mindestens 2 Stunden vor und mindestens 2 Stunden nach der Verabreichung des 1-DNJ, NB-DNJ oder eines pharmazeutisch annehmbaren Salzes davon fastet.

7. 1-DNJ, NB-DNJ oder ein pharmazeutisch annehmbares Salz davon und humane rekombinante saure α-Glucosidase-Enzymersatztherapie zur Verwendung nach Anspruch 1, wobei das 1-DNJ, das NB-DNJ oder das pharmazeutisch annehmbare Salz davon etwa 2 Stunden vor der Verabreichung der humanen rekombinanten sauren α-Glucosidase-Enzymersatztherapie verabreicht wird.

8. 1-DNJ, NB-DNJ oder ein pharmazeutisch annehmbares Salz davon und humane rekombinante saure α-Glucosidase-Enzymersatztherapie zur Verwendung nach Anspruch 6, wobei das 1-DNJ, das NB-DNJ oder das pharmazeutisch annehmbare Salz davon etwa 1 Stunde vor der Verabreichung der humanen rekombinanten sauren α-Glucosidase-Enzymersatztherapie verabreicht wird.

9. 1-DNJ, NB-DNJ oder ein pharmazeutisch annehmbares Salz davon und humane rekombinante saure α-Glucosidase-Enzymersatztherapie zur Verwendung nach Anspruch 1, wobei die humane rekombinante saure α-Glucosidase-Enzymersatztherapie Alglucosidase alfa ist.

10. 1-DNJ, NB-DNJ oder ein pharmazeutisch annehmbares Salz davon und humane rekombinante saure α-Glucosidase-Enzymersatztherapie zur Verwendung nach Anspruch 1, wobei dem Patienten eine zweite Dosis des 1-DNJ, NB-DNJ oder eines pharmazeutisch annehmbaren Salzes davon zwischen der Verabreichung der humanen rekombinanten sauren α-Glucosidase-Enzymersatztherapie und etwa 4 Stunden danach verabreicht wird.

11. 1-DNJ, NB-DNJ oder ein pharmazeutisch annehmbares Salz davon und humane rekombinante saure α-Glucosidase-Enzymersatztherapie zur Verwendung nach einem der Ansprüche 3, 7 oder 8, wobei das 1-DNJ, das NB-DNJ oder das pharmazeutisch annehmbare Salz davon und die humane rekombinante saure α-Glucosidase-Enzymersatztherapie in Abständen von 1 bis 4 Wochen verabreicht werden.

12. 1-DNJ, NB-DNJ oder ein pharmazeutisch annehmbares Salz davon und humane rekombinante saure α-Glucosidase-Enzymersatztherapie zur Verwendung nach Anspruch 11, wobei das 1-DNJ, das NB-DNJ oder das pharmazeutisch annehmbare Salz davon und die humane rekombinante saure α-Glucosidase-Enzymersatztherapie in Abständen von 2 Wochen verabreicht werden.

## Revendications

1. 1-désoxynojirimycine (1-DNJ), n-butyl désoxynojirimycine (NB-DNJ), ou un sel pharmaceutiquement acceptable de celles-ci et une quantité efficace d'une enzymothérapie substitutive par α-glucosidase acide humaine recombinante pour leur utilisation dans un procédé de traitement de la maladie de Pompe chez un sujet humain ; dans lesquels : la 1-DNJ, la NB-DNJ, ou le sel pharmaceutiquement acceptable de celles-ci est administré en une quantité de 250 mg ou 600 mg et est administré de 4 heures avant, à immédiatement avant l'administration de l'enzymothérapie substitutive par α-glucosidase acide humaine recombinante.

2. Kit pour le traitement de la maladie de Pompe chez un sujet humain, le kit comprenant 250 mg ou 600 mg de 1-désoxynojirimycine (1-DNJ), de n-butyl désoxynojirimycine (NB-DNJ), ou d'un sel pharmaceutiquement acceptable de celles-ci, et une quantité efficace d'enzymothérapie substitutive par α-glucosidase acide humaine recombinante, dans lequel la 1-DNJ, la NB-DNJ, ou un sel pharmaceutiquement acceptable de celles-ci est administré de 4 heures avant, à immédiatement avant l'administration de l'enzymothérapie substitutive par α-glucosidase acide humaine recombinante.

3. 1-DNJ, NB-DNJ, ou un sel pharmaceutiquement acceptable de celles-ci, et enzymothérapie substitutive par α-glucosidase acide humaine recombinante pour leur utilisation selon la revendication 1, dans lesquelles la 1-DNJ, la NB-DNJ, ou le sel pharmaceutiquement acceptable de celles-ci est administré environ 1 heure avant l'administration de l'enzymothérapie substitutive par α-glucosidase acide humaine recombinante.

4. Kit pour son utilisation selon la revendication 2, dans lequel la 1-DNJ, la NB-DNJ, ou un sel pharmaceutiquement acceptable de ceux-ci est administré environ 1 heure avant l'administration de l'enzymothérapie substitutive par α-glucosidase acide humaine recombinante.

5. 1-DNJ, NB-DNJ, ou un sel pharmaceutiquement acceptable de celles-ci, et enzymothérapie substitutive par α-glucosidase acide humaine recombinante pour leur utilisation selon la revendication 1, dans lesquels le patient reste à jeun pendant une période commençant 0,5 à 4 heures avant et se terminant 0,5 à 4 heures après l'administration de 1-DNJ, de NB-DNJ, ou d'un sel pharmaceutiquement acceptable de celles-ci.

6. 1-DNJ, NB-DNJ, ou un sel pharmaceutiquement acceptable de celles-ci, et enzymothérapie substitutive par α-glucosidase acide humaine recombinante pour leur utilisation selon la revendication 5, dans lesquels le patient reste à jeun pendant au moins 2 heures avant et au moins 2 heures après l'administration de 1-DNJ, de NB-DNJ, ou d'un sel pharmaceutiquement acceptable de celles-ci.

7. 1-DNJ, NB-DNJ, ou un sel pharmaceutiquement acceptable de celles-ci, et enzymothérapie substitutive par α-glucosidase acide humaine recombinante pour leur utilisation selon la revendication 1, dans lesquels la 1-DNJ, la NB-DNJ, ou un sel pharmaceutiquement acceptable de celles-ci est administré pendant environ 2 heures avant l'administration de l'enzymothérapie substitutive par α-glucosidase acide humaine recombinante.

8. 1-DNJ, NB-DNJ, ou un sel pharmaceutiquement acceptable de celles-ci, et enzymothérapie substitutive par α-glucosidase acide humaine recombinante pour leur utilisation selon la revendication 6, dans lesquels la 1-DNJ, la NB-DNJ, ou un sel pharmaceutiquement acceptable de celles-ci est administré pendant environ 1 heure avant l'administration de l'enzymothérapie substitutive par α-glucosidase acide humaine recombinante.

9. 1-DNJ, NB-DNJ, ou un sel pharmaceutiquement acceptable de celles-ci, et enzymothérapie substitutive par α-glucosidase acide humaine recombinante pour leur utilisation selon la revendication 1, dans lesquels l'enzymothérapie substitutive par α-glucosidase acide humaine recombinante est l'alglucosidase alfa.

10. 1-DNJ, NB-DNJ, ou un sel pharmaceutiquement acceptable de celles-ci, et une enzymothérapie substitutive par α-glucosidase acide humaine recombinante pour leur utilisation selon la revendication 1, dans lesquels est administrée au patient une seconde dose de 1-DNJ, de NB-DNJ, ou d'un sel pharmaceutiquement acceptable de celles-ci entre l'administration de l'enzymothérapie substitutive par α-glucosidase acide humaine recombinante et environ 4 heures après celle-ci.

11. 1-DNJ, NB-DNJ, ou un sel pharmaceutiquement acceptable de celles-ci, et enzymothérapie substitutive par α-glucosidase acide humaine recombinante pour leur utilisation selon les revendications 3, 7, ou 8, dans lesquelles la 1-DNJ, la NB-DNJ, ou un sel pharmaceutiquement acceptable de celles-ci et l'enzymothérapie substitutive par α-glucosidase acide humaine recombinante sont administrés toutes les 1 à 4 semaines.

12. 1-DNJ, NB-DNJ, ou un sel pharmaceutiquement acceptable de celles-ci, et enzymothérapie substitutive par α-glucosidase acide humaine recombinante pour leur utilisation selon la revendication 11, dans lesquels la 1-DNJ, la NB-DNJ, ou un sel pharmaceutiquement acceptable de celles-ci et l'enzymothérapie substitutive par α-glucosidase acide humaine recombinante sont administrés toutes les 2 semaines.

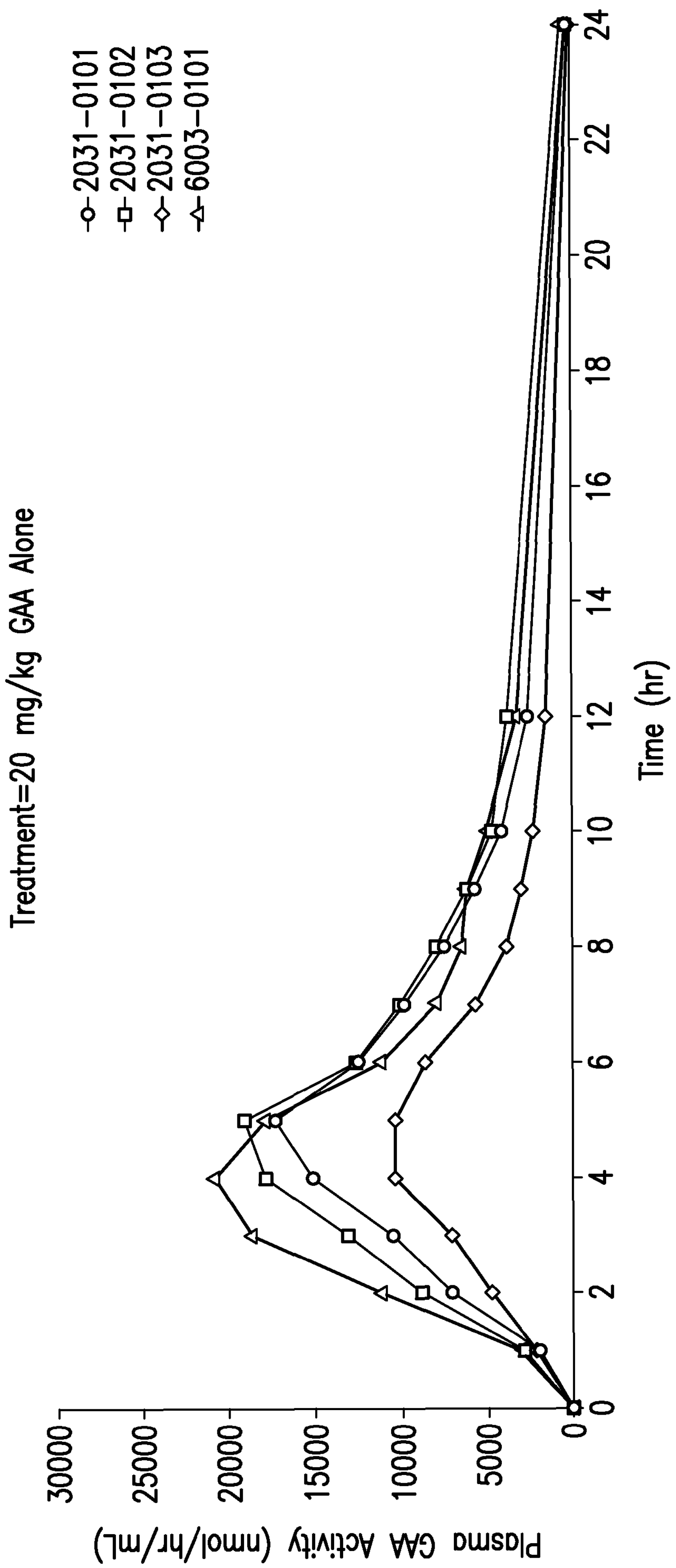
Treatment=20 mg/kg GAA Alone
-o-2031-0101
-□-2031-0102
-◇-2031-0103
-△-6003-0101
Plasma GAA Activity (nmol/hr/mL)
0
5000
10000
15000
20000
25000
30000
0
2
4
6
8
10
12
14
16
18
20
22
24
Time (hr)

FIG. 1A

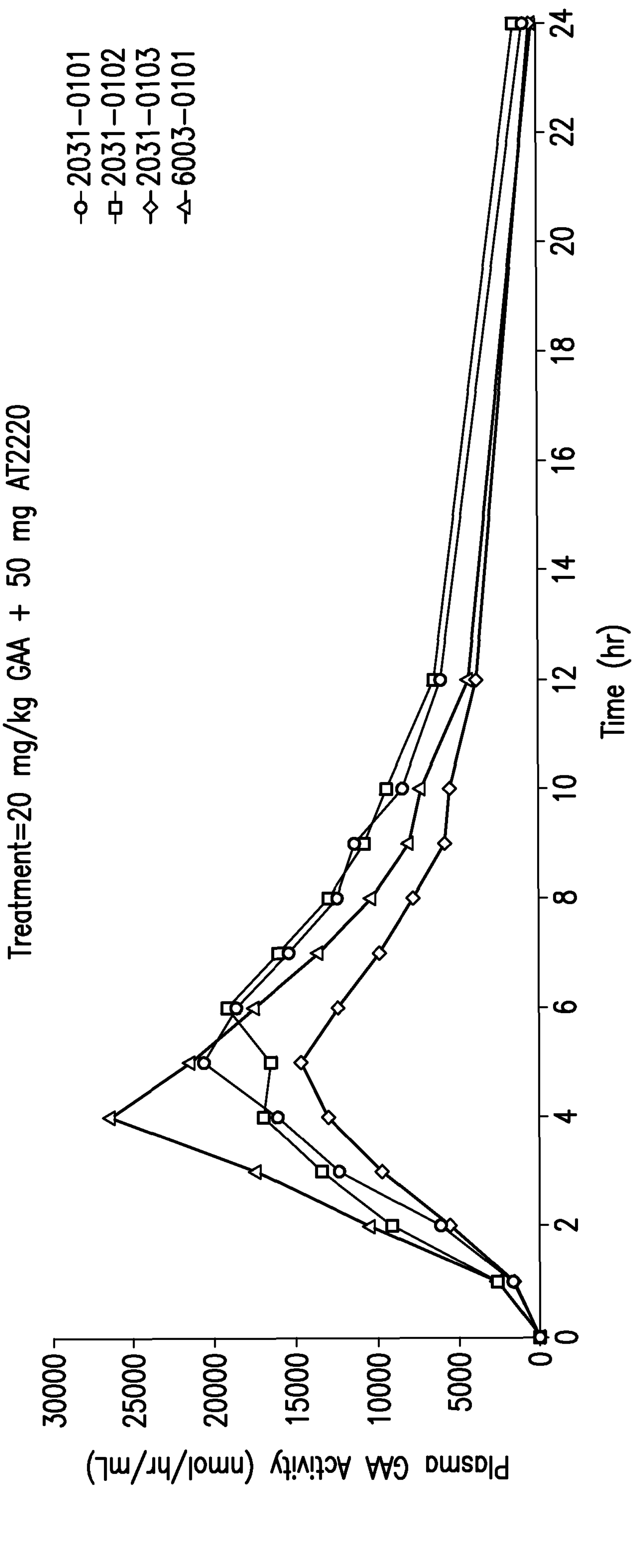
Treatment=20 mg/kg GAA + 50 mg AT2220
2031−0101
2031−0102
2031−0103
6003−0101
Plasma GAA Activity (nmol/hr/mL)
30000
25000
20000
15000
10000
5000
0
0
2
4
6
8
10
12
14
16
18
20
22
24
Time (hr)

FIG. 1B

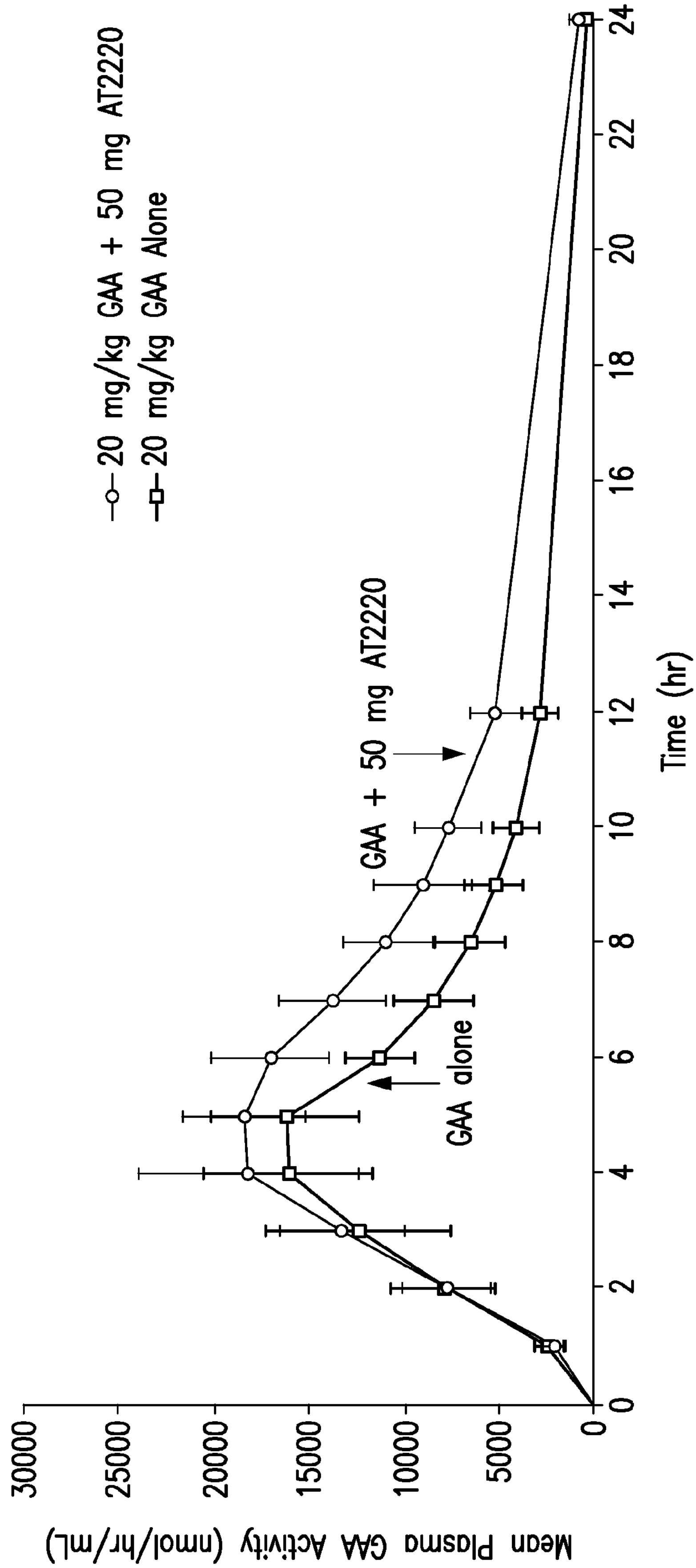

20 mg/kg GAA + 50 mg AT2220
20 mg/kg GAA Alone
GAA + 50 mg AT2220
GAA alone
Mean Plasma GAA Activity (nmol/hr/mL)
30000
25000
20000
15000
10000
5000
0
0
2
4
6
8
10
12
14
16
18
20
22
24
Time (hr)


FIG. 2A

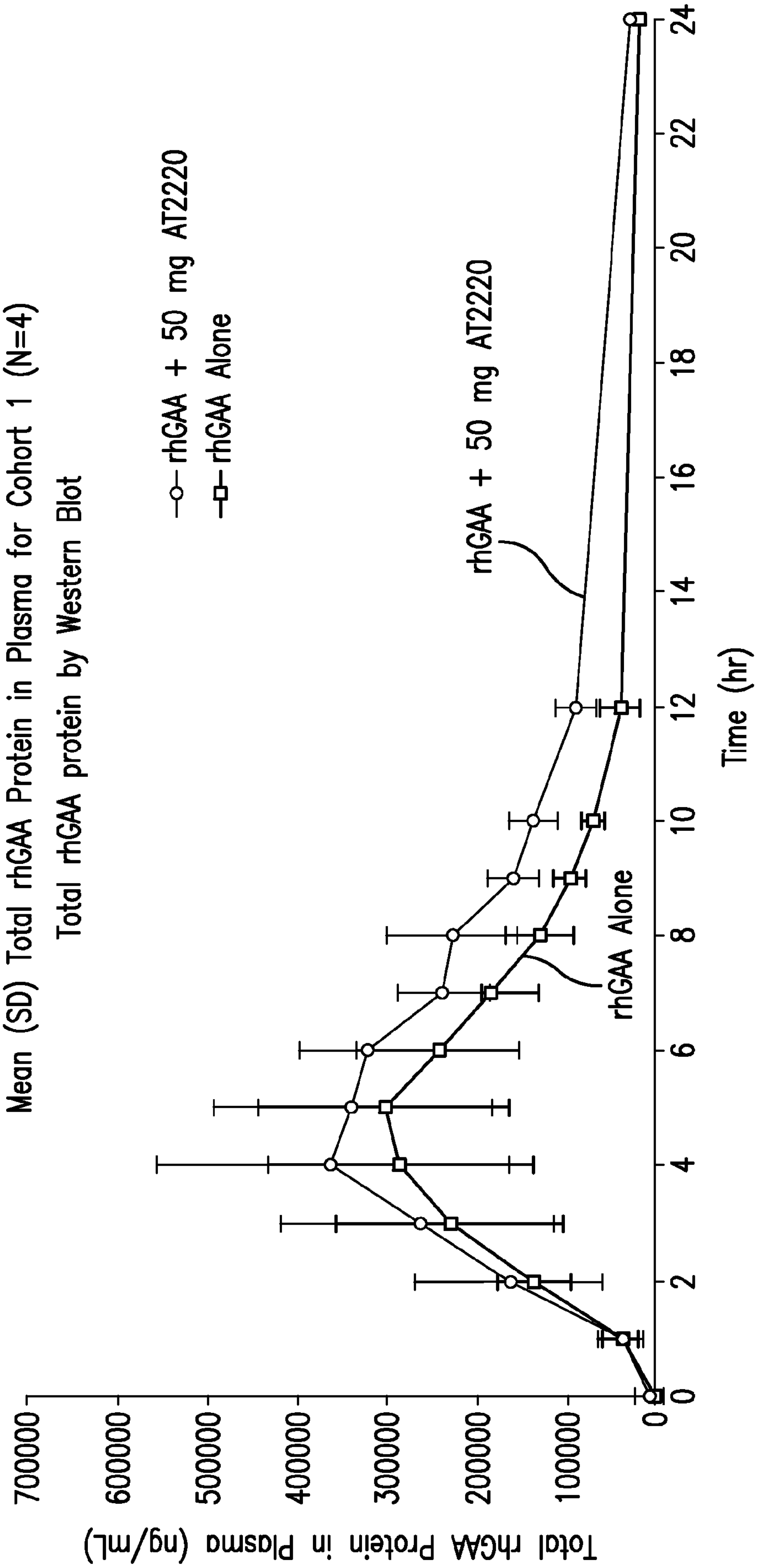
Mean (SD) Total rhGAA Protein in Plasma for Cohort 1 (N=4)
Total rhGAA protein by Western Blot
rhGAA + 50 mg AT2220
rhGAA Alone
rhGAA + 50 mg AT2220
rhGAA Alone
Total rhGAA Protein in Plasma (ng/mL)
700000
600000
500000
400000
300000
200000
100000
0
0
2
4
6
8
10
12
14
16
18
20
22
24
Time (hr)

FIG. 2B

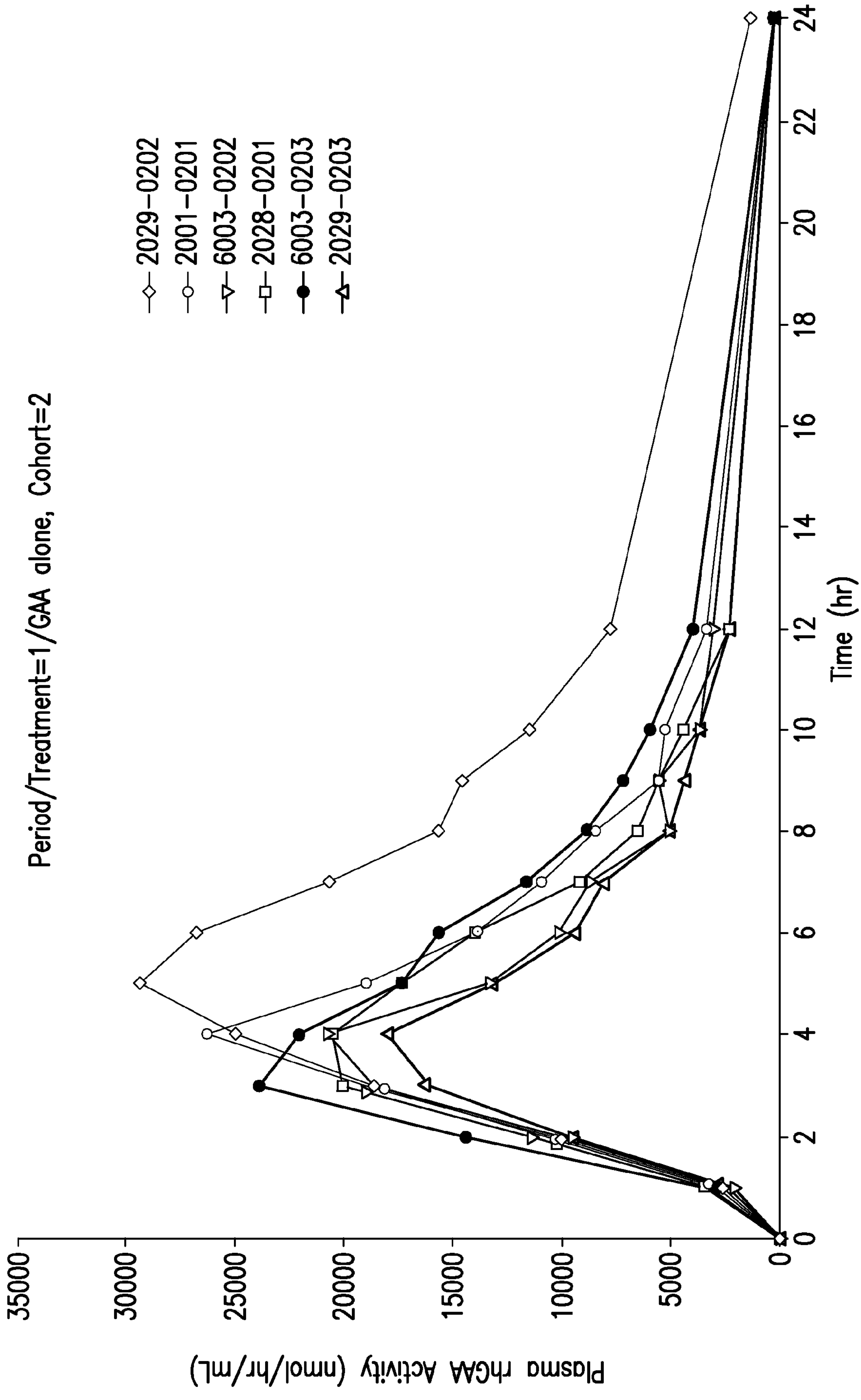
Period/Treatment=1/GAA alone, Cohort=2
2029-0202
2001-0201
6003-0202
2028-0201
6003-0203
2029-0203
Plasma rhGAA Activity (nmol/hr/mL)
35000
30000
25000
20000
15000
10000
5000
0
0
2
4
6
8
10
12
14
16
18
20
22
24
Time (hr)

FIG. 3A

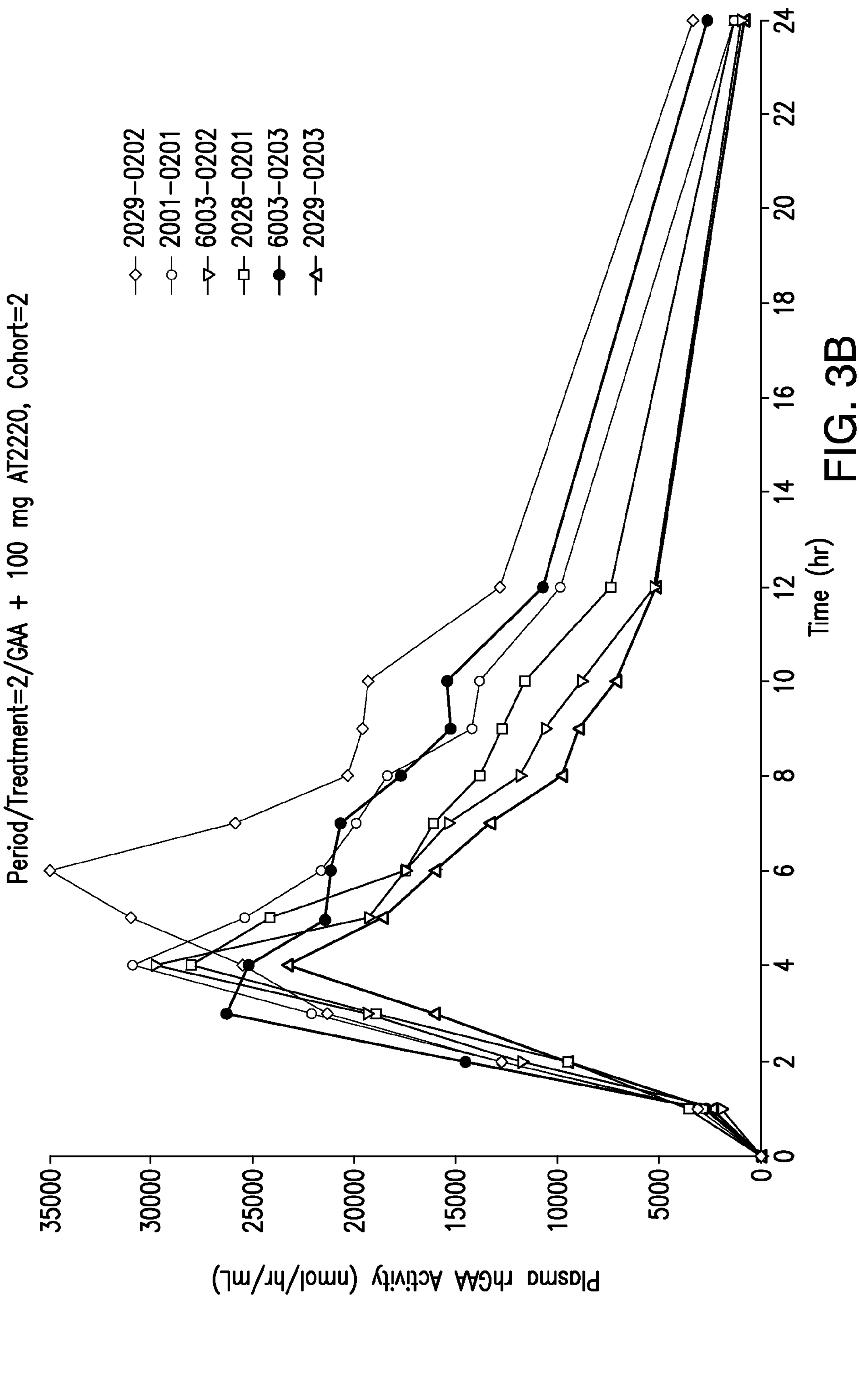
Period/Treatment=2/GAA + 100 mg AT2220, Cohort=2
2029-0202
2001-0201
6003-0202
2028-0201
6003-0203
2029-0203
Plasma rhGAA Activity (nmol/hr/mL)
35000
30000
25000
20000
15000
10000
5000
0
0
2
4
6
8
10
12
14
16
18
20
22
24
Time (hr)

FIG. 3B

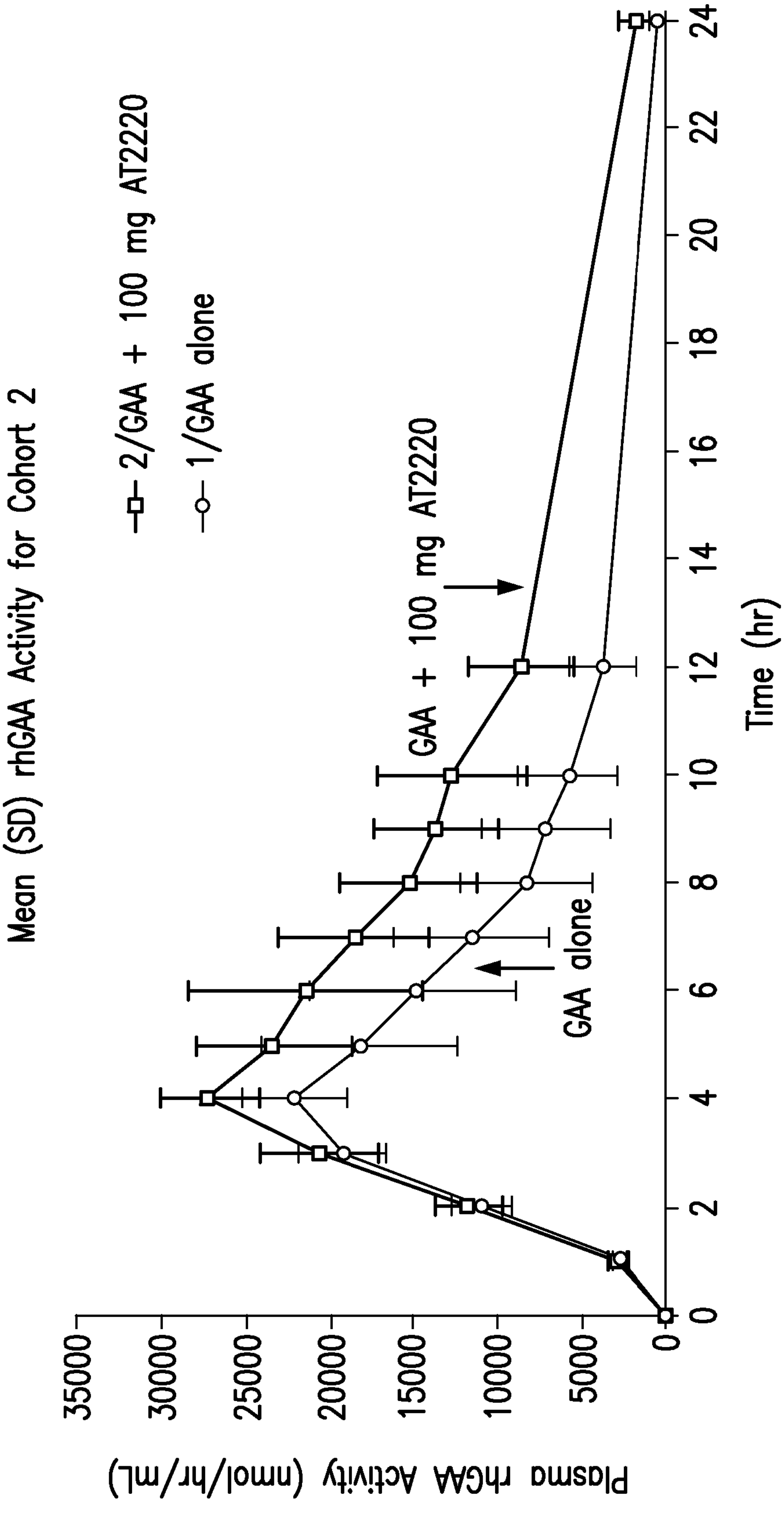
Mean (SD) rhGAA Activity for Cohort 2
2/GAA + 100 mg AT2220
1/GAA alone
GAA + 100 mg AT2220
GAA alone
Plasma rhGAA Activity (nmol/hr/mL)
Time (hr)
0
5000
10000
15000
20000
25000
30000
35000
0
2
4
6
8
10
12
14
16
18
20
22
24

FIG. 4

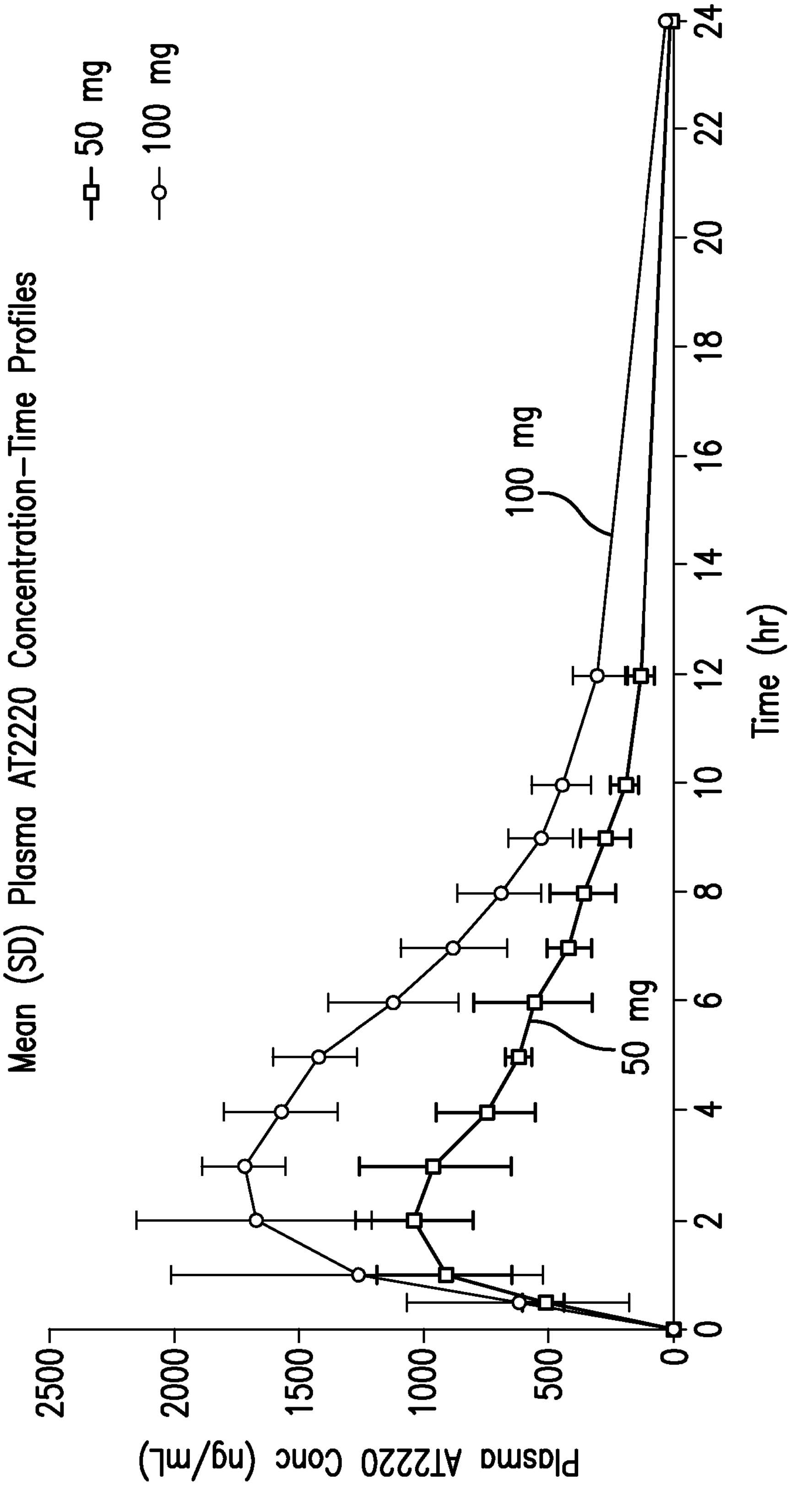
Mean (SD) Plasma AT2220 Concentration-Time Profiles
50 mg
100 mg
Plasma AT2220 Conc (ng/mL)
Time (hr)
0
500
1000
1500
2000
2500
0
2
4
6
8
10
12
14
16
18
20
22
24
100 mg
50 mg

FIG. 5

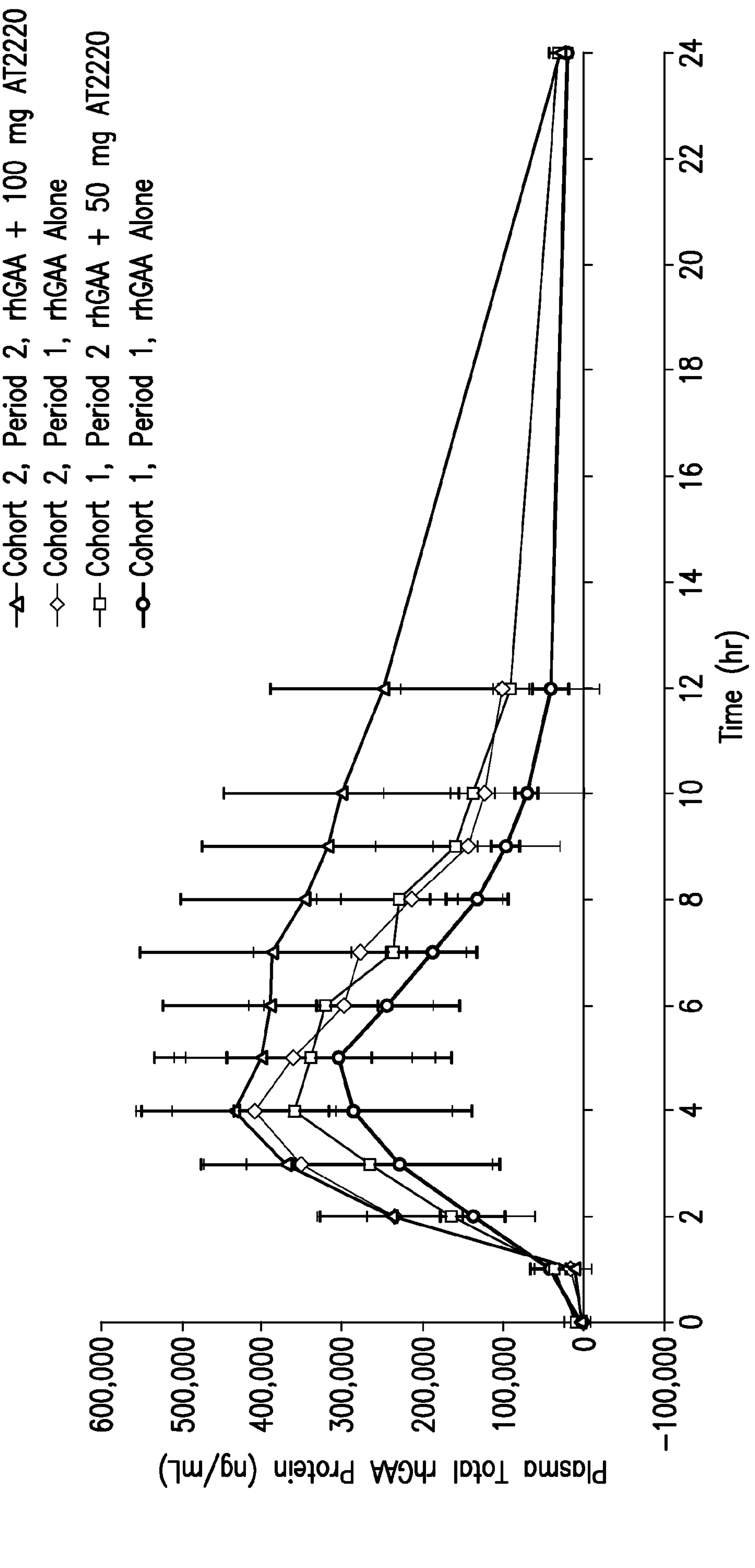
Cohort 2, Period 2, rhGAA + 100 mg AT2220
Cohort 2, Period 1, rhGAA Alone
Cohort 1, Period 2 rhGAA + 50 mg AT2220
Cohort 1, Period 1, rhGAA Alone
Plasma Total rhGAA Protein (ng/mL)
600,000
500,000
400,000
300,000
200,000
100,000
0
−100,000
0
2
4
6
8
10
12
14
16
18
20
22
24
Time (hr)

FIG. 6

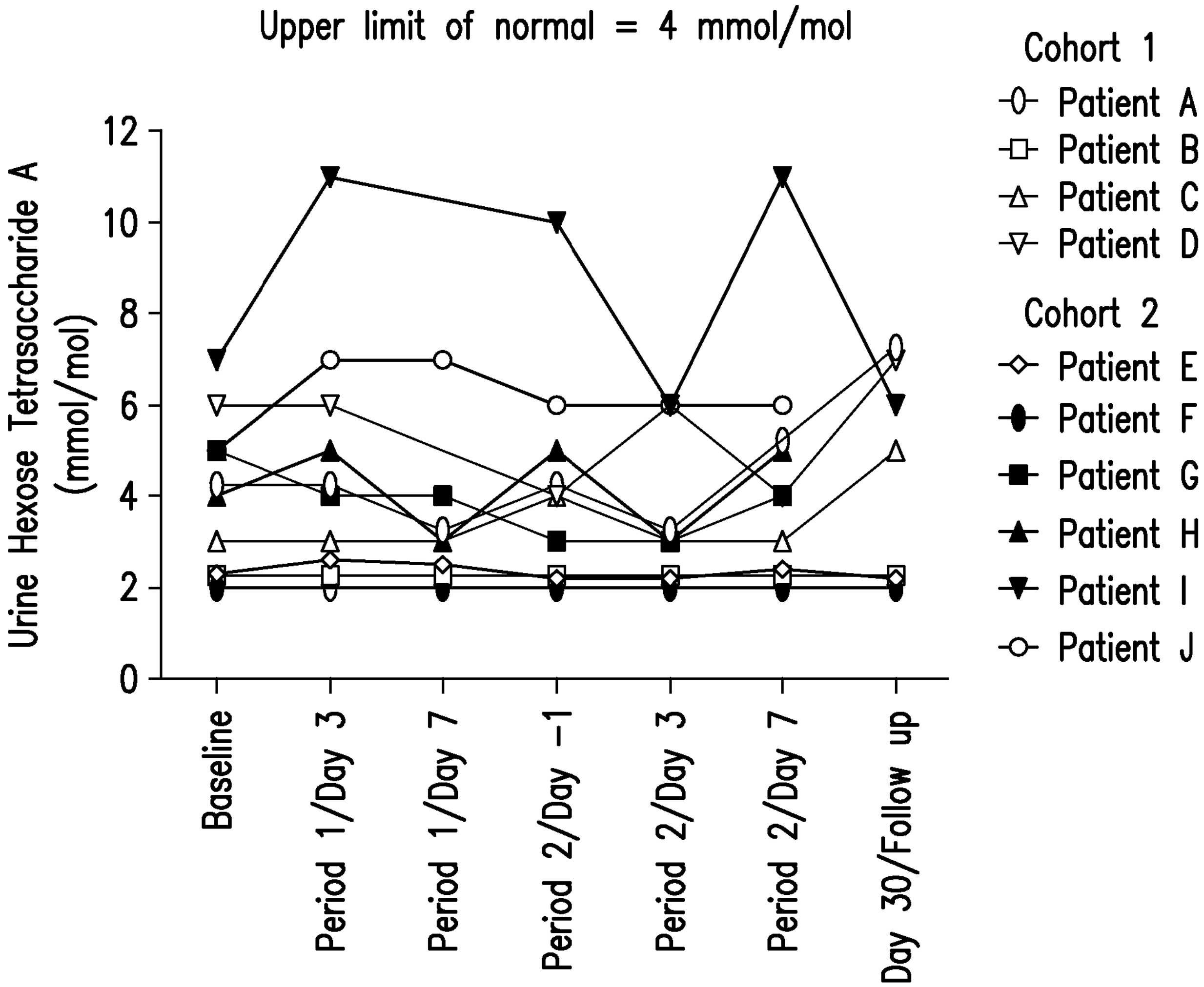
Upper limit of normal = 4 mmol/mol
Urine Hexose Tetrasaccharide A (mmol/mol)
0
2
4
6
8
10
12
Baseline
Period 1/Day 3
Period 1/Day 7
Period 2/Day −1
Period 2/Day 3
Period 2/Day 7
Day 30/Follow up
Cohort 1
Patient A
Patient B
Patient C
Patient D
Cohort 2
Patient E
Patient F
Patient G
Patient H
Patient I
Patient J

FIG. 7

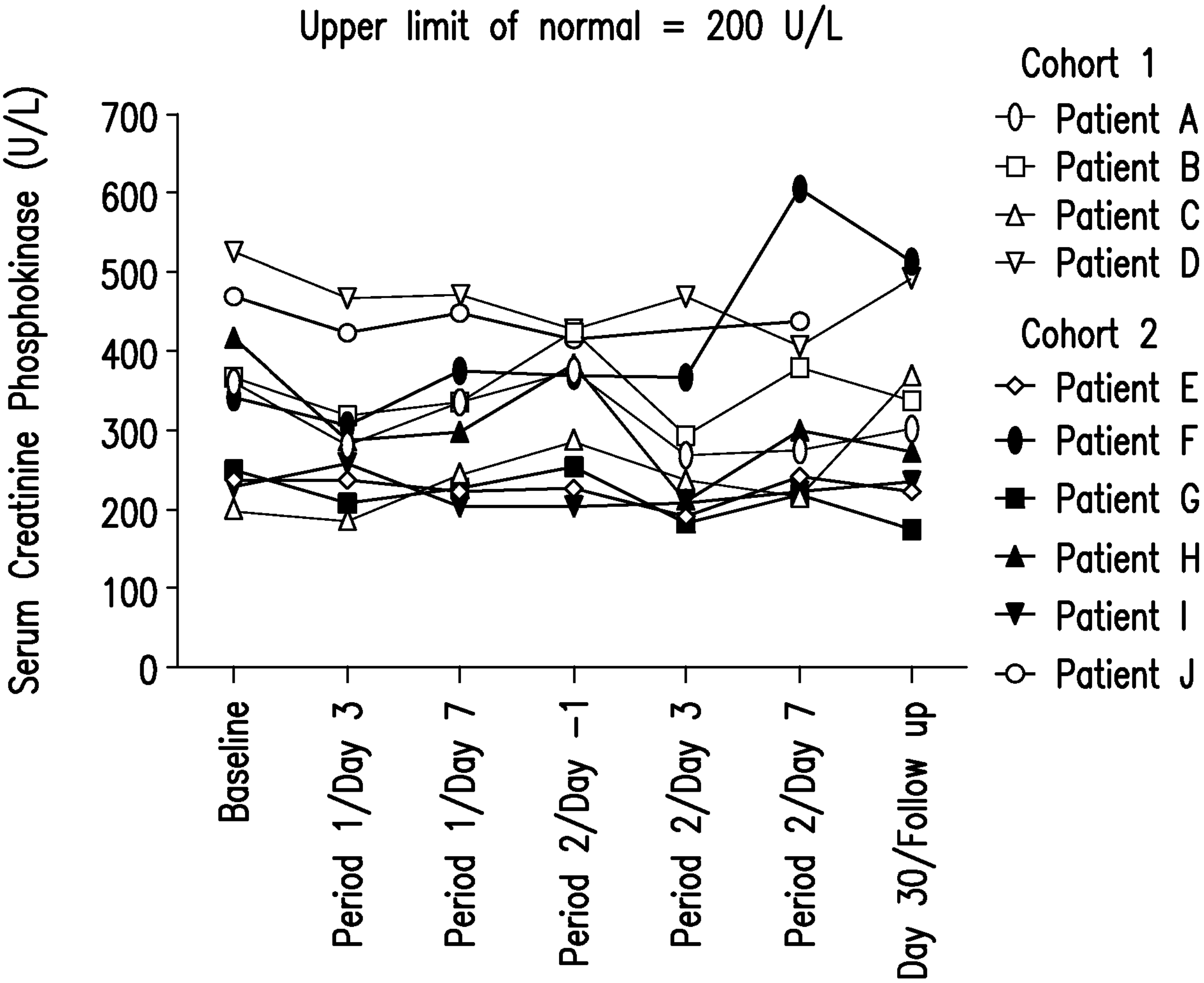

Upper limit of normal = 200 U/L
Serum Creatinine Phosphokinase (U/L)
700
600
500
400
300
200
100
0
Baseline
Period 1/Day 3
Period 1/Day 7
Period 2/Day −1
Period 2/Day 3
Period 2/Day 7
Day 30/Follow up
Cohort 1
Patient A
Patient B
Patient C
Patient D
Cohort 2
Patient E
Patient F
Patient G
Patient H
Patient I
Patient J


FIG. 8

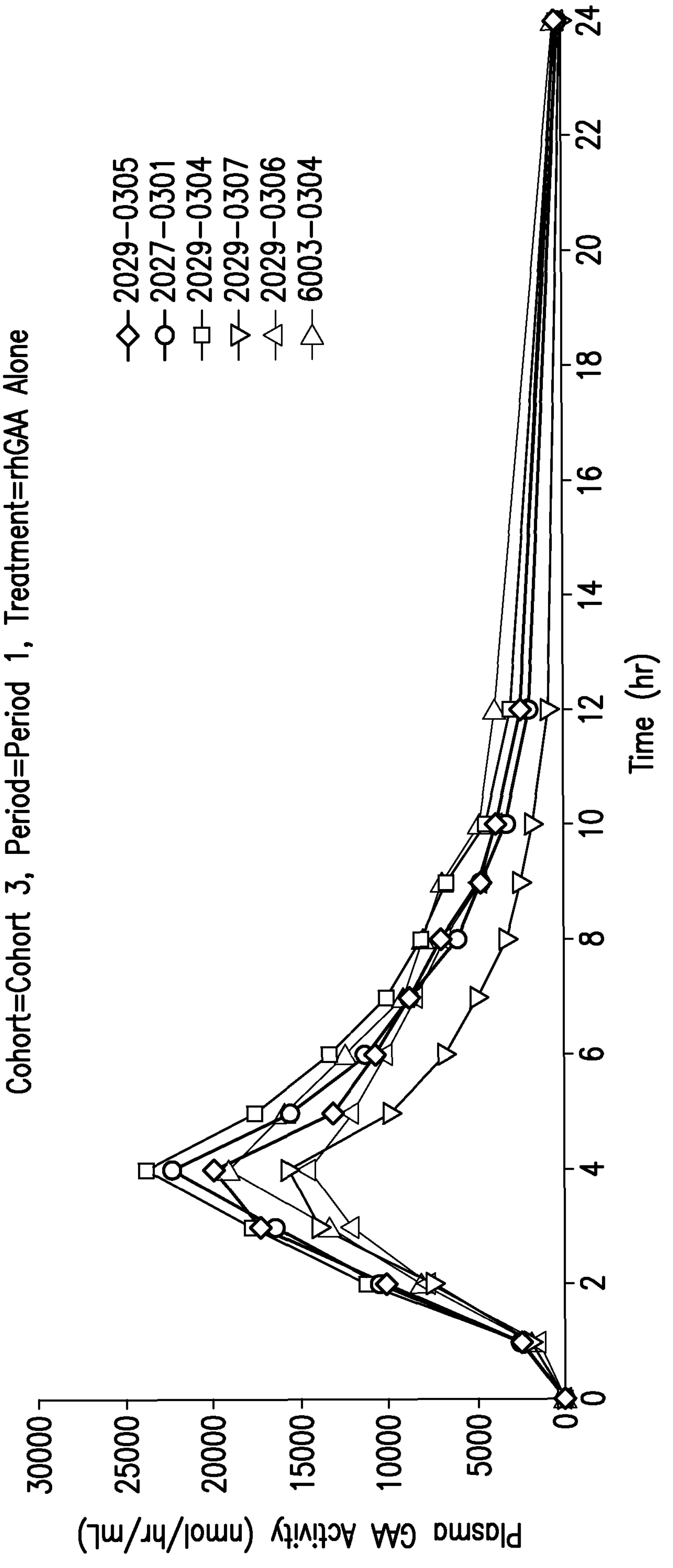
Cohort=Cohort 3, Period=Period 1, Treatment=rhGAA Alone
2029-0305
2027-0301
2029-0304
2029-0307
2029-0306
6003-0304
Plasma GAA Activity (nmol/hr/mL)
30000
25000
20000
15000
10000
5000
0
0
2
4
6
8
10
12
14
16
18
20
22
24
Time (hr)

FIG. 9A

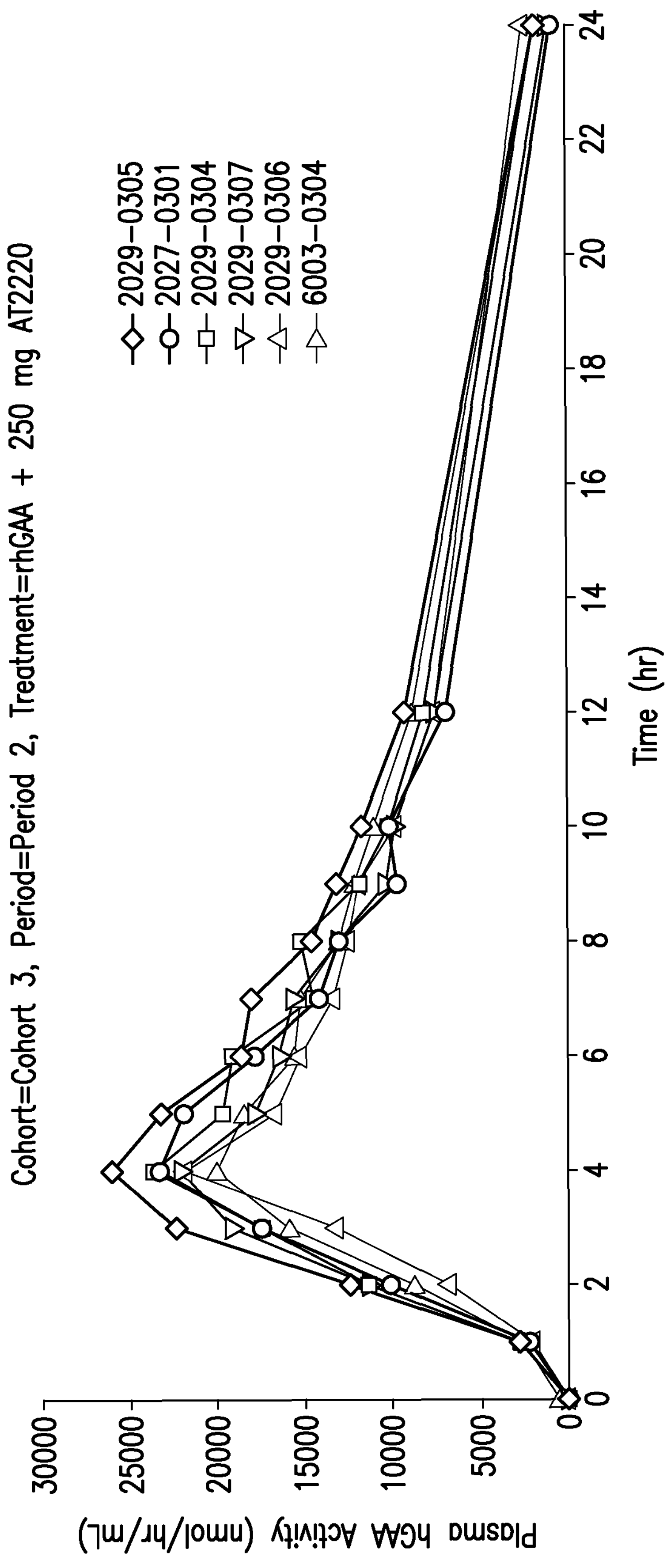
Cohort=Cohort 3, Period=Period 2, Treatment=rhGAA + 250 mg AT2220
2029–0305
2027–0301
2029–0304
2029–0307
2029–0306
6003–0304
Plasma hGAA Activity (nmol/hr/mL)
0
5000
10000
15000
20000
25000
30000
Time (hr)
0
2
4
6
8
10
12
14
16
18
20
22
24

FIG. 9B

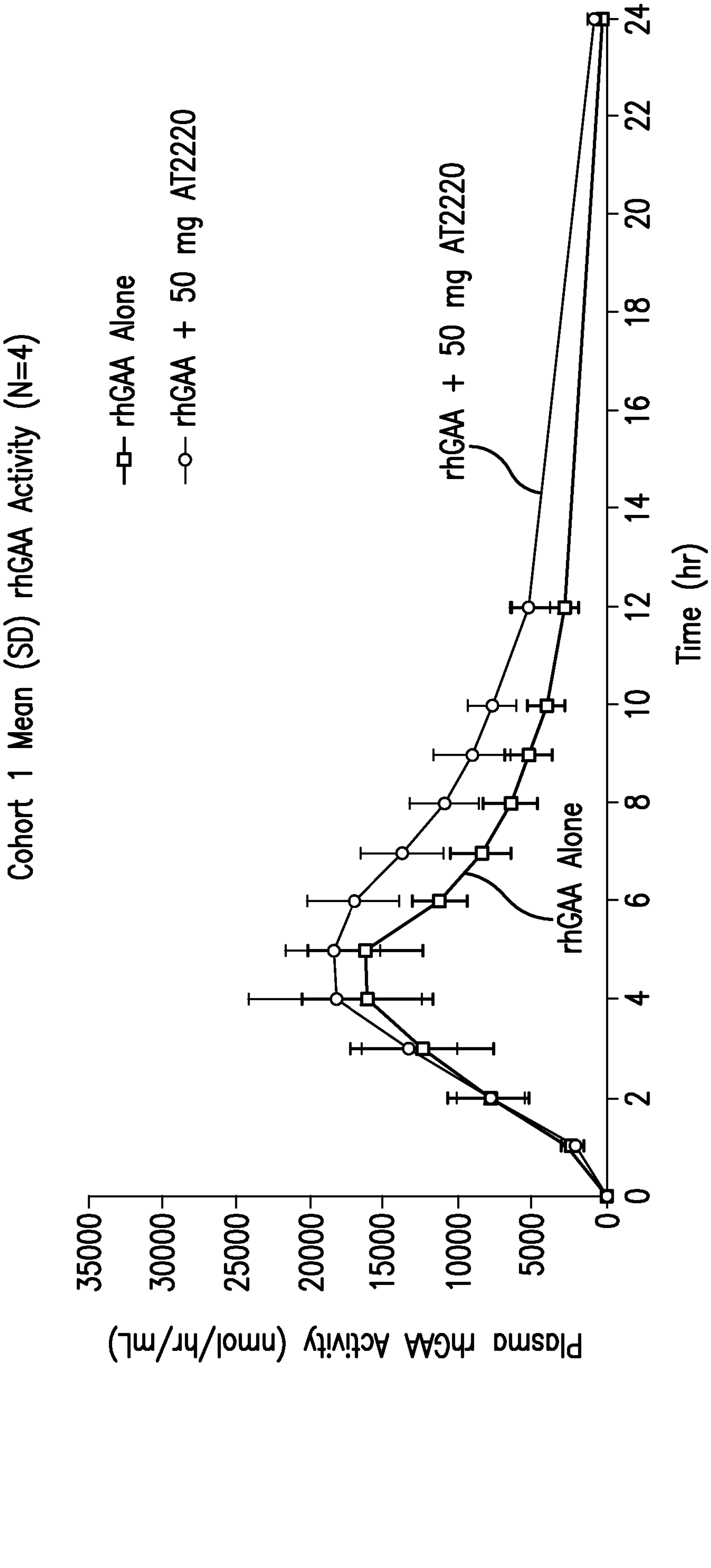

Cohort 1 Mean (SD) rhGAA Activity (N=4)
rhGAA Alone
rhGAA + 50 mg AT2220
rhGAA + 50 mg AT2220
rhGAA Alone
Plasma rhGAA Activity (nmol/hr/mL)
35000
30000
25000
20000
15000
10000
5000
0
0
2
4
6
8
10
12
14
16
18
20
22
24
Time (hr)


FIG. 10A

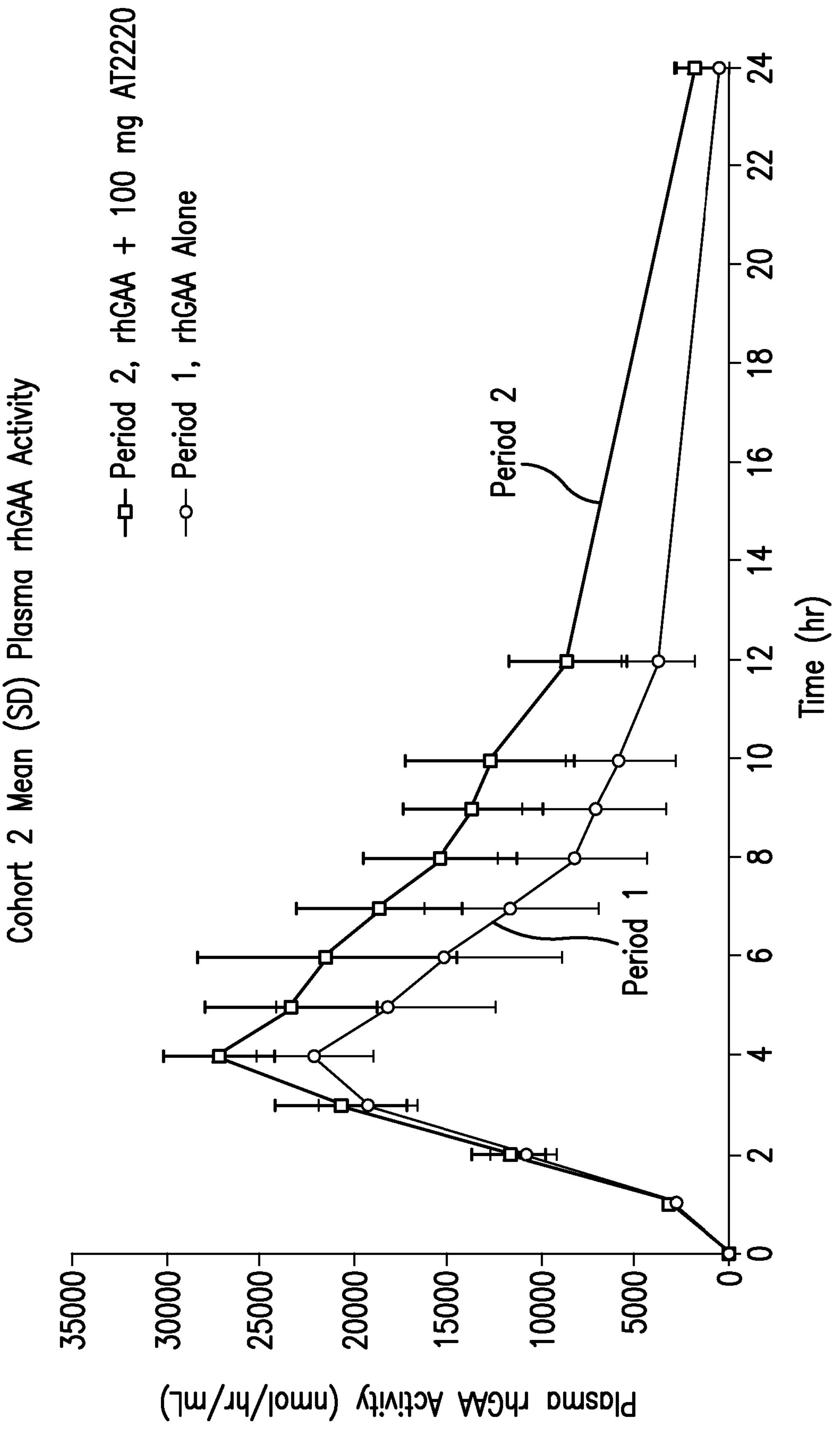
Cohort 2 Mean (SD) Plasma rhGAA Activity
Period 2, rhGAA + 100 mg AT2220
Period 1, rhGAA Alone
Period 2
Period 1
Plasma rhGAA Activity (nmol/hr/mL)
35000
30000
25000
20000
15000
10000
5000
0
0
2
4
6
8
10
12
14
16
18
20
22
24
Time (hr)

FIG. 10B

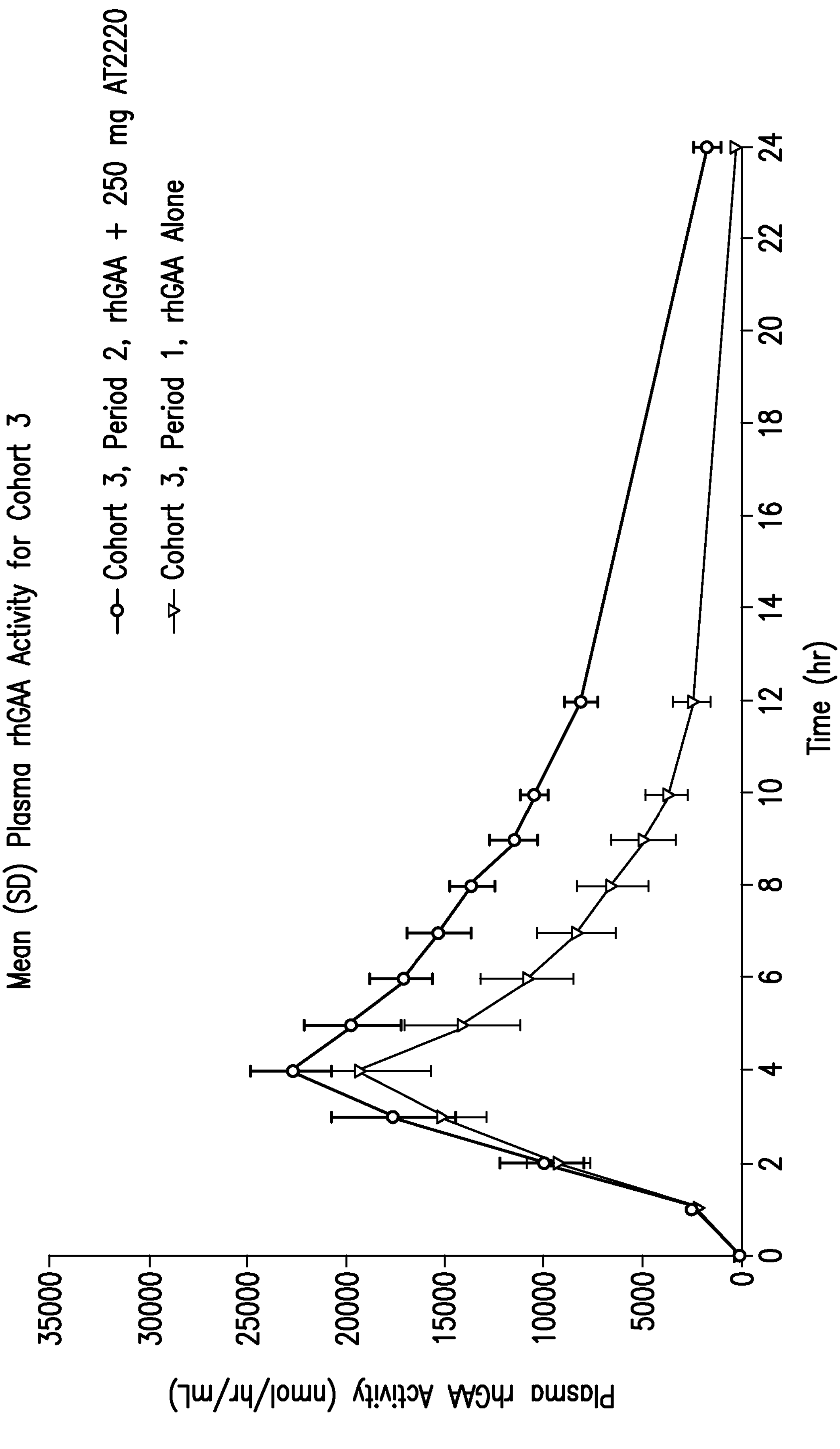
Mean (SD) Plasma rhGAA Activity for Cohort 3
Cohort 3, Period 2, rhGAA + 250 mg AT2220
Cohort 3, Period 1, rhGAA Alone
Plasma rhGAA Activity (nmol/hr/mL)
35000
30000
25000
20000
15000
10000
5000
0
0
2
4
6
8
10
12
14
16
18
20
22
24
Time (hr)

FIG. 10C

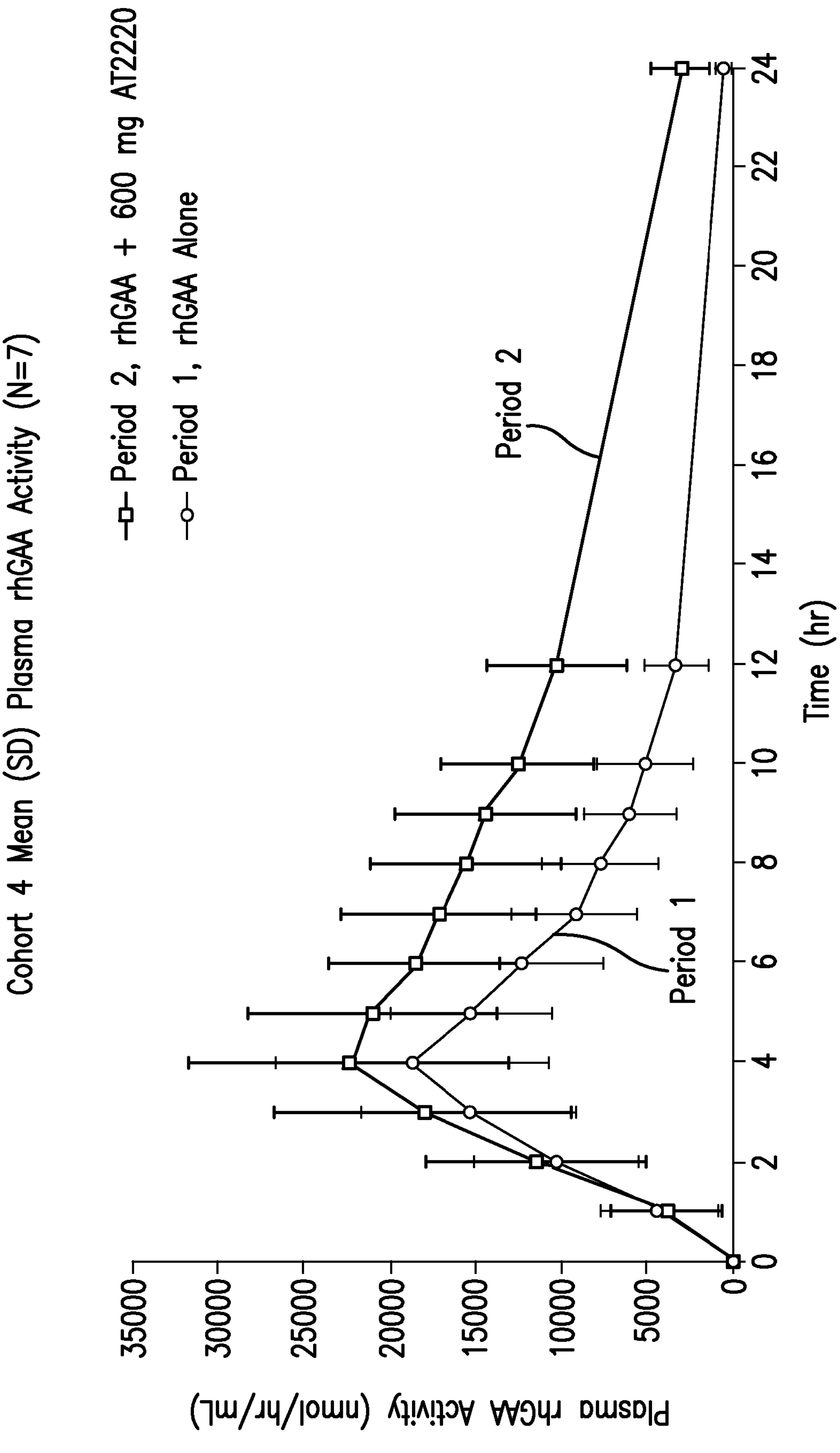
Cohort 4 Mean (SD) Plasma rhGAA Activity (N=7)
Period 2, rhGAA + 600 mg AT2220
Period 1, rhGAA Alone
Period 2
Period 1
Plasma rhGAA Activity (nmol/hr/mL)
0
5000
10000
15000
20000
25000
30000
35000
Time (hr)
0
2
4
6
8
10
12
14
16
18
20
22
24

FIG. 10D

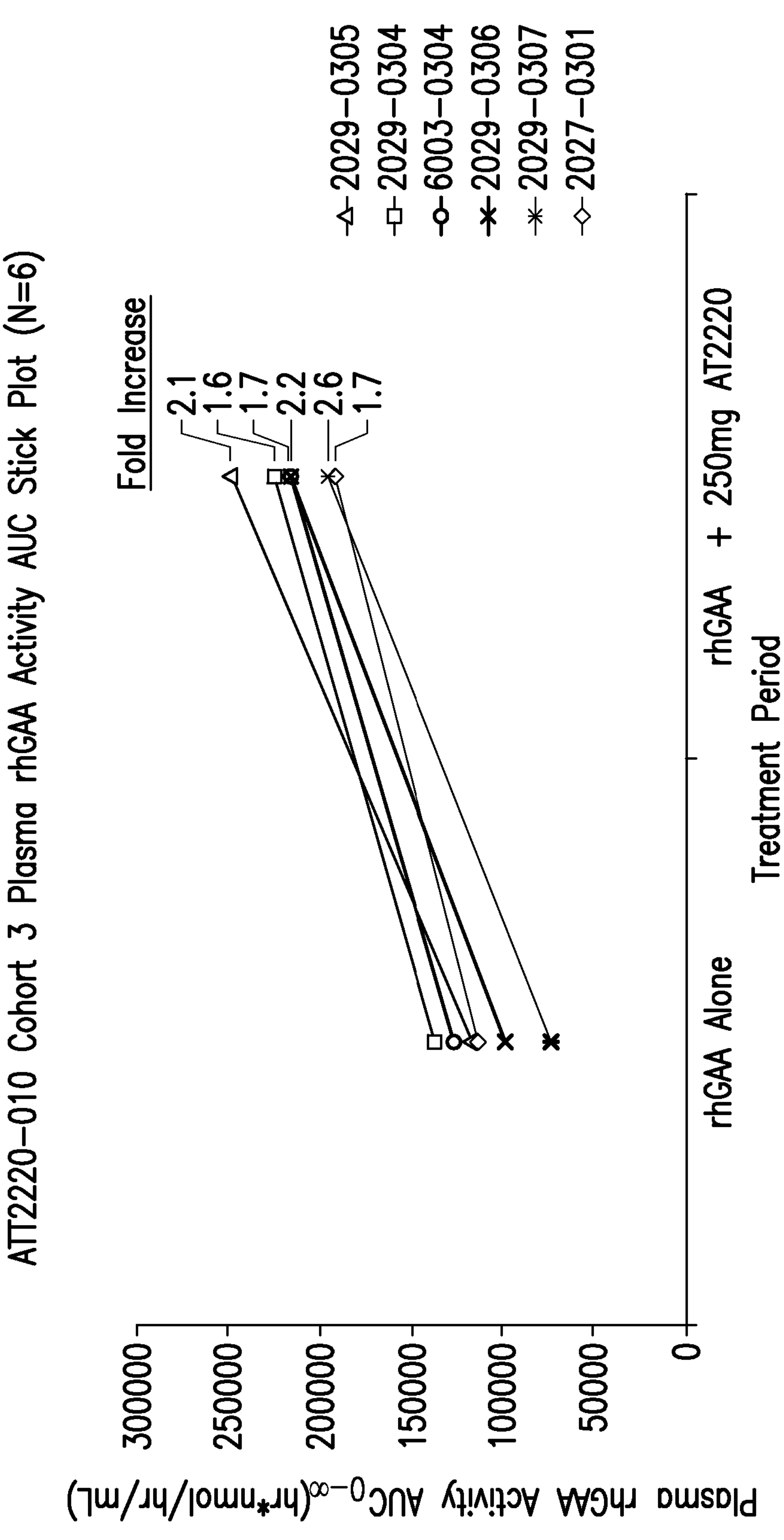
ATT2220-010 Cohort 3 Plasma rhGAA Activity AUC Stick Plot (N=6)
Plasma rhGAA Activity AUC0-∞(hr*nmol/hr/mL)
300000
250000
200000
150000
100000
50000
0
Fold Increase
2.1
1.6
1.7
2.2
2.6
1.7
rhGAA Alone
rhGAA + 250mg AT2220
Treatment Period
2029-0305
2029-0304
6003-0304
2029-0306
2029-0307
2027-0301

FIG. 11

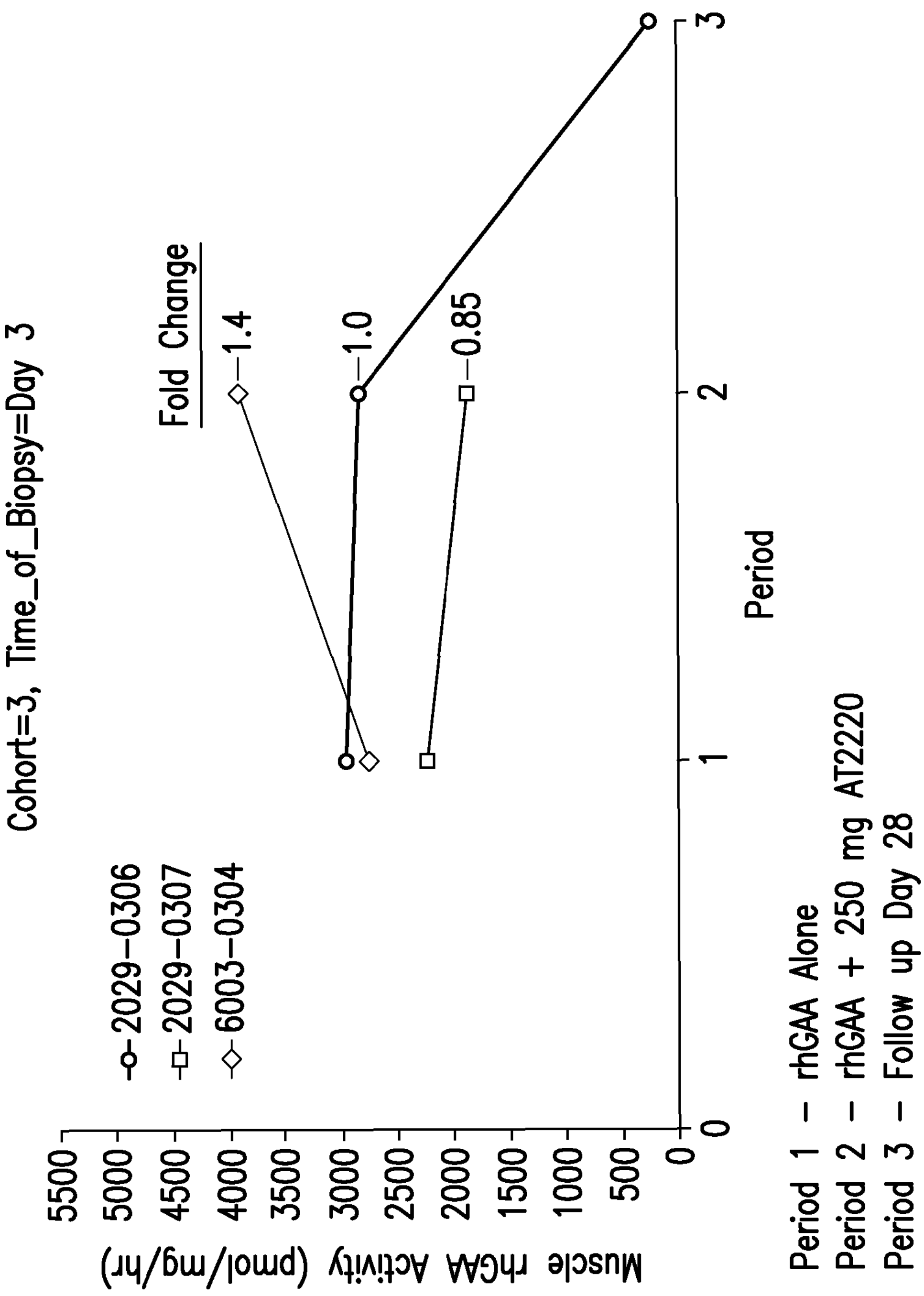

Cohort=3, Time_of_Biopsy=Day 3
2029–0306
2029–0307
6003–0304
Fold Change
1.4
1.0
0.85
Muscle rhGAA Activity (pmol/mg/hr)
5500
5000
4500
4000
3500
3000
2500
2000
1500
1000
500
0
0
1
2
3
Period
Period 1 – rhGAA Alone
Period 2 – rhGAA + 250 mg AT2220
Period 3 – Follow up Day 28


FIG. 12A

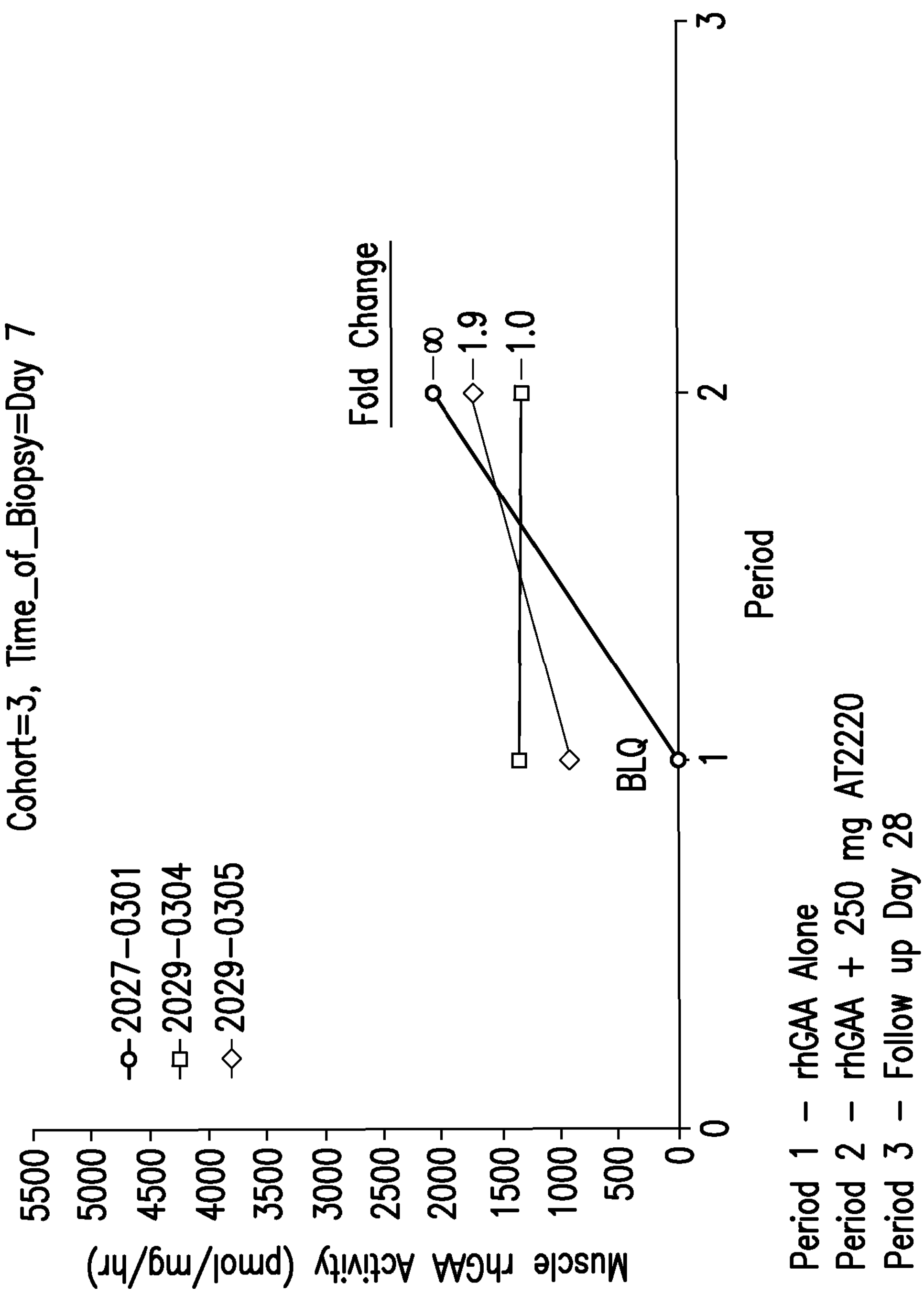
Cohort=3, Time_of_Biopsy=Day 7
2027-0301
2029-0304
2029-0305
Muscle rhGAA Activity (pmol/mg/hr)
5500
5000
4500
4000
3500
3000
2500
2000
1500
1000
500
0
0
1
2
3
Period
BLQ
Fold Change
∞
1.9
1.0
Period 1 – rhGAA Alone
Period 2 – rhGAA + 250 mg AT2220
Period 3 – Follow up Day 28

FIG. 12B

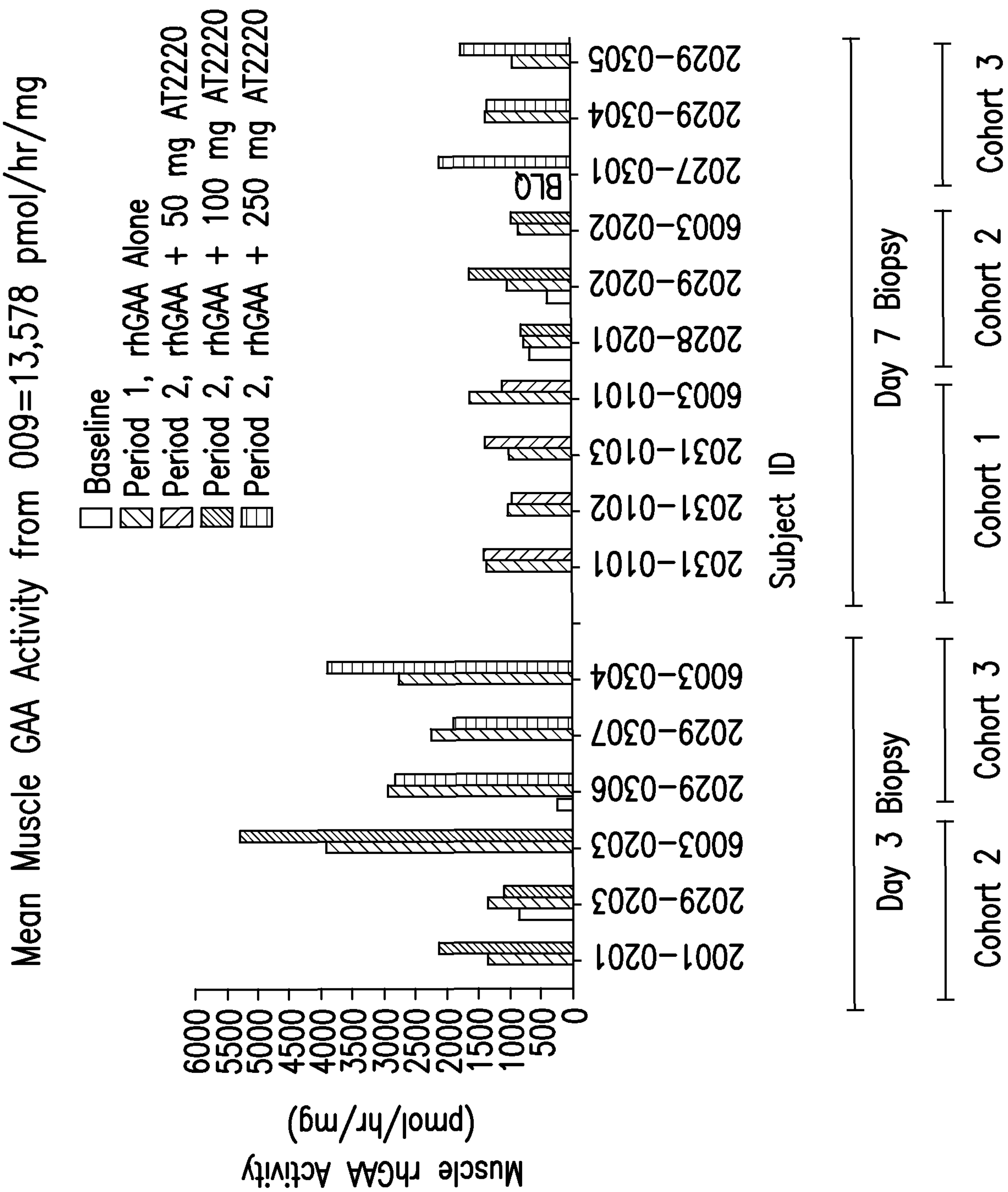
Mean Muscle GAA Activity from 009=13,578 pmol/hr/mg
Baseline
Period 1, rhGAA Alone
Period 2, rhGAA + 50 mg AT2220
Period 2, rhGAA + 100 mg AT2220
Period 2, rhGAA + 250 mg AT2220
Muscle rhGAA Activity (pmol/hr/mg)
6000
5500
5000
4500
4000
3500
3000
2500
2000
1500
1000
500
0
2001-0201
2029-0203
6003-0203
2029-0306
2029-0307
6003-0304
2031-0101
2031-0102
2031-0103
6003-0101
2028-0201
2029-0202
6003-0202
BLQ
2027-0301
2029-0304
2029-0305
Subject ID
Day 3 Biopsy
Day 7 Biopsy
Cohort 2
Cohort 3
Cohort 1
Cohort 2
Cohort 3

FIG. 13

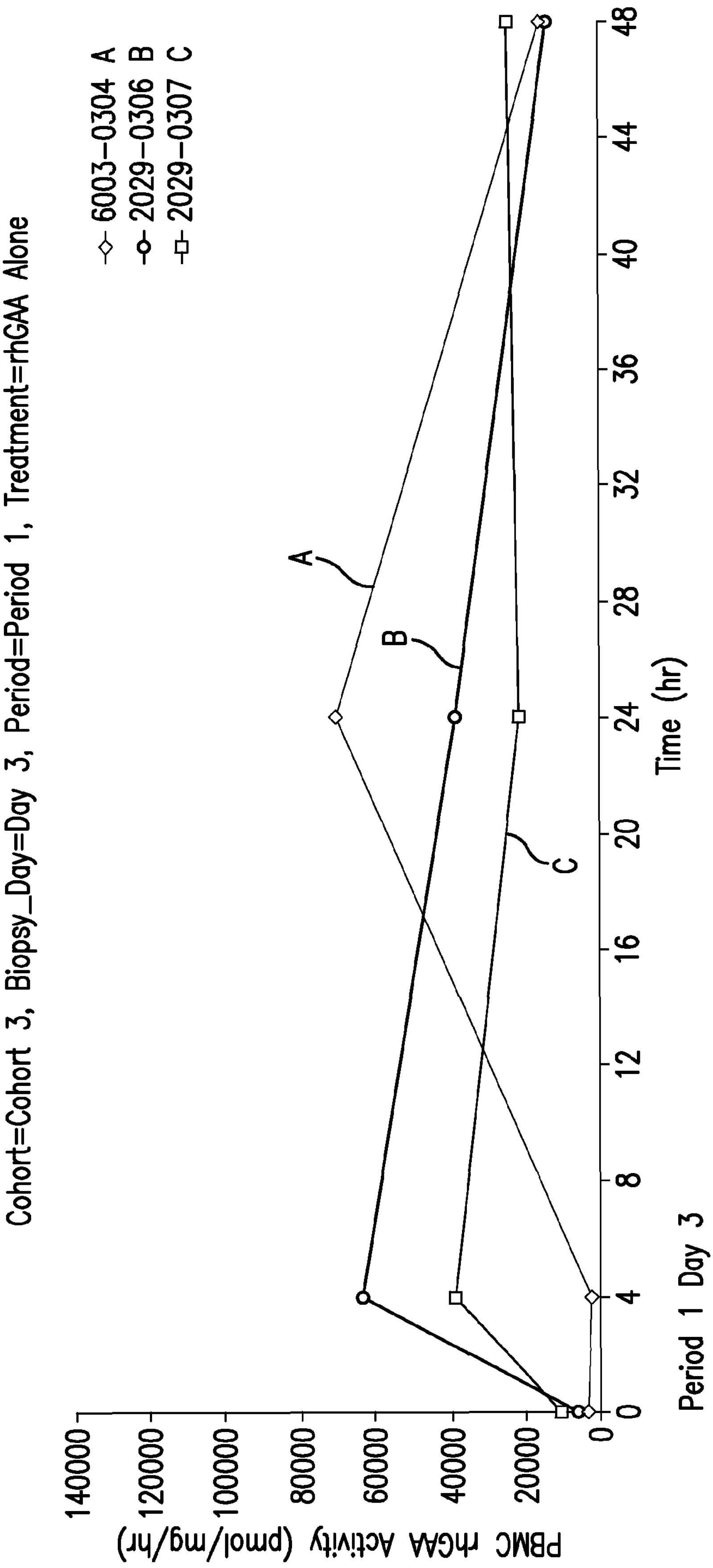
Cohort=Cohort 3, Biopsy_Day=Day 3, Period=Period 1, Treatment=rhGAA Alone
6003-0304 A
2029-0306 B
2029-0307 C
A
B
C
PBMC rhGAA Activity (pmol/mg/hr)
140000
120000
100000
80000
60000
40000
20000
0
0
4
8
12
16
20
24
28
32
36
40
44
48
Time (hr)
Period 1 Day 3

FIG. 14A

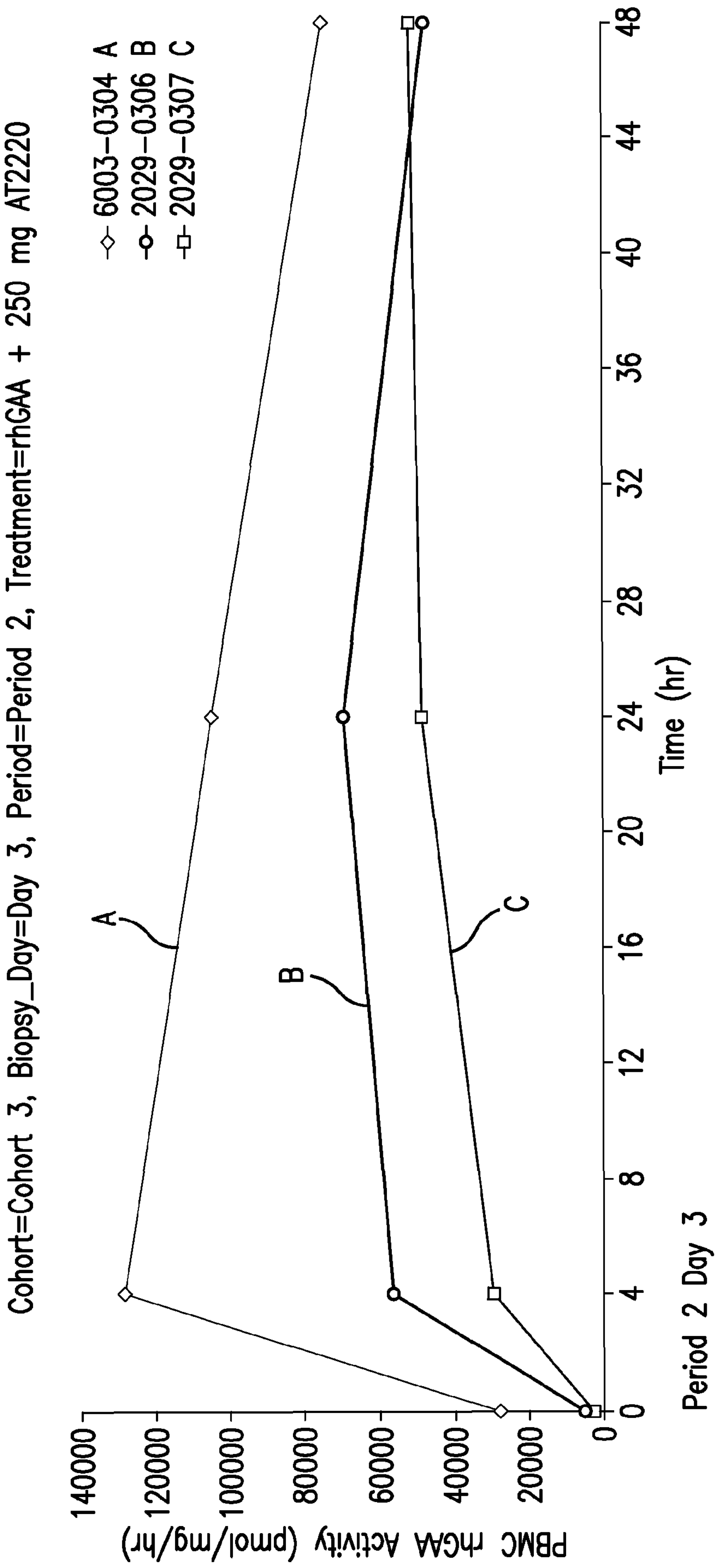

Cohort=Cohort 3, Biopsy_Day=Day 3, Period=Period 2, Treatment=rhGAA + 250 mg AT2220
6003-0304 A
2029-0306 B
2029-0307 C
A
B
C
PBMC rhGAA Activity (pmol/mg/hr)
140000
120000
100000
80000
60000
40000
20000
0
0
4
8
12
16
20
24
28
32
36
40
44
48
Time (hr)
Period 2 Day 3


FIG. 14B

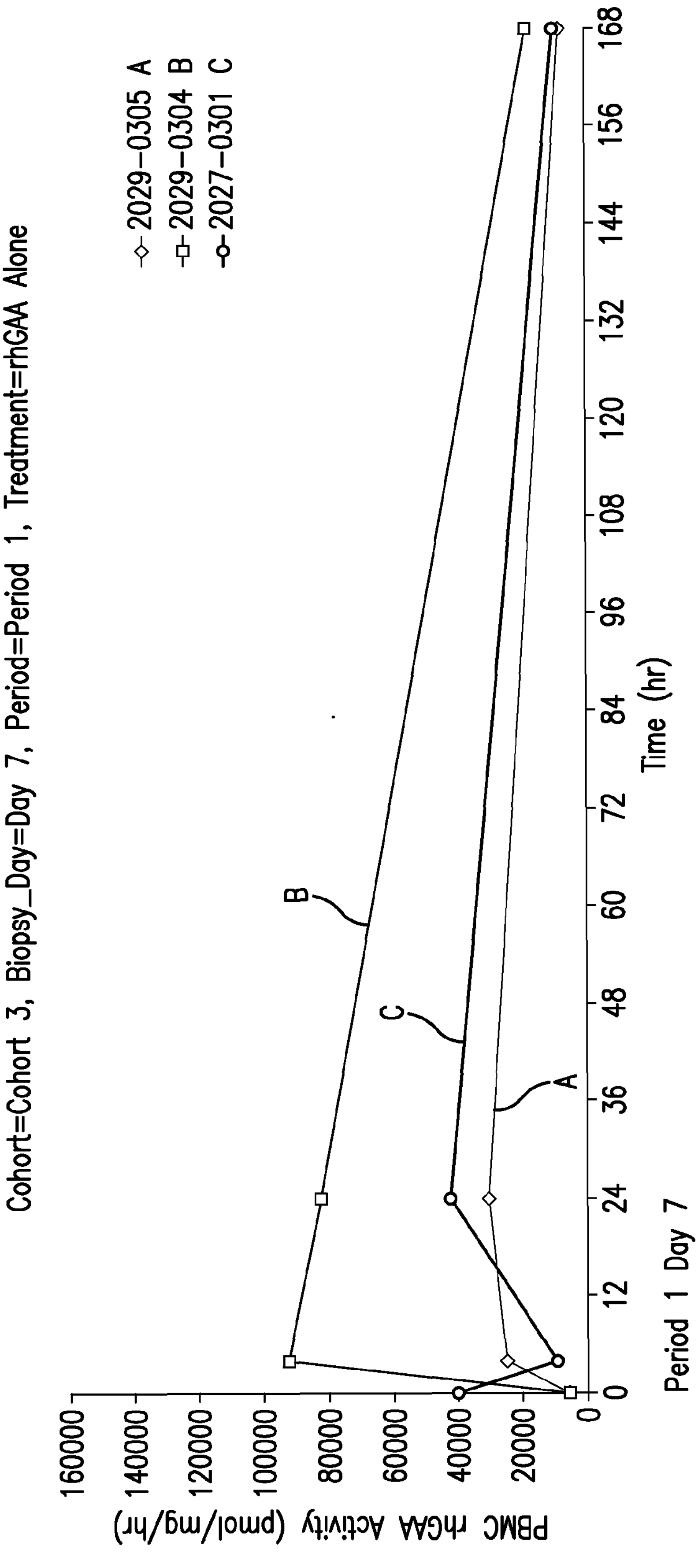

Cohort=Cohort 3, Biopsy_Day=Day 7, Period=Period 1, Treatment=rhGAA Alone
2029−0305 A
2029−0304 B
2027−0301 C
A
B
C
PBMC rhGAA Activity (pmol/mg/hr)
160000
140000
120000
100000
80000
60000
40000
20000
0
0
12
24
36
48
60
72
84
96
108
120
132
144
156
168
Time (hr)
Period 1 Day 7


FIG. 15A

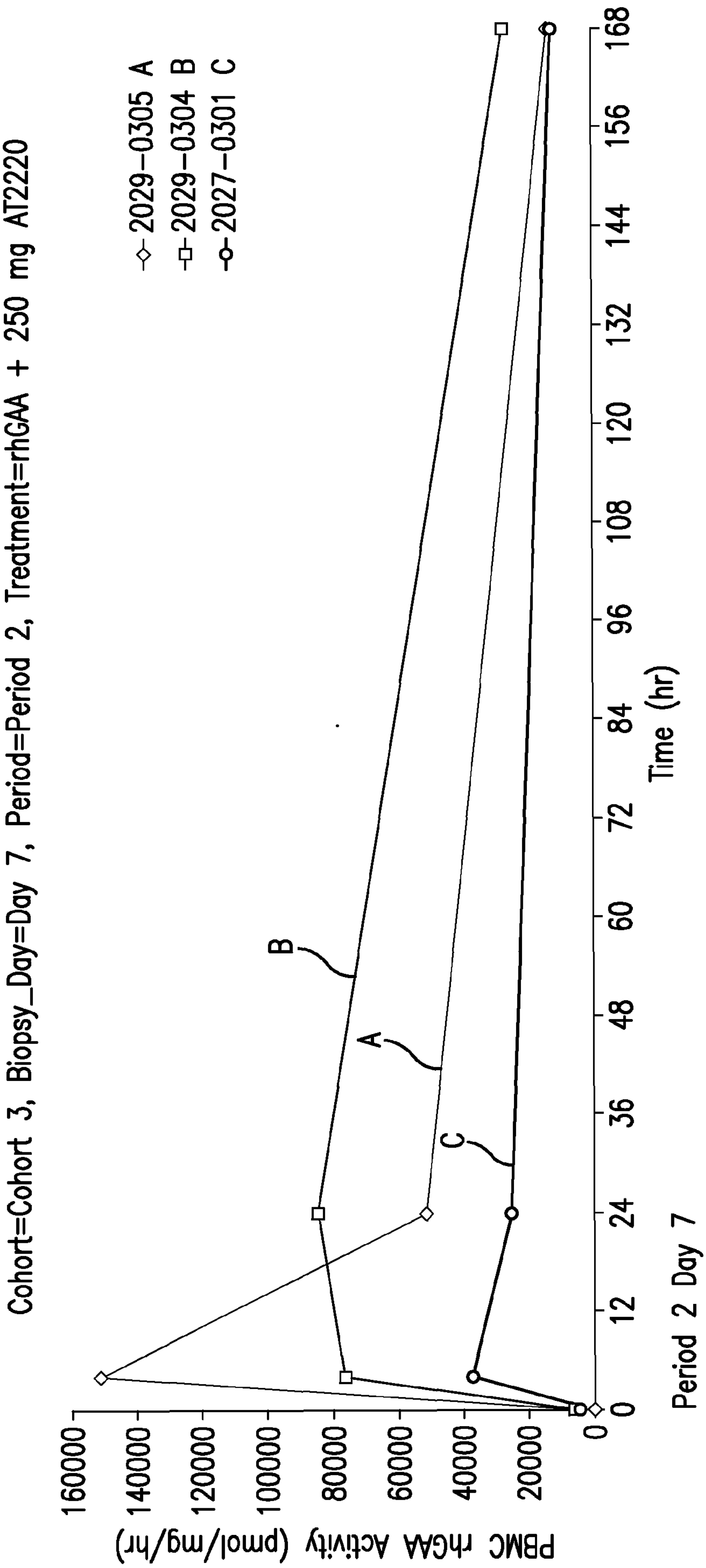
Cohort=Cohort 3, Biopsy_Day=Day 7, Period=Period 2, Treatment=rhGAA + 250 mg AT2220
2029−0305 A
2029−0304 B
2027−0301 C
A
B
C
PBMC rhGAA Activity (pmol/mg/hr)
160000
140000
120000
100000
80000
60000
40000
20000
0
0
12
24
36
48
60
72
84
96
108
120
132
144
156
168
Time (hr)
Period 2 Day 7

FIG. 15B

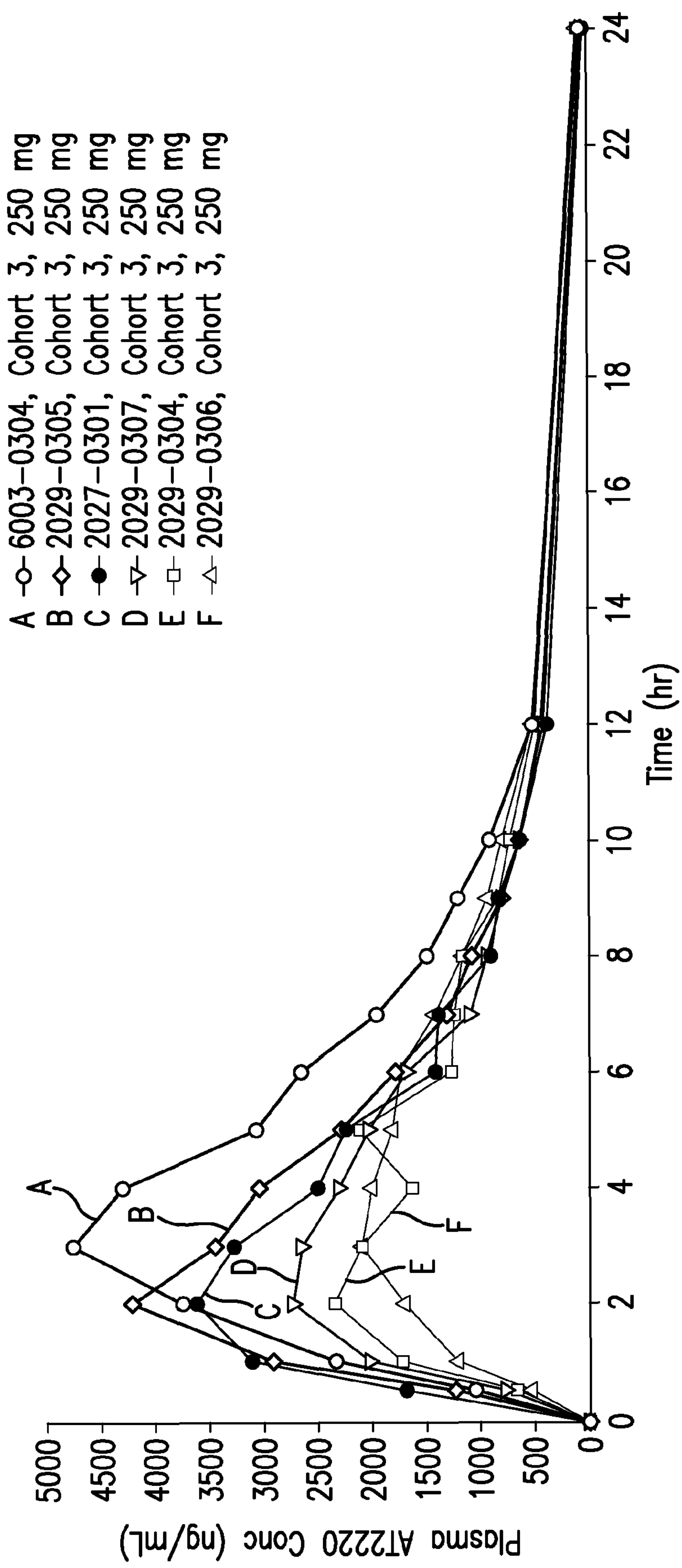
A —○— 6003–0304, Cohort 3, 250 mg
B —◇— 2029–0305, Cohort 3, 250 mg
C —●— 2027–0301, Cohort 3, 250 mg
D —▽— 2029–0307, Cohort 3, 250 mg
E —□— 2029–0304, Cohort 3, 250 mg
F —△— 2029–0306, Cohort 3, 250 mg
Plasma AT2220 Conc (ng/mL)
5000
4500
4000
3500
3000
2500
2000
1500
1000
500
0
Time (hr)
0
2
4
6
8
10
12
14
16
18
20
22
24
A
B
C
D
E
F

FIG. 16A

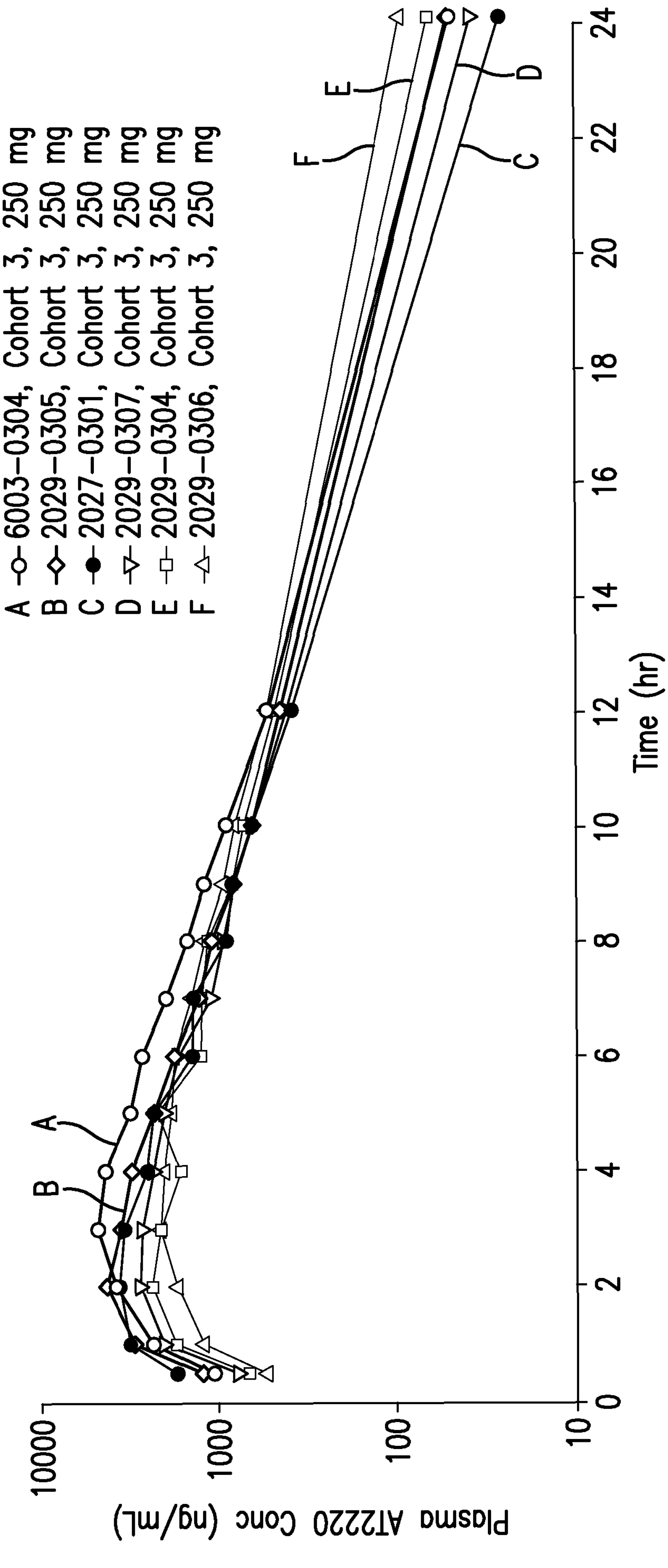
A —○— 6003–0304, Cohort 3, 250 mg
B —◇— 2029–0305, Cohort 3, 250 mg
C —●— 2027–0301, Cohort 3, 250 mg
D —▽— 2029–0307, Cohort 3, 250 mg
E —□— 2029–0304, Cohort 3, 250 mg
F —△— 2029–0306, Cohort 3, 250 mg
Plasma AT2220 Conc (ng/mL)
10000
1000
100
10
Time (hr)
0
2
4
6
8
10
12
14
16
18
20
22
24
A
B
C
D
E
F

FIG. 16B

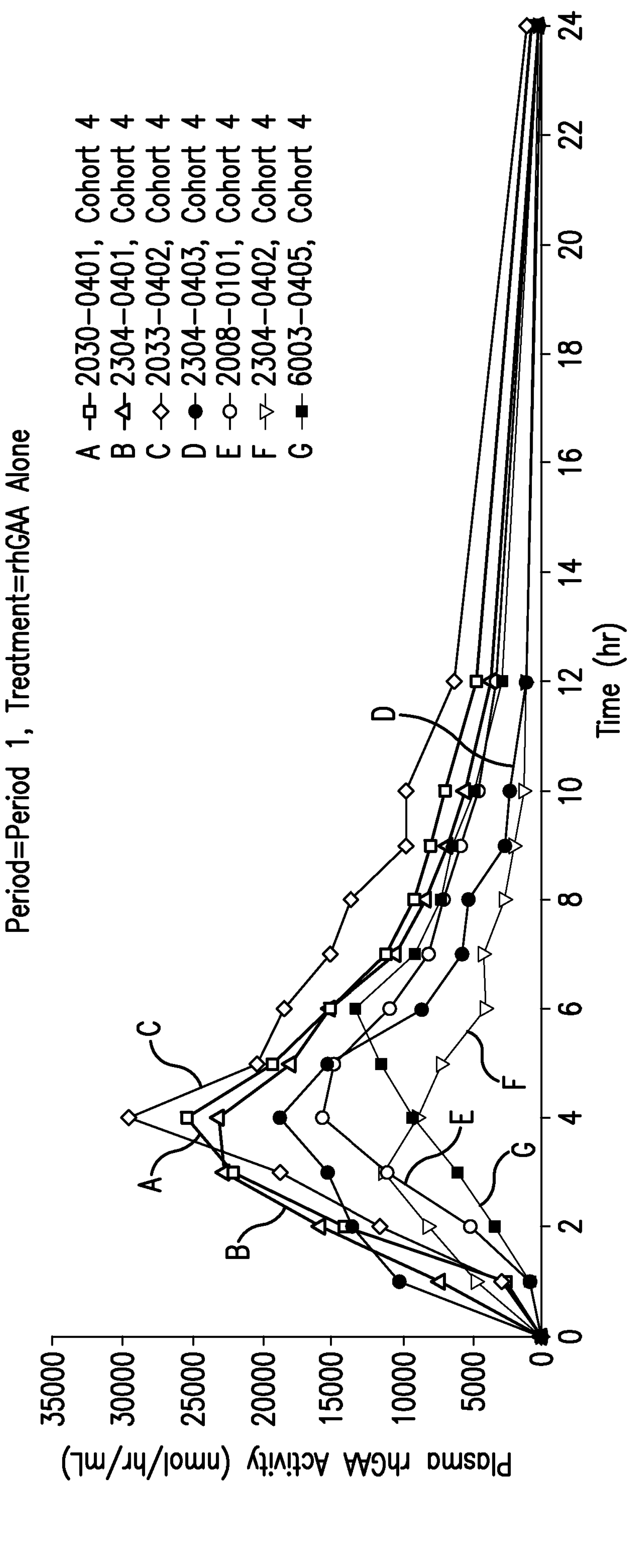
Period=Period 1, Treatment=rhGAA Alone
A —□— 2030−0401, Cohort 4
B —△— 2304−0401, Cohort 4
C —◇— 2033−0402, Cohort 4
D —●— 2304−0403, Cohort 4
E —○— 2008−0101, Cohort 4
F —▽— 2304−0402, Cohort 4
G —■— 6003−0405, Cohort 4
Plasma rhGAA Activity (nmol/hr/mL)
35000
30000
25000
20000
15000
10000
5000
0
Time (hr)
0
2
4
6
8
10
12
14
16
18
20
22
24
A
B
C
D
E
F
G

FIG. 17A

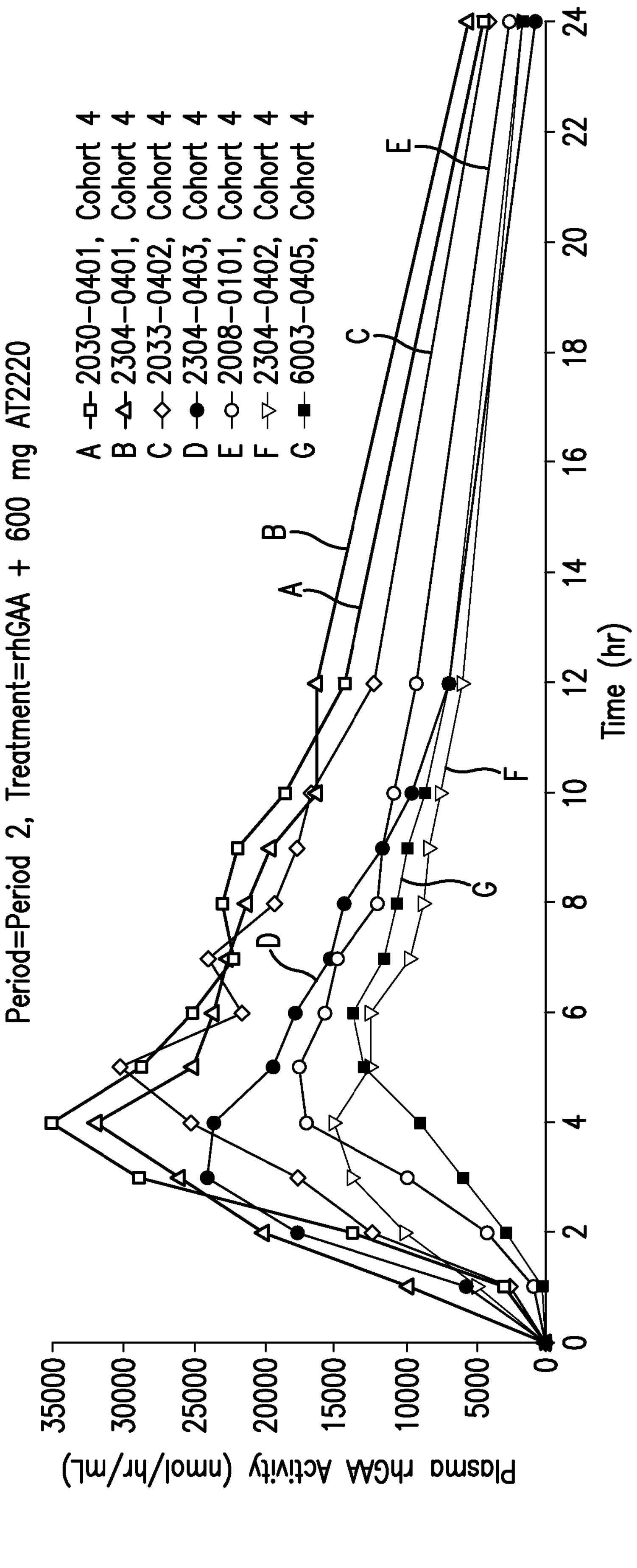
Period=Period 2, Treatment=rhGAA + 600 mg AT2220
A 2030−0401, Cohort 4
B 2304−0401, Cohort 4
C 2033−0402, Cohort 4
D 2304−0403, Cohort 4
E 2008−0101, Cohort 4
F 2304−0402, Cohort 4
G 6003−0405, Cohort 4
Plasma rhGAA Activity (nmol/hr/mL)
35000
30000
25000
20000
15000
10000
5000
0
0
2
4
6
8
10
12
14
16
18
20
22
24
Time (hr)
A
B
C
D
E
F
G

FIG. 17B

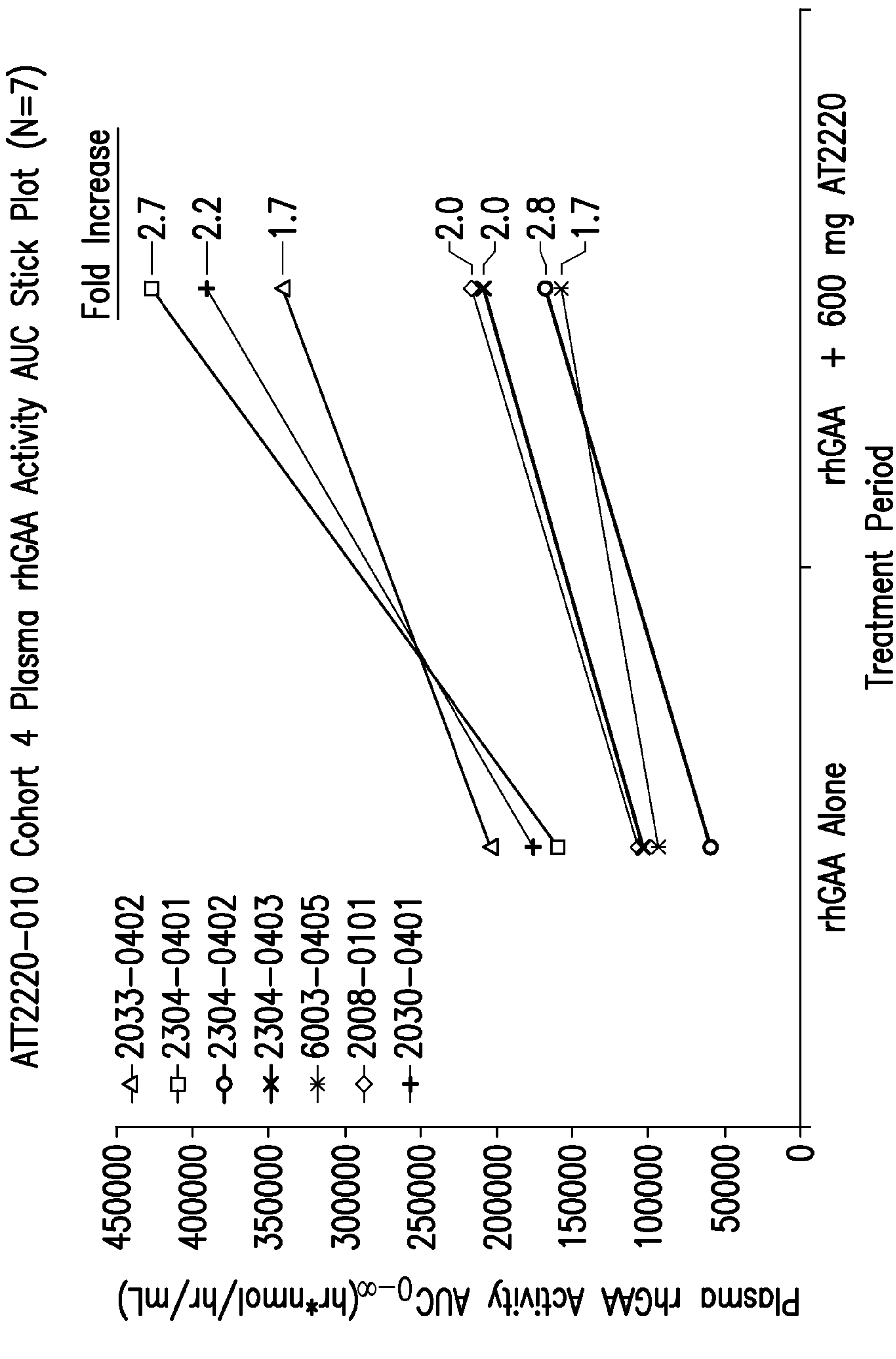
ATT2220-010 Cohort 4 Plasma rhGAA Activity AUC Stick Plot (N=7)
2033-0402
2304-0401
2304-0402
2304-0403
6003-0405
2008-0101
2030-0401
Fold Increase
2.7
2.2
1.7
2.0
2.0
2.8
1.7
Plasma rhGAA Activity $AUC_{0-\infty}$(hr*nmol/hr/mL)
450000
400000
350000
300000
250000
200000
150000
100000
50000
0
rhGAA Alone
rhGAA + 600 mg AT2220
Treatment Period

FIG. 18

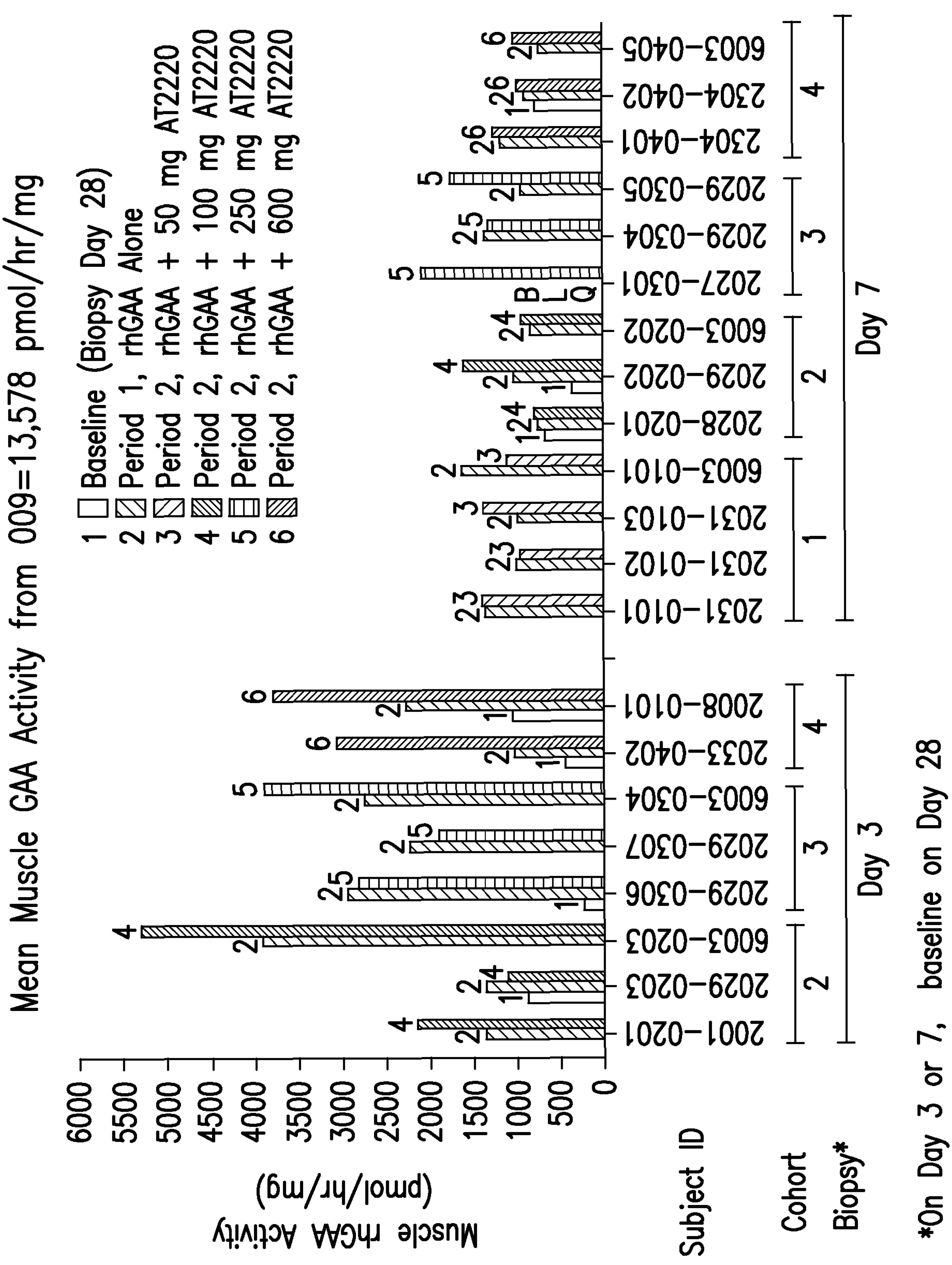
Mean Muscle GAA Activity from 009=13,578 pmol/hr/mg
1 Baseline (Biopsy Day 28)
2 Period 1, rhGAA Alone
3 Period 2, rhGAA + 50 mg AT2220
4 Period 2, rhGAA + 100 mg AT2220
5 Period 2, rhGAA + 250 mg AT2220
6 Period 2, rhGAA + 600 mg AT2220
Muscle rhGAA Activity (pmol/hr/mg)
6000
5500
5000
4500
4000
3500
3000
2500
2000
1500
1000
500
0
Subject ID
2001-0201
2029-0203
6003-0203
2029-0306
2029-0307
6003-0304
2033-0402
2008-0101
2031-0101
2031-0102
2031-0103
6003-0101
2028-0201
2029-0202
6003-0202
2027-0301
2029-0304
2029-0305
2304-0401
2304-0402
6003-0405
BLQ
Cohort
Biopsy*
Day 3
Day 7
*On Day 3 or 7, baseline on Day 28

FIG. 19

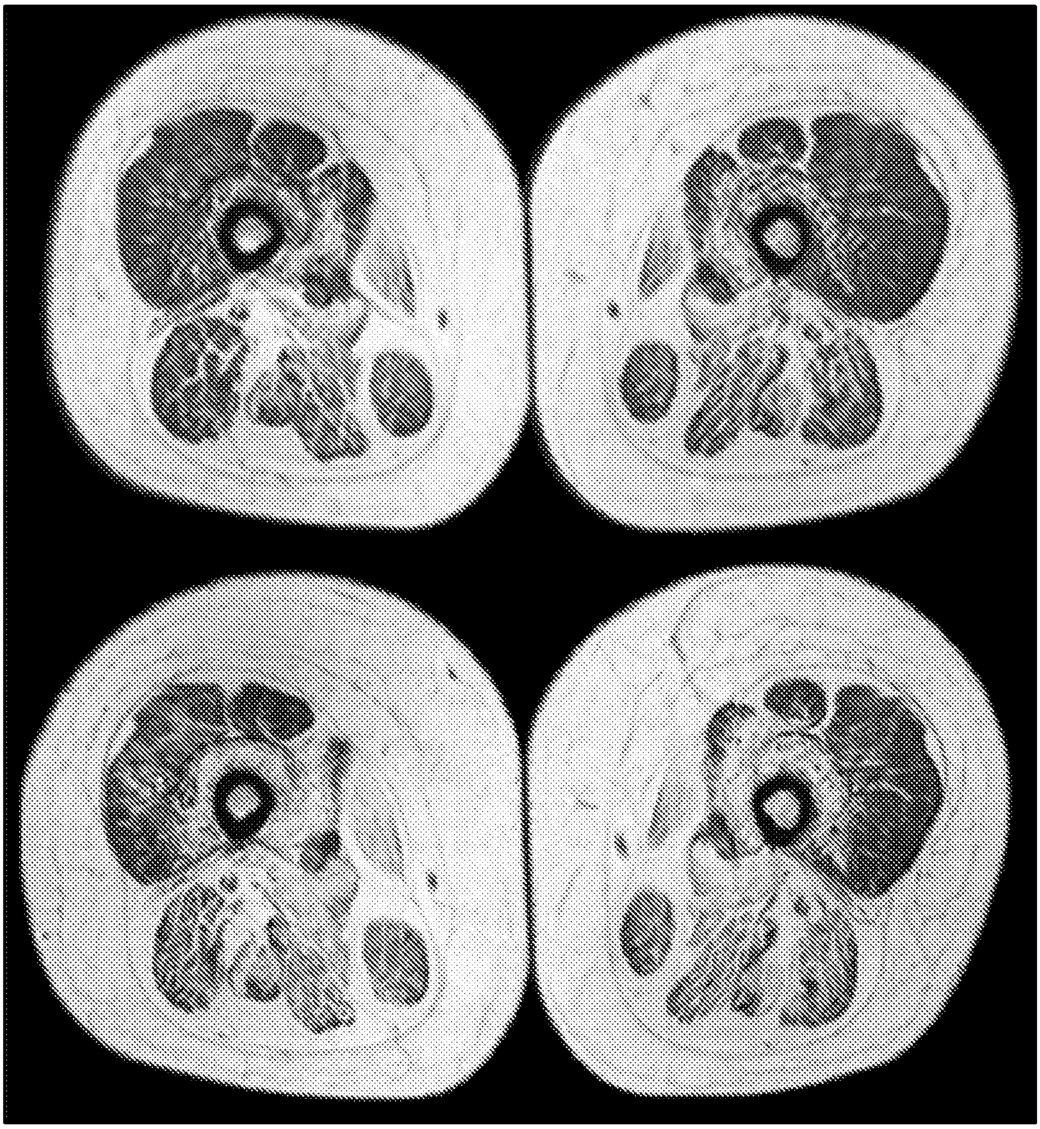

FIG. 20

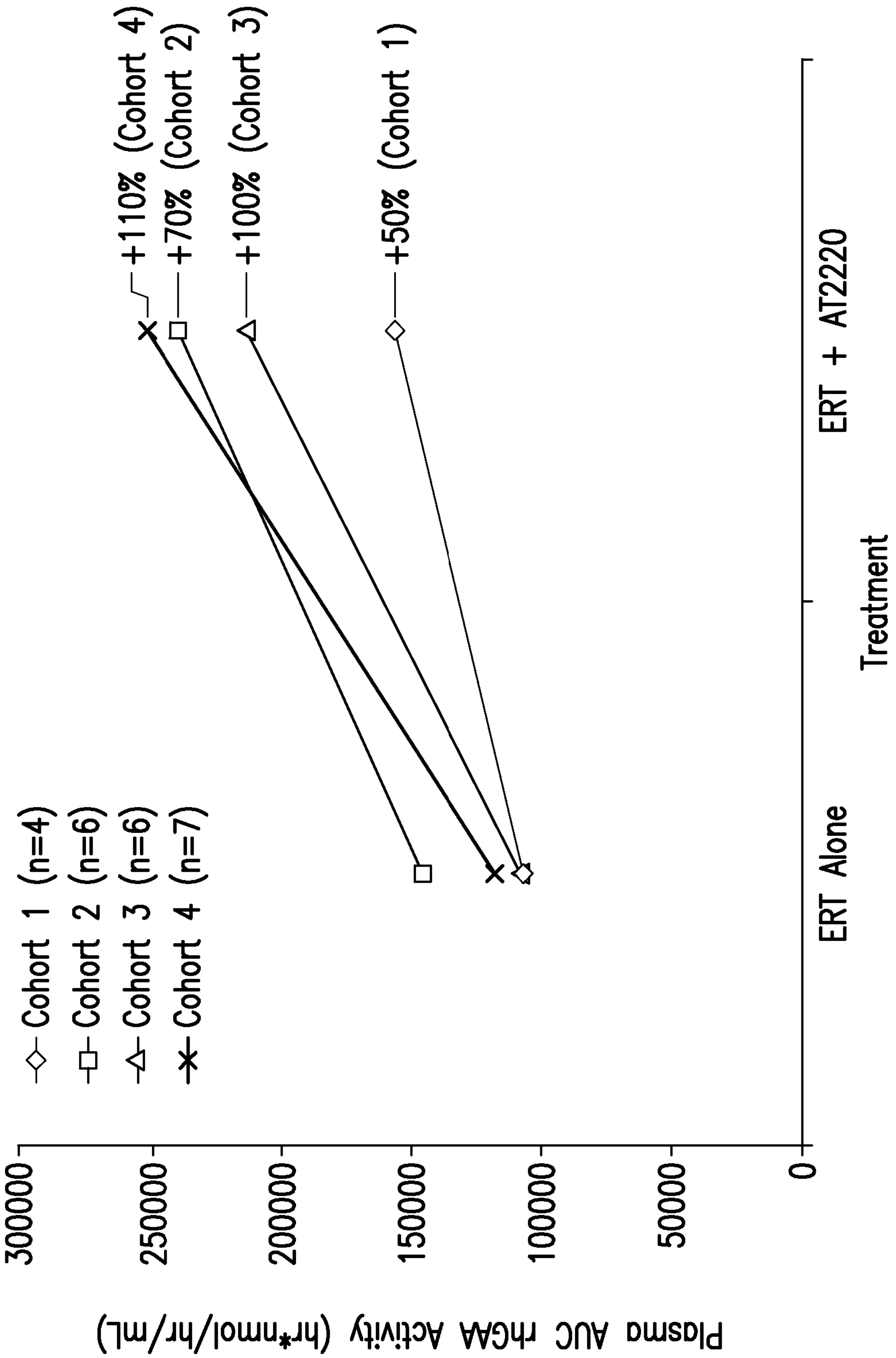
Cohort 1 (n=4)
Cohort 2 (n=6)
Cohort 3 (n=6)
Cohort 4 (n=7)
+110% (Cohort 4)
+70% (Cohort 2)
+100% (Cohort 3)
+50% (Cohort 1)
Plasma AUC rhGAA Activity (hr*nmol/hr/mL)
300000
250000
200000
150000
100000
50000
0
ERT Alone
Treatment
ERT + AT2220

FIG. 21

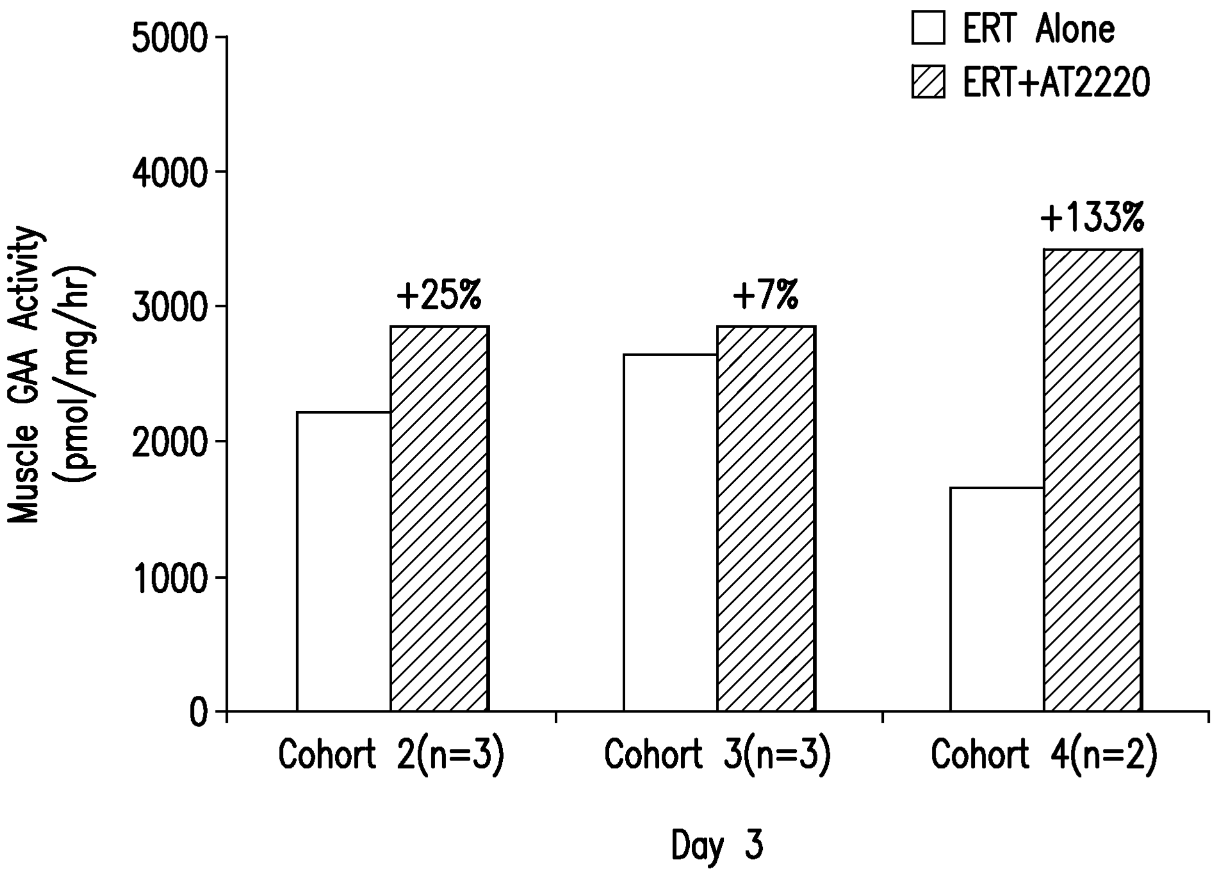
ERT Alone
ERT+AT2220
Muscle GAA Activity (pmol/mg/hr)
5000
4000
3000
2000
1000
0
+25%
+7%
+133%
Cohort 2(n=3)
Cohort 3(n=3)
Cohort 4(n=2)
Day 3

FIG. 22

Tissue Pharmacokinetics for DNJ (AT2220) and N-butyl DNJ (AT2221)

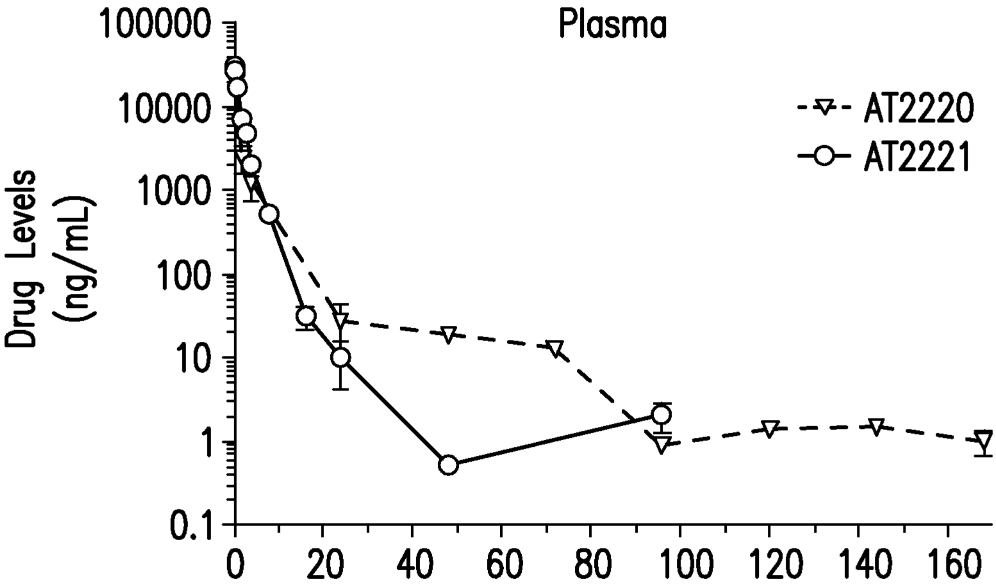


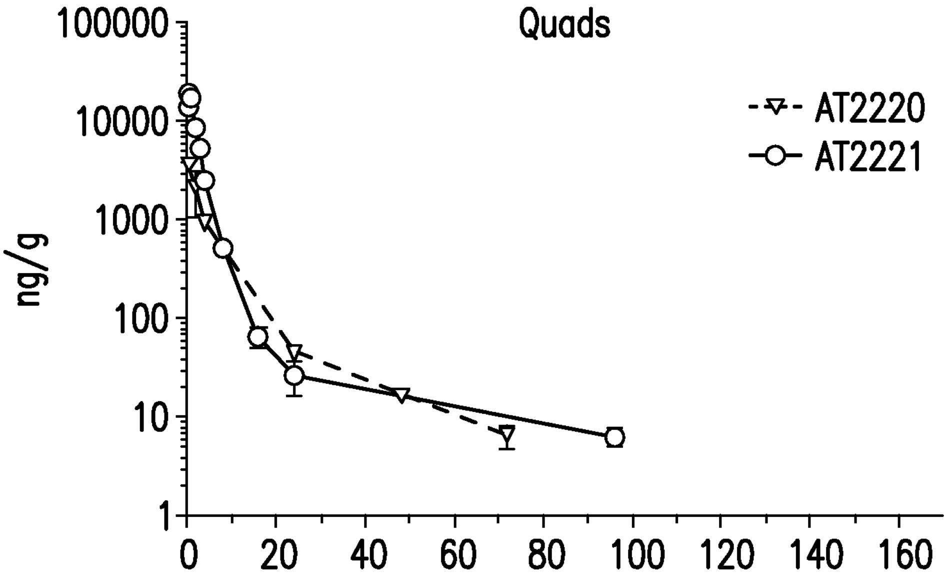


FIG. 23

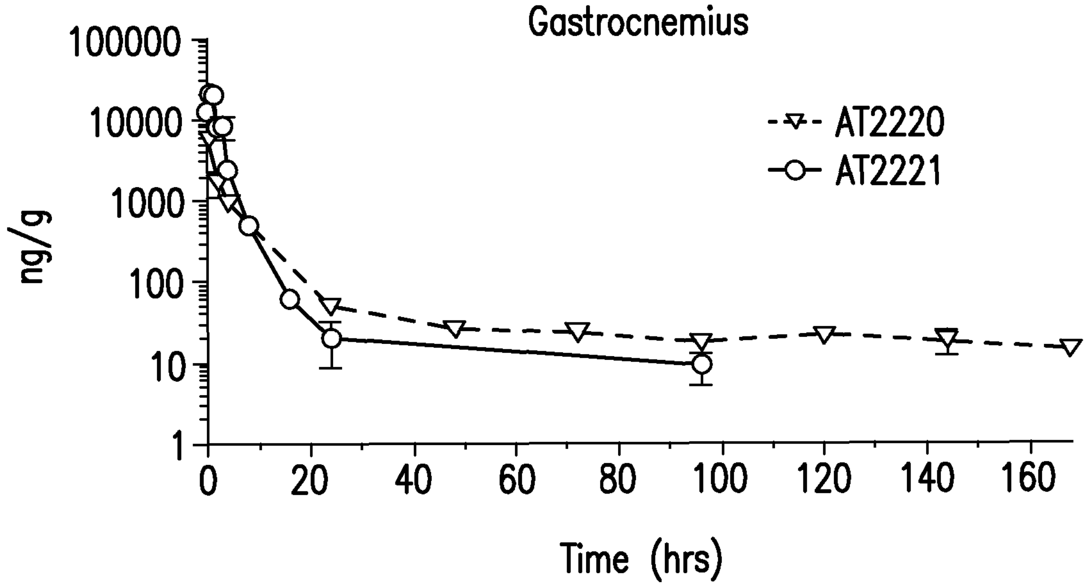


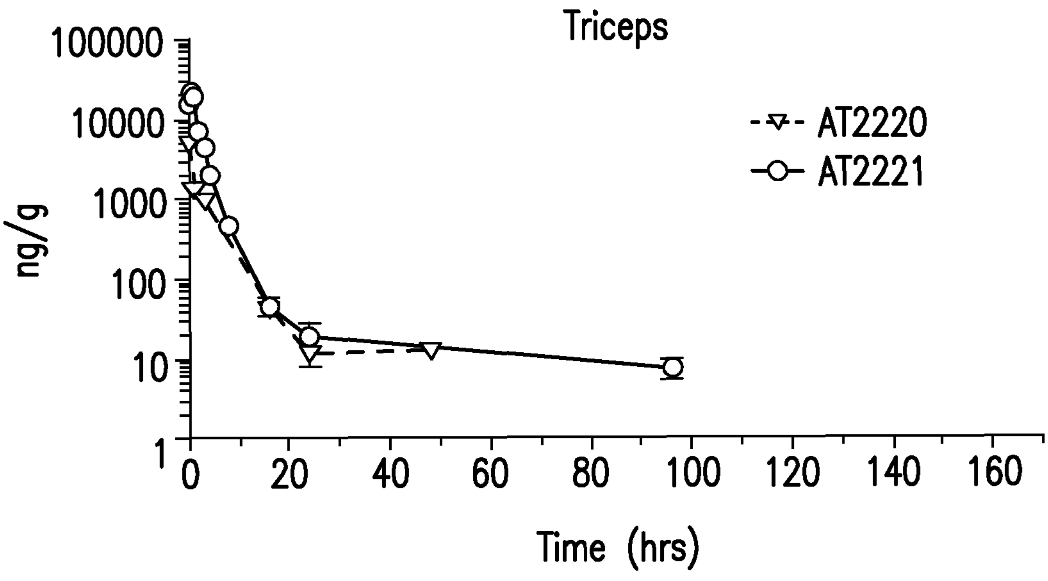


FIG. 23 continued

Effect of DNJ and NB-DNJ on Myozyme Pharmacokinetics. An oral dose of AT2220/DNJ or AT2221/NB-DNJ (100mg/kg) was administered 30 minutes prior i.v. administration of recombinant GAA (Myozyme at 10mg/kg).

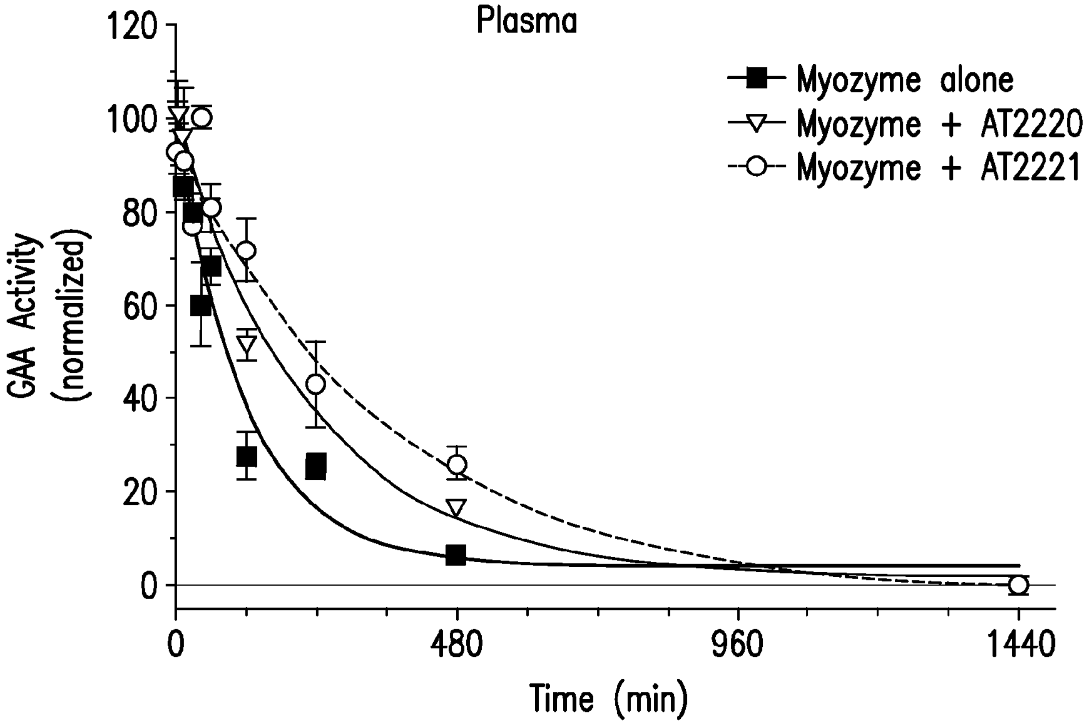


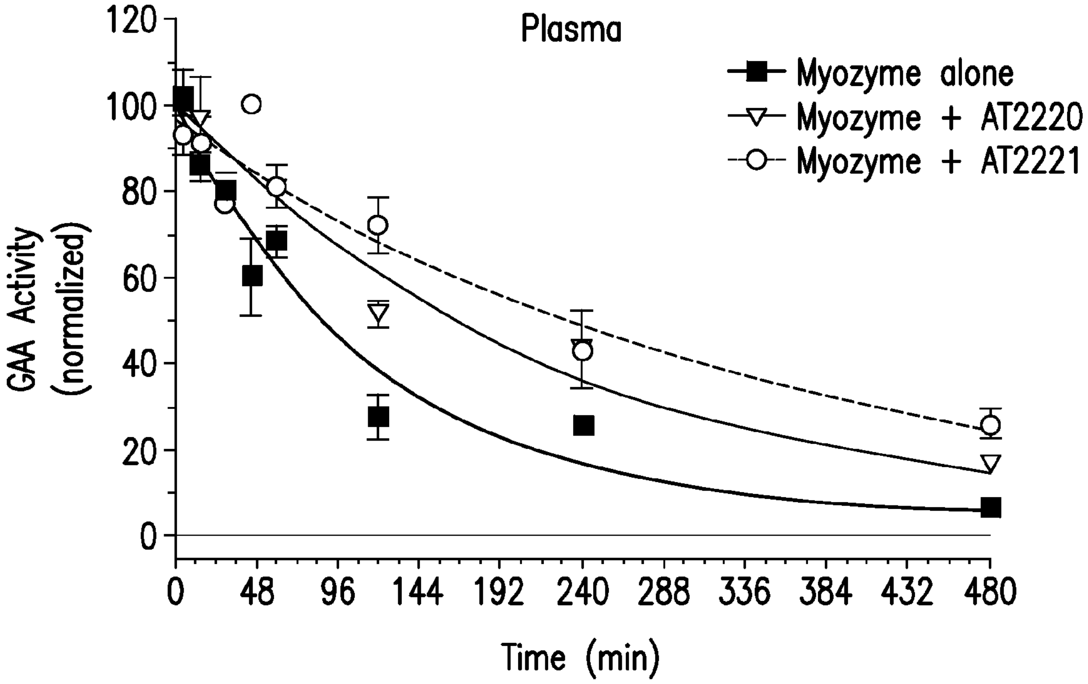


FIG. 24

Weatern blot of recombinant GAA in plasma at 2, 8, and 24 hours after i.v. administration of GAA.

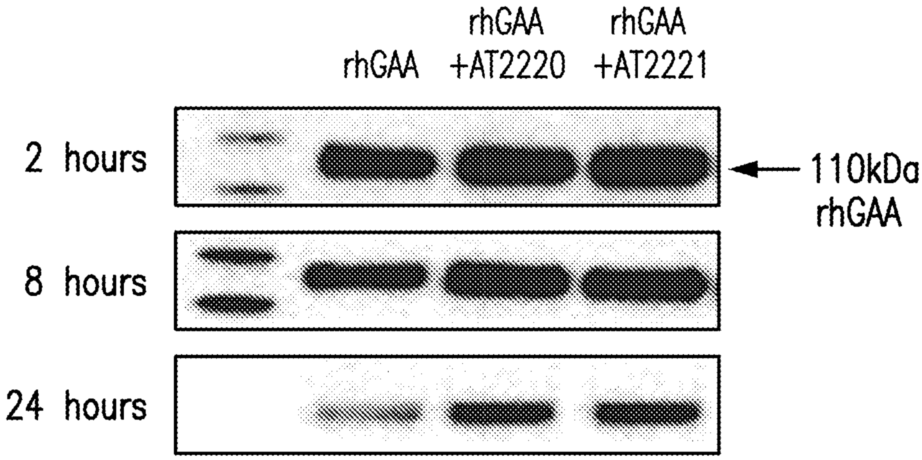


FIG. 25

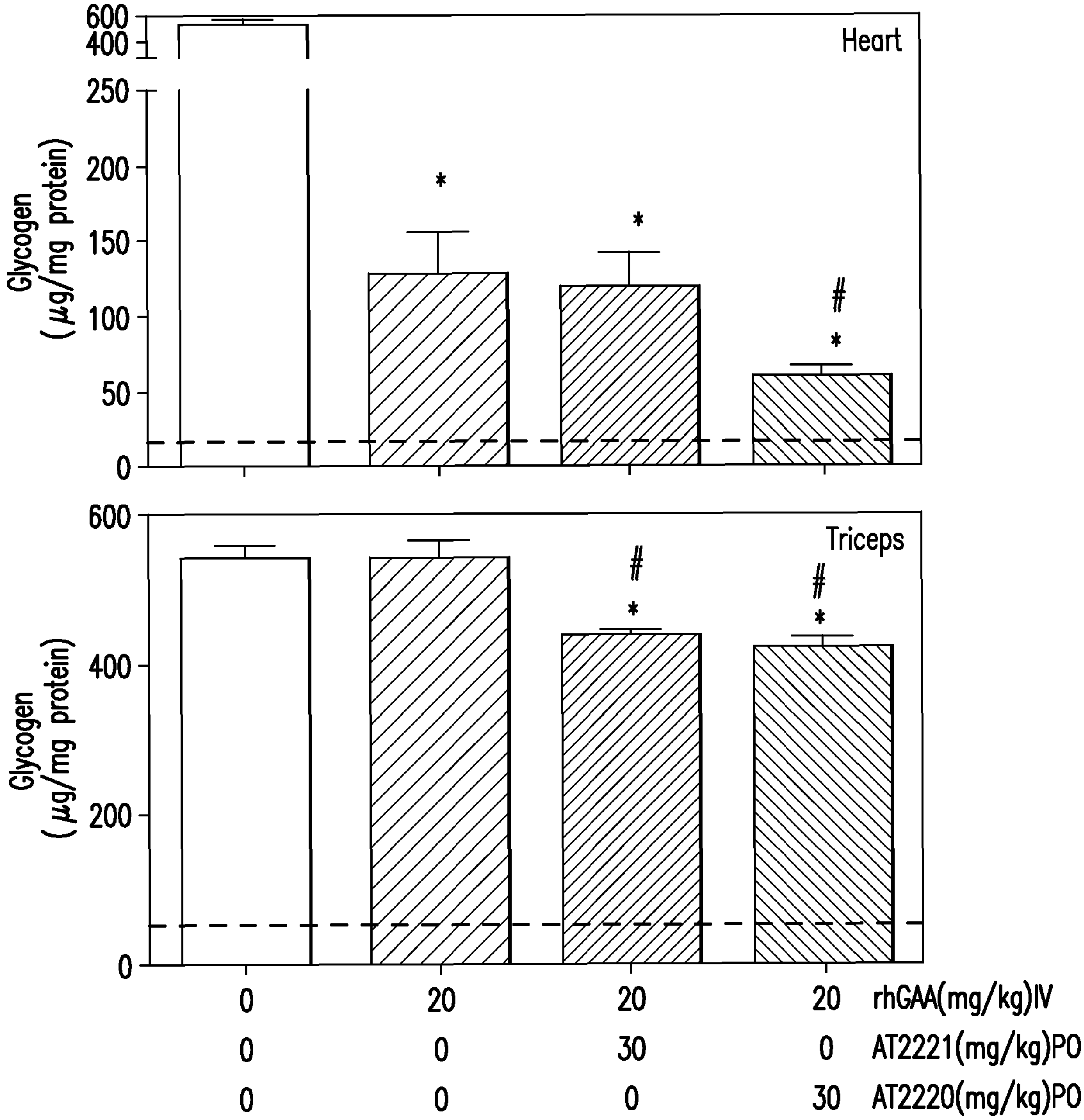
600
400
250
200
150
100
50
0
Glycogen
(μg/mg protein)
Heart
*
*
#
*
600
400
200
0
Glycogen
(μg/mg protein)
Triceps
#
*
#
*
0
20
20
20
rhGAA(mg/kg)IV
0
0
30
0
AT2221(mg/kg)PO
0
0
0
30
AT2220(mg/kg)PO

FIG. 26

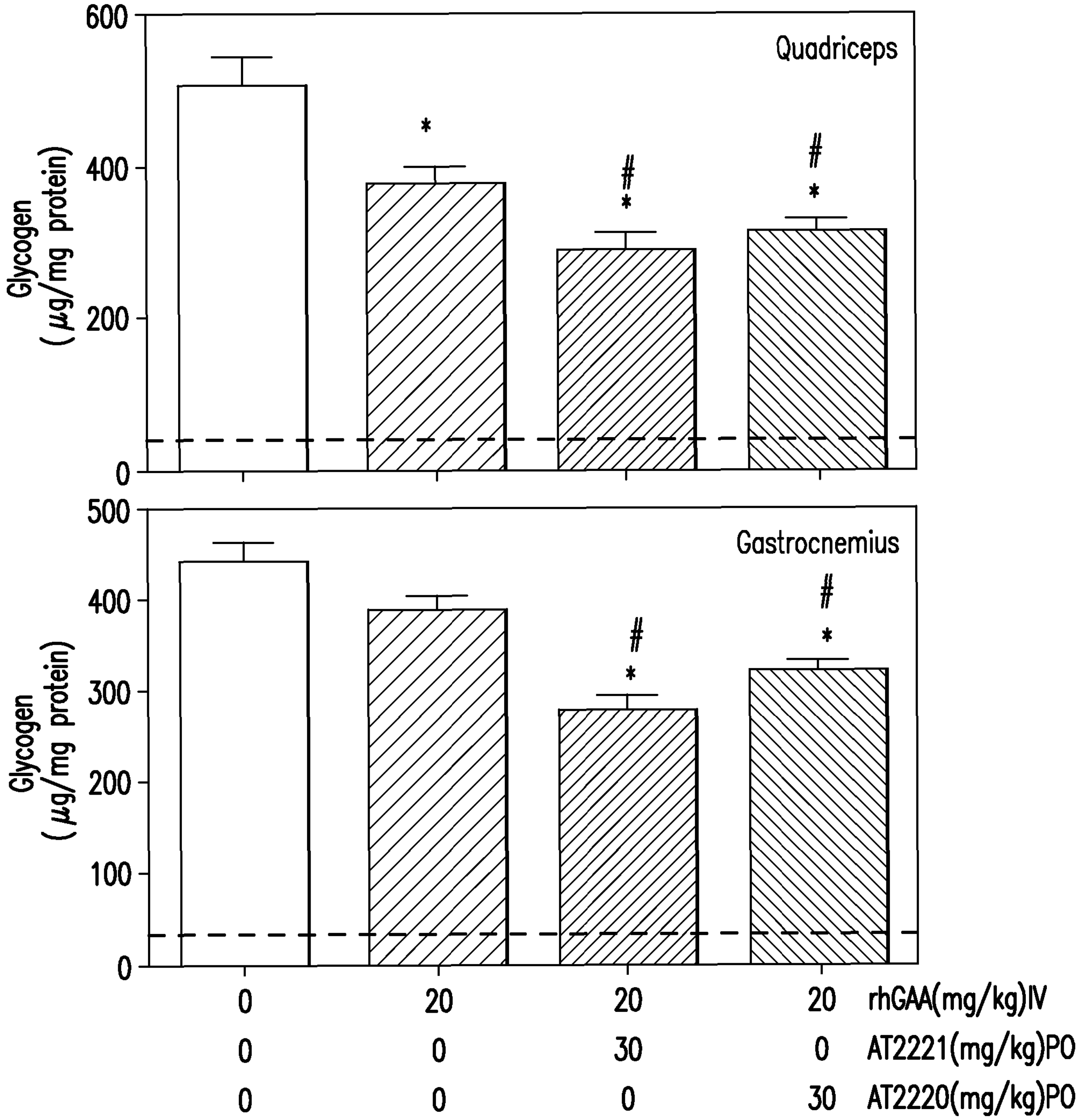


FIG. 26 continued

Table 41–Percentage reduction insubstrate upon treatment with rhGAA with or without DNJ and NB–DNJ coadministration. Numbers represent % reduction [100% = WT glycogen levels, 0% = KO glycogen levels]

| Tissues collected 21 days after 4th rhGAA injection | rhGAA (20mg/kg) | rhGAA + NB–DNJ(30mg/kg) | rhGAA + DNJ (30mg/kg) |
|---|---|---|---|
| Heart | 82 | 83 | 95 |
| Quads | 14 | 40 | 36 |
| Gastrocnemius | 11 | 40 | 37 |
| Triceps | 0 | 26 | 28 |

FIG. 26 continued

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6537785 B, Canfield
- US 2006264467 A
- US 2010119502 A
- US 7560424 B
- US 7396811 B, Lebowitz
- US 20090203575 A
- US 20090029467 A
- US 20080299640 A
- US 20080241118 A
- US 20060121018 A
- US 20050244400 A, Lebowitz
- US 7423135 B
- US 6534300 B
- US 2005077093 A
- US 20070280925 A
- US 20040029779 A


### Non-patent literature cited in the description


- **KLINGE et al.** *Neuropediatrics,* 2005, vol. 36 (1), 6-11
- **RABEN et al.** *Mol Ther.,* 2005, vol. 11 (1), 48-56
- **E.W. MARTIN.** Remington's Pharmaceutical Sciences
- REMINGTON S PHARMACEUTICAL SCIENCES
- **KHANNA et al.** *PLoS ONE,* 2012, vol. 7 (7), e40776